# EUROPÄISCHE PATENTANMELDUNG

(11) **EP 4 180 815 A2**
(43) Veröffentlichungstag der Anmeldung: **17.05.2023**
(21) Anmeldenummer: 22200826.0
(22) Anmeldetag: 25.06.2014
(51) Int. Cl.: G01N 33/68, G01N 33/543, G01N 21/64

(54) **IMMUNOLOGISCHER FESTPHASEN-TEST MIT ORTSAUFGELÖSTEM SIGNAL (SFIDA) ZUR QUANTIFIZIERUNG VON MINDESTENS 2 PROTEINAGGREGATEN**

(30) Priorität: 26.06.2013 DE 102013106713
(62) Teilanmeldung aus: 14752793.1
(71) Anmelder: Forschungszentrum Jülich GmbH, 52425 Jülich (DE)
(72) Erfinder: WILLBOLD, Dieter, 52428 Jülich (DE); BIRKMANN, Eva, 40589 Düsseldorf (DE); KRAVCHENKO, Kateryna, 45279 Essen (DE); FUNKE, Aileen, 96242 Sonnefeld (DE); BANNACH, Oliver, 40595 Düsseldorf (DE); HÜBINGER, Steffen, 40217 Düsseldorf (DE); KORTH, Carsten, 40219 Düsseldorf (DE); BADER, Verian, 45657 Recklinghausen (DE)
(74) Vertreter: Rupprecht, Kay

(57) **Zusammenfassung**

Die Erfindung betrifft ein Verfahren zur Ermittlung von Indikatoren zur Bestimmung von Krankheiten (Krankheitsindikatoren), in denen Aggregate fehlgefalteter Proteine eine Rolle spielen, sowie ein Verfahren zur selektiven Quantifizierung und/oder Charakterisierung dieser Krankheitsindikatoren.

<110>  Forschungszentrum Juelich

<120>  Verfahren zur Ermittlung von Indikatoren zur Bestimmung von
       Krankheiten

<130>  FZJ 1303 PCT

<160>  15

<170>  PatentIn version 3.5

<210>  1
<211>  122
<212>  PRT
<213>  Homo sapiens

<400>  1

Met Lys Leu Leu Thr Gly Leu Val Phe Cys Ser Leu Val Leu Gly Val
1               5                   10                  15

Ser Ser Arg Ser Phe Phe Ser Phe Leu Gly Glu Ala Phe Asp Gly Ala
            20                  25                  30

Arg Asp Met Trp Arg Ala Tyr Ser Asp Met Arg Glu Ala Asn Tyr Ile
        35                  40                  45

Gly Ser Asp Lys Tyr Phe His Ala Arg Gly Asn Tyr Asp Ala Ala Lys
    50                  55                  60

Arg Gly Pro Gly Gly Val Trp Ala Ala Glu Ala Ile Ser Asp Ala Arg
65                  70                  75                  80

Glu Asn Ile Gln Arg Phe Phe Gly His Gly Ala Glu Asp Ser Leu Ala
                85                  90                  95

Asp Gln Ala Ala Asn Glu Trp Gly Arg Ser Gly Lys Asp Pro Asn His
            100                 105                 110

Phe Arg Pro Ala Gly Leu Pro Glu Lys Tyr
        115                 120

<210>  2
<211>  213
<212>  PRT
<213>  Homo sapiens

<400>  2

Met Arg Pro Gly Thr Gly Gln Gly Gly Leu Glu Ala Pro Gly Glu Pro
1               5                   10                  15

Gly Pro Asn Leu Arg Gln Arg Trp Pro Leu Leu Leu Leu Gly Leu Ala
            20                  25                  30

Val Val Thr His Gly Leu Leu Arg Pro Thr Ala Ala Ser Gln Ser Arg
        35                  40                  45

Ala Leu Gly Pro Gly Ala Pro Gly Gly Ser Ser Arg Ser Ser Leu Arg
    50                  55                  60

Ser Arg Trp Gly Arg Phe Leu Leu Gln Arg Gly Ser Trp Thr Gly Pro
65                  70                  75                  80

Arg Cys Trp Pro Arg Gly Phe Gln Ser Lys His Asn Ser Val Thr His
                85                  90                  95

Val Phe Gly Ser Gly Thr Gln Leu Thr Val Leu Ser Gln Pro Lys Ala
            100                 105                 110

Thr Pro Ser Val Thr Leu Phe Pro Pro Ser Ser Glu Glu Leu Gln Ala
        115                 120                 125

Asn Lys Ala Thr Leu Val Cys Leu Met Asn Asp Phe Tyr Pro Gly Ile
    130                 135                 140

Leu Thr Val Thr Trp Lys Ala Asp Gly Thr Pro Ile Thr Gln Gly Val
145                 150                 155                 160

Glu Met Thr Thr Pro Ser Lys Gln Ser Asn Asn Lys Tyr Ala Ala Ser
                165                 170                 175

Ser Tyr Leu Ser Leu Thr Pro Glu Gln Trp Arg Ser Arg Arg Ser Tyr
            180                 185                 190

Ser Cys Gln Val Met His Glu Gly Ser Thr Val Glu Lys Thr Val Ala
        195                 200                 205

Pro Ala Glu Cys Ser
    210


<210>  3
<211>  267
<212>  PRT
<213>  Homo sapiens

<400>  3

<210>  4
<211>  100
<212>  PRT
<213>  Homo sapiens

<400>  4

Met Lys Leu Leu Ala Ala Thr Val Leu Leu Leu Thr Ile Cys Ser Leu
1               5                   10                  15

Glu Gly Ala Leu Val Arg Arg Gln Ala Lys Glu Pro Cys Val Glu Ser
            20                  25                  30

Leu Val Ser Gln Tyr Phe Gln Thr Val Thr Asp Tyr Gly Lys Asp Leu
        35                  40                  45

Met Glu Lys Val Lys Ser Pro Glu Leu Gln Ala Glu Ala Lys Ser Tyr
    50                  55                  60

Phe Glu Lys Ser Lys Glu Gln Leu Thr Pro Leu Ile Lys Lys Ala Gly
65                  70                  75                  80

Thr Glu Leu Val Asn Phe Leu Ser Tyr Phe Val Glu Leu Gly Thr Gln
                85                  90                  95

Pro Ala Thr Gln
            100

<210>  5
<211>  147
<212>  PRT
<213>  Homo sapiens

<400>  5

<210>  6
<211>  854
<212>  PRT
<213>  Homo sapiens

<400>  6

Met Pro Gly Gly Gly Pro Gln Gly Ala Pro Ala Ala Ala Gly Gly Gly
1               5                   10                  15

Gly Val Ser His Arg Ala Gly Ser Arg Asp Cys Leu Pro Pro Ala Ala
            20                  25                  30

Cys Phe Arg Arg Arg Arg Leu Ala Arg Arg Pro Gly Tyr Met Arg Ser
        35                  40                  45

Ser Thr Gly Pro Gly Ile Gly Phe Leu Ser Pro Ala Val Gly Thr Leu
    50                  55                  60

Phe Arg Phe Pro Gly Gly Val Ser Gly Glu Glu Ser His His Ser Glu
65                  70                  75                  80

Ser Arg Ala Arg Gln Cys Gly Leu Asp Ser Arg Gly Leu Leu Val Arg
                85                  90                  95

Ser Pro Val Ser Lys Ser Ala Ala Ala Pro Thr Val Thr Ser Val Arg
            100                 105                 110

Gly Thr Ser Ala His Phe Gly Ile Gln Leu Arg Gly Gly Thr Arg Leu
        115                 120                 125

Pro Asp Arg Leu Ser Trp Pro Cys Gly Pro Gly Ser Ala Gly Trp Gln
    130                 135                 140

Gln Glu Phe Ala Ala Met Asp Ser Ser Glu Thr Leu Asp Ala Ser Trp
145                 150                 155                 160

Glu Ala Ala Cys Ser Asp Gly Ala Arg Arg Val Arg Ala Ala Gly Ser
                165                 170                 175

Leu Pro Ser Ala Glu Leu Ser Ser Asn Ser Cys Ser Pro Gly Cys Gly
            180                 185                 190

Pro Glu Val Pro Pro Thr Pro Pro Gly Ser His Ser Ala Phe Thr Ser
        195                 200                 205

Ser Phe Ser Phe Ile Arg Leu Ser Leu Gly Ser Ala Gly Glu Arg Gly
    210                 215                 220

Glu Ala Glu Gly Cys Pro Pro Ser Arg Glu Ala Glu Ser His Cys Gln
225                 230                 235                 240

Ser Pro Gln Glu Met Gly Ala Lys Ala Ala Ser Leu Asp Gly Pro His
                245                 250                 255

Glu Asp Pro Arg Cys Leu Ser Arg Pro Phe Ser Leu Leu Ala Thr Arg
            260                 265                 270

Val Ser Ala Asp Leu Ala Gln Ala Ala Arg Asn Ser Ser Arg Pro Glu
        275                 280                 285

Arg Asp Met His Ser Leu Pro Asp Met Asp Pro Gly Ser Ser Ser Ser
    290                 295                 300

Leu Asp Pro Ser Leu Ala Gly Cys Gly Gly Asp Gly Ser Ser Gly Ser
305                 310                 315                 320

Gly Asp Ala His Ser Trp Asp Thr Leu Leu Arg Lys Trp Glu Pro Val
                325                 330                 335

Leu Arg Asp Cys Leu Leu Arg Asn Arg Arg Gln Met Glu Val Ile Ser
            340                 345                 350

Leu Arg Leu Lys Leu Gln Lys Leu Gln Glu Asp Ala Val Glu Asn Asp
        355                 360                 365

Asp Tyr Asp Lys Ala Glu Thr Leu Gln Gln Arg Leu Glu Asp Leu Glu
    370                 375                 380

Gln Glu Lys Ile Ser Leu His Phe Gln Leu Pro Ser Arg Gln Pro Ala
385                 390                 395                 400

Leu Ser Ser Phe Leu Gly His Leu Ala Ala Gln Val Gln Ala Ala Leu
                405                 410                 415

Arg Arg Gly Ala Thr Gln Gln Ala Ser Gly Asp Asp Thr His Thr Pro
            420                 425                 430

Leu Arg Met Glu Pro Arg Leu Leu Glu Pro Thr Ala Gln Asp Ser Leu
        435                 440                 445

His Val Ser Ile Thr Arg Arg Asp Trp Leu Leu Gln Glu Lys Gln Gln
    450                 455                 460

Leu Gln Lys Glu Ile Glu Ala Leu Gln Ala Arg Met Phe Val Leu Glu
465                 470                 475                 480

Ala Lys Asp Gln Gln Leu Arg Arg Glu Ile Glu Glu Gln Glu Gln Gln
                485                 490                 495

Leu Gln Trp Gln Gly Cys Asp Leu Thr Pro Leu Val Gly Gln Leu Ser
            500                 505                 510

Leu Gly Gln Leu Gln Glu Val Ser Lys Ala Leu Gln Asp Thr Leu Ala
        515                 520                 525

Ser Ala Gly Gln Ile Pro Phe His Ala Glu Pro Pro Glu Thr Ile Arg
    530                 535                 540

Ser Leu Gln Glu Arg Ile Lys Ser Leu Asn Leu Ser Leu Lys Glu Ile
545                 550                 555                 560

Thr Thr Lys Val Cys Met Ser Glu Lys Phe Cys Ser Thr Leu Arg Lys
                565                 570                 575

Lys Val Asn Asp Ile Glu Thr Gln Leu Pro Ala Leu Leu Glu Ala Lys
            580                 585                 590

Met His Ala Ile Ser Gly Asn His Phe Trp Thr Ala Lys Asp Leu Thr

595                 600                 605

Glu Glu Ile Arg Ser Leu Thr Ser Glu Arg Glu Gly Leu Glu Gly Leu
    610                 615                 620

Leu Ser Lys Leu Leu Val Leu Ser Ser Arg Asn Val Lys Lys Leu Gly
625                 630                 635                 640

Ser Val Lys Glu Asp Tyr Asn Arg Leu Arg Arg Glu Val Glu His Gln
                645                 650                 655

Glu Thr Ala Tyr Glu Thr Ser Val Lys Glu Asn Thr Met Lys Tyr Met
            660                 665                 670

Glu Thr Leu Lys Asn Lys Leu Cys Ser Cys Lys Cys Pro Leu Leu Gly
        675                 680                 685

Lys Val Trp Glu Ala Asp Leu Glu Ala Cys Arg Leu Leu Ile Gln Ser
    690                 695                 700

Leu Gln Leu Gln Glu Ala Arg Gly Ser Leu Ser Val Glu Asp Glu Arg
705                 710                 715                 720

Gln Met Asp Asp Leu Glu Gly Ala Ala Pro Pro Ile Pro Pro Arg Leu
                725                 730                 735

His Ser Glu Asp Lys Arg Lys Thr Pro Leu Lys Val Leu Glu Glu Trp
            740                 745                 750

Lys Thr His Leu Ile Pro Ser Leu His Cys Ala Gly Gly Glu Gln Lys
        755                 760                 765

Glu Glu Ser Tyr Ile Leu Ser Ala Glu Leu Gly Glu Lys Cys Glu Asp
    770                 775                 780

Ile Gly Lys Lys Leu Leu Tyr Leu Glu Asp Gln Leu His Thr Ala Ile
785                 790                 795                 800

His Ser His Asp Glu Asp Leu Ile Gln Ser Leu Arg Arg Glu Leu Gln
                805                 810                 815

Met Val Lys Glu Thr Leu Gln Ala Met Ile Leu Gln Leu Gln Pro Ala
            820                 825                 830

Lys Glu Ala Gly Glu Arg Glu Ala Ala Ala Ser Cys Met Thr Ala Gly
        835                 840                 845

Val His Glu Ala Gln Ala
    850

<210>  7
<211>  526
<212>  PRT
<213>  Homo sapiens

<400> 7

<210>  8
<211>  89
<212>  PRT
<213>  Homo sapiens

<400>  8

Met Gly Ile Leu Lys Leu Gln Val Phe Leu Ile Val Leu Ser Val Ala
1               5                   10                  15

Leu Asn His Leu Lys Ala Thr Pro Ile Glu Ser His Gln Val Glu Lys
            20                  25                  30

Arg Lys Cys Asn Thr Ala Thr Cys Ala Thr Gln Arg Leu Ala Asn Phe
        35                  40                  45

Leu Val His Ser Ser Asn Asn Phe Gly Ala Ile Leu Ser Ser Thr Asn
    50                  55                  60

Val Gly Ser Asn Thr Tyr Gly Lys Arg Asn Ala Val Glu Val Leu Lys
65                  70                  75                  80

Arg Glu Pro Leu Asn Tyr Leu Pro Leu
                85

<210>  9
<211>  154
<212>  PRT
<213>  Homo sapiens

<400>  9

<210>  10
<211>  140
<212>  PRT
<213>  Homo sapiens

<400>  10

<210>  11
<211>  758
<212>  PRT
<213>  Homo sapiens

<400>  11

Met Ala Glu Pro Arg Gln Glu Phe Glu Val Met Glu Asp His Ala Gly
1               5                   10                  15

Thr Tyr Gly Leu Gly Asp Arg Lys Asp Gln Gly Gly Tyr Thr Met His
            20                  25                  30

Gln Asp Gln Glu Gly Asp Thr Asp Ala Gly Leu Lys Glu Ser Pro Leu
        35                  40                  45

Gln Thr Pro Thr Glu Asp Gly Ser Glu Glu Pro Gly Ser Glu Thr Ser
    50                  55                  60

Asp Ala Lys Ser Thr Pro Thr Ala Glu Asp Val Thr Ala Pro Leu Val
65                  70                  75                  80

Asp Glu Gly Ala Pro Gly Lys Gln Ala Ala Ala Gln Pro His Thr Glu

                85                  90                  95

Ile Pro Glu Gly Thr Thr Ala Glu Glu Ala Gly Ile Gly Asp Thr Pro
            100                 105                 110

Ser Leu Glu Asp Glu Ala Ala Gly His Val Thr Gln Glu Pro Glu Ser
        115                 120                 125

Gly Lys Val Val Gln Glu Gly Phe Leu Arg Glu Pro Gly Pro Pro Gly
    130                 135                 140

Leu Ser His Gln Leu Met Ser Gly Met Pro Gly Ala Pro Leu Leu Pro
145                 150                 155                 160

Glu Gly Pro Arg Glu Ala Thr Arg Gln Pro Ser Gly Thr Gly Pro Glu
                165                 170                 175

Asp Thr Glu Gly Gly Arg His Ala Pro Glu Leu Leu Lys His Gln Leu
            180                 185                 190

Leu Gly Asp Leu His Gln Glu Gly Pro Pro Leu Lys Gly Ala Gly Gly
        195                 200                 205

Lys Glu Arg Pro Gly Ser Lys Glu Glu Val Asp Glu Asp Arg Asp Val
    210                 215                 220

Asp Glu Ser Ser Pro Gln Asp Ser Pro Pro Ser Lys Ala Ser Pro Ala
225                 230                 235                 240

Gln Asp Gly Arg Pro Pro Gln Thr Ala Ala Arg Glu Ala Thr Ser Ile
                245                 250                 255

Pro Gly Phe Pro Ala Glu Gly Ala Ile Pro Leu Pro Val Asp Phe Leu
            260                 265                 270

Ser Lys Val Ser Thr Glu Ile Pro Ala Ser Glu Pro Asp Gly Pro Ser
        275                 280                 285

Val Gly Arg Ala Lys Gly Gln Asp Ala Pro Leu Glu Phe Thr Phe His
    290                 295                 300

Val Glu Ile Thr Pro Asn Val Gln Lys Glu Gln Ala His Ser Glu Glu
305                 310                 315                 320

His Leu Gly Arg Ala Ala Phe Pro Gly Ala Pro Gly Glu Gly Pro Glu
                325                 330                 335

Ala Arg Gly Pro Ser Leu Gly Glu Asp Thr Lys Glu Ala Asp Leu Pro
            340                 345                 350

Glu Pro Ser Glu Lys Gln Pro Ala Ala Ala Pro Arg Gly Lys Pro Val
        355                 360                 365

Ser Arg Val Pro Gln Leu Lys Ala Arg Met Val Ser Lys Ser Lys Asp
    370                 375                 380

Gly Thr Gly Ser Asp Asp Lys Lys Ala Lys Thr Ser Thr Arg Ser Ser
385                 390                 395                 400

Ala Lys Thr Leu Lys Asn Arg Pro Cys Leu Ser Pro Lys His Pro Thr
                405                 410                 415

Pro Gly Ser Ser Asp Pro Leu Ile Gln Pro Ser Ser Pro Ala Val Cys
            420                 425                 430

Pro Glu Pro Pro Ser Ser Pro Lys Tyr Val Ser Ser Val Thr Ser Arg
        435                 440                 445

Thr Gly Ser Ser Gly Ala Lys Glu Met Lys Leu Lys Gly Ala Asp Gly
    450                 455                 460

Lys Thr Lys Ile Ala Thr Pro Arg Gly Ala Ala Pro Pro Gly Gln Lys
465                 470                 475                 480

Gly Gln Ala Asn Ala Thr Arg Ile Pro Ala Lys Thr Pro Pro Ala Pro
                485                 490                 495

Lys Thr Pro Pro Ser Ser Gly Glu Pro Pro Lys Ser Gly Asp Arg Ser
            500                 505                 510

Gly Tyr Ser Ser Pro Gly Ser Pro Gly Thr Pro Gly Ser Arg Ser Arg
        515                 520                 525

Thr Pro Ser Leu Pro Thr Pro Pro Thr Arg Glu Pro Lys Lys Val Ala
    530                 535                 540

Val Val Arg Thr Pro Pro Lys Ser Pro Ser Ser Ala Lys Ser Arg Leu
545                 550                 555                 560

Gln Thr Ala Pro Val Pro Met Pro Asp Leu Lys Asn Val Lys Ser Lys
                565                 570                 575

Ile Gly Ser Thr Glu Asn Leu Lys His Gln Pro Gly Gly Gly Lys Val
            580                 585                 590

Gln Ile Ile Asn Lys Lys Leu Asp Leu Ser Asn Val Gln Ser Lys Cys
        595                 600                 605

Gly Ser Lys Asp Asn Ile Lys His Val Pro Gly Gly Gly Ser Val Gln
    610                 615                 620

Ile Val Tyr Lys Pro Val Asp Leu Ser Lys Val Thr Ser Lys Cys Gly
625                 630                 635                 640

Ser Leu Gly Asn Ile His His Lys Pro Gly Gly Gly Gln Val Glu Val
                645                 650                 655

Lys Ser Glu Lys Leu Asp Phe Lys Asp Arg Val Gln Ser Lys Ile Gly
            660                 665                 670

Ser Leu Asp Asn Ile Thr His Val Pro Gly Gly Gly Asn Lys Lys Ile
        675                 680                 685

Glu Thr His Lys Leu Thr Phe Arg Glu Asn Ala Lys Ala Lys Thr Asp
    690                 695                 700

His Gly Ala Glu Ile Val Tyr Lys Ser Pro Val Val Ser Gly Asp Thr
705                 710                 715                 720

Ser Pro Arg His Leu Ser Asn Val Ser Ser Thr Gly Ser Ile Asp Met
                725                 730                 735

Val Asp Ser Pro Gln Leu Ala Thr Leu Ala Asp Glu Val Ser Ala Ser
            740                 745                 750

Leu Ala Lys Gln Gly Leu
        755

<210>  12
<211>  414
<212>  PRT
<213>  Homo sapiens

<400>  12

<210>  13
<211>  3142

<212>  PRT
<213>  Homo sapiens

<400>  13

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Pro Pro Pro Pro Pro Pro Pro Pro Pro Pro
        35                  40                  45

Pro Gln Leu Pro Gln Pro Pro Pro Gln Ala Gln Pro Leu Leu Pro Gln
    50                  55                  60

Pro Gln Pro Pro Pro Pro Pro Pro Pro Pro Pro Pro Gly Pro Ala Val
65                  70                  75                  80

Ala Glu Glu Pro Leu His Arg Pro Lys Lys Glu Leu Ser Ala Thr Lys
                85                  90                  95

Lys Asp Arg Val Asn His Cys Leu Thr Ile Cys Glu Asn Ile Val Ala
            100                 105                 110

Gln Ser Val Arg Asn Ser Pro Glu Phe Gln Lys Leu Leu Gly Ile Ala
        115                 120                 125

Met Glu Leu Phe Leu Leu Cys Ser Asp Asp Ala Glu Ser Asp Val Arg
    130                 135                 140

Met Val Ala Asp Glu Cys Leu Asn Lys Val Ile Lys Ala Leu Met Asp
145                 150                 155                 160

Ser Asn Leu Pro Arg Leu Gln Leu Glu Leu Tyr Lys Glu Ile Lys Lys
                165                 170                 175

Asn Gly Ala Pro Arg Ser Leu Arg Ala Ala Leu Trp Arg Phe Ala Glu
            180                 185                 190

Leu Ala His Leu Val Arg Pro Gln Lys Cys Arg Pro Tyr Leu Val Asn
        195                 200                 205

Leu Leu Pro Cys Leu Thr Arg Thr Ser Lys Arg Pro Glu Glu Ser Val
    210                 215                 220

Gln Glu Thr Leu Ala Ala Ala Val Pro Lys Ile Met Ala Ser Phe Gly
225                 230                 235                 240

Asn Phe Ala Asn Asp Asn Glu Ile Lys Val Leu Leu Lys Ala Phe Ile
                245                 250                 255

Ala Asn Leu Lys Ser Ser Ser Pro Thr Ile Arg Arg Thr Ala Ala Gly
            260                 265                 270

Ser Ala Val Ser Ile Cys Gln His Ser Arg Arg Thr Gln Tyr Phe Tyr
        275                 280                 285

Ser Trp Leu Leu Asn Val Leu Leu Gly Leu Leu Val Pro Val Glu Asp
    290                 295                 300

Glu His Ser Thr Leu Leu Ile Leu Gly Val Leu Leu Thr Leu Arg Tyr
305                 310                 315                 320

Leu Val Pro Leu Leu Gln Gln Gln Val Lys Asp Thr Ser Leu Lys Gly
                325                 330                 335

Ser Phe Gly Val Thr Arg Lys Glu Met Glu Val Ser Pro Ser Ala Glu
            340                 345                 350

Gln Leu Val Gln Val Tyr Glu Leu Thr Leu His His Thr Gln His Gln
        355                 360                 365

Asp His Asn Val Val Thr Gly Ala Leu Glu Leu Leu Gln Gln Leu Phe
    370                 375                 380

Arg Thr Pro Pro Pro Glu Leu Leu Gln Thr Leu Thr Ala Val Gly Gly
385                 390                 395                 400

Ile Gly Gln Leu Thr Ala Ala Lys Glu Glu Ser Gly Gly Arg Ser Arg
                405                 410                 415

Ser Gly Ser Ile Val Glu Leu Ile Ala Gly Gly Gly Ser Ser Cys Ser
            420                 425                 430

Pro Val Leu Ser Arg Lys Gln Lys Gly Lys Val Leu Leu Gly Glu Glu
        435                 440                 445

Glu Ala Leu Glu Asp Asp Ser Glu Ser Arg Ser Asp Val Ser Ser Ser
    450                 455                 460

Ala Leu Thr Ala Ser Val Lys Asp Glu Ile Ser Gly Glu Leu Ala Ala
465                 470                 475                 480

Ser Ser Gly Val Ser Thr Pro Gly Ser Ala Gly His Asp Ile Ile Thr
                485                 490                 495

Glu Gln Pro Arg Ser Gln His Thr Leu Gln Ala Asp Ser Val Asp Leu
            500                 505                 510

Ala Ser Cys Asp Leu Thr Ser Ser Ala Thr Asp Gly Asp Glu Glu Asp
        515                 520                 525

Ile Leu Ser His Ser Ser Ser Gln Val Ser Ala Val Pro Ser Asp Pro
    530                 535                 540

Ala Met Asp Leu Asn Asp Gly Thr Gln Ala Ser Ser Pro Ile Ser Asp

545                 550                 555                 560

Ser Ser Gln Thr Thr Thr Glu Gly Pro Asp Ser Ala Val Thr Pro Ser
                565                 570                 575

Asp Ser Ser Glu Ile Val Leu Asp Gly Thr Asp Asn Gln Tyr Leu Gly
            580                 585                 590

Leu Gln Ile Gly Gln Pro Gln Asp Glu Asp Glu Glu Ala Thr Gly Ile
        595                 600                 605

Leu Pro Asp Glu Ala Ser Glu Ala Phe Arg Asn Ser Ser Met Ala Leu
    610                 615                 620

Gln Gln Ala His Leu Leu Lys Asn Met Ser His Cys Arg Gln Pro Ser
625                 630                 635                 640

Asp Ser Ser Val Asp Lys Phe Val Leu Arg Asp Glu Ala Thr Glu Pro
                645                 650                 655

Gly Asp Gln Glu Asn Lys Pro Cys Arg Ile Lys Gly Asp Ile Gly Gln
            660                 665                 670

Ser Thr Asp Asp Asp Ser Ala Pro Leu Val His Cys Val Arg Leu Leu
        675                 680                 685

Ser Ala Ser Phe Leu Leu Thr Gly Gly Lys Asn Val Leu Val Pro Asp
    690                 695                 700

Arg Asp Val Arg Val Ser Val Lys Ala Leu Ala Leu Ser Cys Val Gly
705                 710                 715                 720

Ala Ala Val Ala Leu His Pro Glu Ser Phe Phe Ser Lys Leu Tyr Lys
                725                 730                 735

Val Pro Leu Asp Thr Thr Glu Tyr Pro Glu Glu Gln Tyr Val Ser Asp
            740                 745                 750

Ile Leu Asn Tyr Ile Asp His Gly Asp Pro Gln Val Arg Gly Ala Thr
        755                 760                 765

Ala Ile Leu Cys Gly Thr Leu Ile Cys Ser Ile Leu Ser Arg Ser Arg
    770                 775                 780

Phe His Val Gly Asp Trp Met Gly Thr Ile Arg Thr Leu Thr Gly Asn
785                 790                 795                 800

Thr Phe Ser Leu Ala Asp Cys Ile Pro Leu Leu Arg Lys Thr Leu Lys
                805                 810                 815

Asp Glu Ser Ser Val Thr Cys Lys Leu Ala Cys Thr Ala Val Arg Asn
            820                 825                 830

Cys Val Met Ser Leu Cys Ser Ser Ser Tyr Ser Glu Leu Gly Leu Gln
        835                 840                 845

Leu Ile Ile Asp Val Leu Thr Leu Arg Asn Ser Ser Tyr Trp Leu Val
    850                 855                 860

Arg Thr Glu Leu Leu Glu Thr Leu Ala Glu Ile Asp Phe Arg Leu Val
865                 870                 875                 880

Ser Phe Leu Glu Ala Lys Ala Glu Asn Leu His Arg Gly Ala His His
                885                 890                 895

Tyr Thr Gly Leu Leu Lys Leu Gln Glu Arg Val Leu Asn Asn Val Val
            900                 905                 910

Ile His Leu Leu Gly Asp Glu Asp Pro Arg Val Arg His Val Ala Ala
        915                 920                 925

Ala Ser Leu Ile Arg Leu Val Pro Lys Leu Phe Tyr Lys Cys Asp Gln
    930                 935                 940

Gly Gln Ala Asp Pro Val Val Ala Val Ala Arg Asp Gln Ser Ser Val
945                 950                 955                 960

Tyr Leu Lys Leu Leu Met His Glu Thr Gln Pro Pro Ser His Phe Ser
                965                 970                 975

Val Ser Thr Ile Thr Arg Ile Tyr Arg Gly Tyr Asn Leu Leu Pro Ser
            980                 985                 990

Ile Thr Asp Val Thr Met Glu Asn  Asn Leu Ser Arg Val  Ile Ala Ala
        995                 1000                 1005

Val Ser  His Glu Leu Ile Thr  Ser Thr Thr Arg Ala  Leu Thr Phe
    1010                 1015                 1020

Gly Cys  Cys Glu Ala Leu Cys  Leu Leu Ser Thr Ala  Phe Pro Val
    1025                 1030                 1035

Cys Ile  Trp Ser Leu Gly Trp  His Cys Gly Val Pro  Pro Leu Ser
    1040                 1045                 1050

Ala Ser  Asp Glu Ser Arg Lys  Ser Cys Thr Val Gly  Met Ala Thr
    1055                 1060                 1065

Met Ile  Leu Thr Leu Leu Ser  Ser Ala Trp Phe Pro  Leu Asp Leu
    1070                 1075                 1080

Ser Ala  His Gln Asp Ala Leu  Ile Leu Ala Gly Asn  Leu Leu Ala
    1085                 1090                 1095

Ala Ser  Ala Pro Lys Ser Leu  Arg Ser Ser Trp Ala  Ser Glu Glu
    1100                 1105                 1110

Glu Ala Asn Pro Ala Ala Thr Lys Gln Glu Glu Val Trp Pro Ala
    1115                1120                1125

Leu Gly Asp Arg Ala Leu Val Pro Met Val Glu Gln Leu Phe Ser
    1130                1135                1140

His Leu Leu Lys Val Ile Asn Ile Cys Ala His Val Leu Asp Asp
    1145                1150                1155

Val Ala Pro Gly Pro Ala Ile Lys Ala Ala Leu Pro Ser Leu Thr
    1160                1165                1170

Asn Pro Pro Ser Leu Ser Pro Ile Arg Arg Lys Gly Lys Glu Lys
    1175                1180                1185

Glu Pro Gly Glu Gln Ala Ser Val Pro Leu Ser Pro Lys Lys Gly
    1190                1195                1200

Ser Glu Ala Ser Ala Ala Ser Arg Gln Ser Asp Thr Ser Gly Pro
    1205                1210                1215

Val Thr Thr Ser Lys Ser Ser Ser Leu Gly Ser Phe Tyr His Leu
    1220                1225                1230

Pro Ser Tyr Leu Lys Leu His Asp Val Leu Lys Ala Thr His Ala
    1235                1240                1245

Asn Tyr Lys Val Thr Leu Asp Leu Gln Asn Ser Thr Glu Lys Phe
    1250                1255                1260

Gly Gly Phe Leu Arg Ser Ala Leu Asp Val Leu Ser Gln Ile Leu
    1265                1270                1275

Glu Leu Ala Thr Leu Gln Asp Ile Gly Lys Cys Val Glu Glu Ile
    1280                1285                1290

Leu Gly Tyr Leu Lys Ser Cys Phe Ser Arg Glu Pro Met Met Ala
    1295                1300                1305

Thr Val Cys Val Gln Gln Leu Leu Lys Thr Leu Phe Gly Thr Asn
    1310                1315                1320

Leu Ala Ser Gln Phe Asp Gly Leu Ser Ser Asn Pro Ser Lys Ser
    1325                1330                1335

Gln Gly Arg Ala Gln Arg Leu Gly Ser Ser Ser Val Arg Pro Gly
    1340                1345                1350

Leu Tyr His Tyr Cys Phe Met Ala Pro Tyr Thr His Phe Thr Gln
    1355                1360                1365

Ala Leu Ala Asp Ala Ser Leu Arg Asn Met Val Gln Ala Glu Gln
    1370                1375                1380

Glu Asn Asp Thr Ser Gly Trp Phe Asp Val Leu Gln Lys Val Ser
    1385                1390                1395

Thr Gln Leu Lys Thr Asn Leu Thr Ser Val Thr Lys Asn Arg Ala
    1400                1405                1410

Asp Lys Asn Ala Ile His Asn His Ile Arg Leu Phe Glu Pro Leu
    1415                1420                1425

Val Ile Lys Ala Leu Lys Gln Tyr Thr Thr Thr Thr Cys Val Gln
    1430                1435                1440

Leu Gln Lys Gln Val Leu Asp Leu Leu Ala Gln Leu Val Gln Leu
    1445                1450                1455

Arg Val Asn Tyr Cys Leu Leu Asp Ser Asp Gln Val Phe Ile Gly
    1460                1465                1470

Phe Val Leu Lys Gln Phe Glu Tyr Ile Glu Val Gly Gln Phe Arg
    1475                1480                1485

Glu Ser Glu Ala Ile Ile Pro Asn Ile Phe Phe Phe Leu Val Leu
    1490                1495                1500

Leu Ser Tyr Glu Arg Tyr His Ser Lys Gln Ile Ile Gly Ile Pro
    1505                1510                1515

Lys Ile Ile Gln Leu Cys Asp Gly Ile Met Ala Ser Gly Arg Lys
    1520                1525                1530

Ala Val Thr His Ala Ile Pro Ala Leu Gln Pro Ile Val His Asp
    1535                1540                1545

Leu Phe Val Leu Arg Gly Thr Asn Lys Ala Asp Ala Gly Lys Glu
    1550                1555                1560

Leu Glu Thr Gln Lys Glu Val Val Val Ser Met Leu Leu Arg Leu
    1565                1570                1575

Ile Gln Tyr His Gln Val Leu Glu Met Phe Ile Leu Val Leu Gln
    1580                1585                1590

Gln Cys His Lys Glu Asn Glu Asp Lys Trp Lys Arg Leu Ser Arg
    1595                1600                1605

Gln Ile Ala Asp Ile Ile Leu Pro Met Leu Ala Lys Gln Gln Met
    1610                1615                1620

His Ile Asp Ser His Glu Ala Leu Gly Val Leu Asn Thr Leu Phe
    1625                1630                1635

Glu Ile Leu Ala Pro Ser Ser Leu Arg Pro Val Asp Met Leu Leu

1640                    1645                    1650

Arg Ser  Met Phe Val Thr Pro  Asn Thr Met Ala Ser  Val Ser Thr
    1655                    1660                    1665

Val Gln  Leu Trp Ile Ser Gly  Ile Leu Ala Ile Leu  Arg Val Leu
    1670                    1675                    1680

Ile Ser  Gln Ser Thr Glu Asp  Ile Val Leu Ser Arg  Ile Gln Glu
    1685                    1690                    1695

Leu Ser  Phe Ser Pro Tyr Leu  Ile Ser Cys Thr Val  Ile Asn Arg
    1700                    1705                    1710

Leu Arg  Asp Gly Asp Ser Thr  Ser Thr Leu Glu Glu  His Ser Glu
    1715                    1720                    1725

Gly Lys  Gln Ile Lys Asn Leu  Pro Glu Glu Thr Phe  Ser Arg Phe
    1730                    1735                    1740

Leu Leu  Gln Leu Val Gly Ile  Leu Leu Glu Asp Ile  Val Thr Lys
    1745                    1750                    1755

Gln Leu  Lys Val Glu Met Ser  Glu Gln Gln His Thr  Phe Tyr Cys
    1760                    1765                    1770

Gln Glu  Leu Gly Thr Leu Leu  Met Cys Leu Ile His  Ile Phe Lys
    1775                    1780                    1785

Ser Gly  Met Phe Arg Arg Ile  Thr Ala Ala Ala Thr  Arg Leu Phe
    1790                    1795                    1800

Arg Ser  Asp Gly Cys Gly Gly  Ser Phe Tyr Thr Leu  Asp Ser Leu
    1805                    1810                    1815

Asn Leu  Arg Ala Arg Ser Met  Ile Thr Thr His Pro  Ala Leu Val
    1820                    1825                    1830

Leu Leu  Trp Cys Gln Ile Leu  Leu Leu Val Asn His  Thr Asp Tyr
    1835                    1840                    1845

Arg Trp  Trp Ala Glu Val Gln  Gln Thr Pro Lys Arg  His Ser Leu
    1850                    1855                    1860

Ser Ser  Thr Lys Leu Leu Ser  Pro Gln Met Ser Gly  Glu Glu Glu
    1865                    1870                    1875

Asp Ser  Asp Leu Ala Ala Lys  Leu Gly Met Cys Asn  Arg Glu Ile
    1880                    1885                    1890

Val Arg  Arg Gly Ala Leu Ile  Leu Phe Cys Asp Tyr  Val Cys Gln
    1895                    1900                    1905

Asn Leu His Asp Ser Glu His Leu Thr Trp Leu Ile Val Asn His
    1910                1915                1920

Ile Gln Asp Leu Ile Ser Leu Ser His Glu Pro Pro Val Gln Asp
    1925                1930                1935

Phe Ile Ser Ala Val His Arg Asn Ser Ala Ala Ser Gly Leu Phe
    1940                1945                1950

Ile Gln Ala Ile Gln Ser Arg Cys Glu Asn Leu Ser Thr Pro Thr
    1955                1960                1965

Met Leu Lys Lys Thr Leu Gln Cys Leu Glu Gly Ile His Leu Ser
    1970                1975                1980

Gln Ser Gly Ala Val Leu Thr Leu Tyr Val Asp Arg Leu Leu Cys
    1985                1990                1995

Thr Pro Phe Arg Val Leu Ala Arg Met Val Asp Ile Leu Ala Cys
    2000                2005                2010

Arg Arg Val Glu Met Leu Leu Ala Ala Asn Leu Gln Ser Ser Met
    2015                2020                2025

Ala Gln Leu Pro Met Glu Glu Leu Asn Arg Ile Gln Glu Tyr Leu
    2030                2035                2040

Gln Ser Ser Gly Leu Ala Gln Arg His Gln Arg Leu Tyr Ser Leu
    2045                2050                2055

Leu Asp Arg Phe Arg Leu Ser Thr Met Gln Asp Ser Leu Ser Pro
    2060                2065                2070

Ser Pro Pro Val Ser Ser His Pro Leu Asp Gly Asp Gly His Val
    2075                2080                2085

Ser Leu Glu Thr Val Ser Pro Asp Lys Asp Trp Tyr Val His Leu
    2090                2095                2100

Val Lys Ser Gln Cys Trp Thr Arg Ser Asp Ser Ala Leu Leu Glu
    2105                2110                2115

Gly Ala Glu Leu Val Asn Arg Ile Pro Ala Glu Asp Met Asn Ala
    2120                2125                2130

Phe Met Met Asn Ser Glu Phe Asn Leu Ser Leu Leu Ala Pro Cys
    2135                2140                2145

Leu Ser Leu Gly Met Ser Glu Ile Ser Gly Gly Gln Lys Ser Ala
    2150                2155                2160

Leu Phe Glu Ala Ala Arg Glu Val Thr Leu Ala Arg Val Ser Gly
    2165                2170                2175

Thr Val Gln Gln Leu Pro Ala Val His His Val Phe Gln Pro Glu
    2180                2185                2190

Leu Pro Ala Glu Pro Ala Ala Tyr Trp Ser Lys Leu Asn Asp Leu
    2195                2200                2205

Phe Gly Asp Ala Ala Leu Tyr Gln Ser Leu Pro Thr Leu Ala Arg
    2210                2215                2220

Ala Leu Ala Gln Tyr Leu Val Val Val Ser Lys Leu Pro Ser His
    2225                2230                2235

Leu His Leu Pro Pro Glu Lys Glu Lys Asp Ile Val Lys Phe Val
    2240                2245                2250

Val Ala Thr Leu Glu Ala Leu Ser Trp His Leu Ile His Glu Gln
    2255                2260                2265

Ile Pro Leu Ser Leu Asp Leu Gln Ala Gly Leu Asp Cys Cys Cys
    2270                2275                2280

Leu Ala Leu Gln Leu Pro Gly Leu Trp Ser Val Val Ser Ser Thr
    2285                2290                2295

Glu Phe Val Thr His Ala Cys Ser Leu Ile Tyr Cys Val His Phe
    2300                2305                2310

Ile Leu Glu Ala Val Ala Val Gln Pro Gly Glu Gln Leu Leu Ser
    2315                2320                2325

Pro Glu Arg Arg Thr Asn Thr Pro Lys Ala Ile Ser Glu Glu Glu
    2330                2335                2340

Glu Glu Val Asp Pro Asn Thr Gln Asn Pro Lys Tyr Ile Thr Ala
    2345                2350                2355

Ala Cys Glu Met Val Ala Glu Met Val Glu Ser Leu Gln Ser Val
    2360                2365                2370

Leu Ala Leu Gly His Lys Arg Asn Ser Gly Val Pro Ala Phe Leu
    2375                2380                2385

Thr Pro Leu Leu Arg Asn Ile Ile Ile Ser Leu Ala Arg Leu Pro
    2390                2395                2400

Leu Val Asn Ser Tyr Thr Arg Val Pro Pro Leu Val Trp Lys Leu
    2405                2410                2415

Gly Trp Ser Pro Lys Pro Gly Gly Asp Phe Gly Thr Ala Phe Pro
    2420                2425                2430

Glu Ile Pro Val Glu Phe Leu Gln Glu Lys Glu Val Phe Lys Glu
    2435                2440                2445

Phe Ile Tyr Arg Ile Asn Thr Leu Gly Trp Thr Ser Arg Thr Gln
    2450                2455                2460

Phe Glu Glu Thr Trp Ala Thr Leu Leu Gly Val Leu Val Thr Gln
    2465                2470                2475

Pro Leu Val Met Glu Gln Glu Glu Ser Pro Pro Glu Glu Asp Thr
    2480                2485                2490

Glu Arg Thr Gln Ile Asn Val Leu Ala Val Gln Ala Ile Thr Ser
    2495                2500                2505

Leu Val Leu Ser Ala Met Thr Val Pro Val Ala Gly Asn Pro Ala
    2510                2515                2520

Val Ser Cys Leu Glu Gln Gln Pro Arg Asn Lys Pro Leu Lys Ala
    2525                2530                2535

Leu Asp Thr Arg Phe Gly Arg Lys Leu Ser Ile Ile Arg Gly Ile
    2540                2545                2550

Val Glu Gln Glu Ile Gln Ala Met Val Ser Lys Arg Glu Asn Ile
    2555                2560                2565

Ala Thr His His Leu Tyr Gln Ala Trp Asp Pro Val Pro Ser Leu
    2570                2575                2580

Ser Pro Ala Thr Thr Gly Ala Leu Ile Ser His Glu Lys Leu Leu
    2585                2590                2595

Leu Gln Ile Asn Pro Glu Arg Glu Leu Gly Ser Met Ser Tyr Lys
    2600                2605                2610

Leu Gly Gln Val Ser Ile His Ser Val Trp Leu Gly Asn Ser Ile
    2615                2620                2625

Thr Pro Leu Arg Glu Glu Glu Trp Asp Glu Glu Glu Glu Glu Glu
    2630                2635                2640

Ala Asp Ala Pro Ala Pro Ser Ser Pro Pro Thr Ser Pro Val Asn
    2645                2650                2655

Ser Arg Lys His Arg Ala Gly Val Asp Ile His Ser Cys Ser Gln
    2660                2665                2670

Phe Leu Leu Glu Leu Tyr Ser Arg Trp Ile Leu Pro Ser Ser Ser
    2675                2680                2685

Ala Arg Arg Thr Pro Ala Ile Leu Ile Ser Glu Val Val Arg Ser
    2690                2695                2700

Leu Leu Val Val Ser Asp Leu Phe Thr Glu Arg Asn Gln Phe Glu

2705                    2710                    2715

Leu Met Tyr Val Thr Leu Thr Glu Leu Arg Arg Val His Pro Ser
    2720                    2725                    2730

Glu Asp Glu Ile Leu Ala Gln Tyr Leu Val Pro Ala Thr Cys Lys
    2735                    2740                    2745

Ala Ala Ala Val Leu Gly Met Asp Lys Ala Val Ala Glu Pro Val
    2750                    2755                    2760

Ser Arg Leu Leu Glu Ser Thr Leu Arg Ser Ser His Leu Pro Ser
    2765                    2770                    2775

Arg Val Gly Ala Leu His Gly Val Leu Tyr Val Leu Glu Cys Asp
    2780                    2785                    2790

Leu Leu Asp Asp Thr Ala Lys Gln Leu Ile Pro Val Ile Ser Asp
    2795                    2800                    2805

Tyr Leu Leu Ser Asn Leu Lys Gly Ile Ala His Cys Val Asn Ile
    2810                    2815                    2820

His Ser Gln Gln His Val Leu Val Met Cys Ala Thr Ala Phe Tyr
    2825                    2830                    2835

Leu Ile Glu Asn Tyr Pro Leu Asp Val Gly Pro Glu Phe Ser Ala
    2840                    2845                    2850

Ser Ile Ile Gln Met Cys Gly Val Met Leu Ser Gly Ser Glu Glu
    2855                    2860                    2865

Ser Thr Pro Ser Ile Ile Tyr His Cys Ala Leu Arg Gly Leu Glu
    2870                    2875                    2880

Arg Leu Leu Leu Ser Glu Gln Leu Ser Arg Leu Asp Ala Glu Ser
    2885                    2890                    2895

Leu Val Lys Leu Ser Val Asp Arg Val Asn Val His Ser Pro His
    2900                    2905                    2910

Arg Ala Met Ala Ala Leu Gly Leu Met Leu Thr Cys Met Tyr Thr
    2915                    2920                    2925

Gly Lys Glu Lys Val Ser Pro Gly Arg Thr Ser Asp Pro Asn Pro
    2930                    2935                    2940

Ala Ala Pro Asp Ser Glu Ser Val Ile Val Ala Met Glu Arg Val
    2945                    2950                    2955

Ser Val Leu Phe Asp Arg Ile Arg Lys Gly Phe Pro Cys Glu Ala
    2960                    2965                    2970

Arg Val Val Ala Arg Ile Leu Pro Gln Phe Leu Asp Asp Phe Phe
    2975                2980                2985

Pro Pro Gln Asp Ile Met Asn Lys Val Ile Gly Glu Phe Leu Ser
    2990                2995                3000

Asn Gln Gln Pro Tyr Pro Gln Phe Met Ala Thr Val Val Tyr Lys
    3005                3010                3015

Val Phe Gln Thr Leu His Ser Thr Gly Gln Ser Ser Met Val Arg
    3020                3025                3030

Asp Trp Val Met Leu Ser Leu Ser Asn Phe Thr Gln Arg Ala Pro
    3035                3040                3045

Val Ala Met Ala Thr Trp Ser Leu Ser Cys Phe Phe Val Ser Ala
    3050                3055                3060

Ser Thr Ser Pro Trp Val Ala Ala Ile Leu Pro His Val Ile Ser
    3065                3070                3075

Arg Met Gly Lys Leu Glu Gln Val Asp Val Asn Leu Phe Cys Leu
    3080                3085                3090

Val Ala Thr Asp Phe Tyr Arg His Gln Ile Glu Glu Glu Leu Asp
    3095                3100                3105

Arg Arg Ala Phe Gln Ser Val Leu Glu Val Val Ala Ala Pro Gly
    3110                3115                3120

Ser Pro Tyr His Arg Leu Leu Thr Cys Leu Arg Asn Val His Lys
    3125                3130                3135

Val Thr Thr Cys
    3140

<210> 14
<211> 148
<212> PRT
<213> Homo sapiens

<400> 14

<210>  15
<211>  43
<212>  PRT
<213>  Homo sapiens

<400>  15

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40

## Beschreibung

Die Erfindung betrifft ein Verfahren zur Ermittlung von Indikatoren zur Bestimmung von Krankheiten (Krankheitsindikatoren), in denen Aggregate fehlgefalteter Proteine eine Rolle spielen, sowie ein Verfahren zur selektiven Quantifizierung und/oder Charakterisierung dieser Krankheitsindikatoren.

Pathologische Aggregate aus körpereigenen Proteinen, wie z.B. Oligomere oder Fibrillen, treten in vielen neurodegenerativen Erkrankungen auf.

Amyloidosen sind Krankheiten, die durch extrazelluläre Ablagerungen von unlöslichen, fehlgefalteten Proteinen charakterisiert sind. Diese Aggregate liegen in der Regel als Fasern, sog. Beta-Fibrillen vor. Der Begriff Amyloid (=stärkeähnlich) leitet sich von den spektralphotometrischen Gemeinsamkeiten von Kongorot-gefärbten Fibrillen und Stärke ab. Die Ätiologie der Amyloidosen kann sowohl hereditär als auch spontan sein. Auch treten einige Amyloidosen als Sekundärerkrankungen auf.

Bei der Alzheimer-Krankheit (AD, Alzheimersche Demenz, lateinisch = Morbus Alzheimer) treten A-Beta-Aggregate auf. AD gehört neben Morbus Parkinson zu einer heterogenen Gruppe klinischer Zustände, deren gemeinsames Kriterium in vielen Fällen (aber nicht ausschließlich) extrazelluläre, systemische oder lokale Ablagerungen eines jeweils spezifischen Proteins ist, meist in beta-Faltblattreichen Konformationen.

Die Amyloid-Beta-Peptid-Ablagerungen (oder Peptid-Fibrillen) stellen allerdings lediglich das Endstadium eines Prozesses dar. Pathologische Hauptmerkmale der AD sind die Bildung von senilen oder amyloiden Plaques, bestehend aus dem A-Beta-Peptid, und zusätzlichen neurofibrillären Ablagerungen aus dem Tau-Protein. Das Vorläufer-Protein des A-Beta-Peptids, APP, ist in der Zellwand von Neuronen lokalisiert. Durch proteolytischen Abbau und nachträgliche Modifikation entstehen daraus A-Beta-Fragmente unterschiedlicher Länge und Art, wie z.B. A-Beta 1-40, A-Beta 1-42 oder pGluA-Beta 3-42. Monomere A-Beta-Peptide entstehen während des gesamten Lebens auch im gesunden Organismus.

Gemäß der Amyloid-Kaskaden-Hypothese aus den 1990er Jahren sind die A-Beta-Ablagerungen in Form von Plaques die Auslöser der Krankheitssymptome. In den letzten Jahren weisen jedoch unterschiedliche Studien darauf hin, dass besonders die kleinen, frei diffundierenden A-Beta-Oligomere die größte Toxizität unter allen A-Beta-Spezies besitzen und für die Entstehung und den Fortschritt der AD verantwortlich sind. Somit sind Aggregate des A-Beta-Peptids unmittelbar mit der AD-Pathogenese verknüpft und möglicherweise als Biomarker für AD zu verwenden (Wang-Dietrich et al., J. Alzheimer's Disease 34, 2013, 985-994). Eine Unterstützung der Diagnose durch ein weiteres Routineverfahren wäre allerdings hilfreich, insbesondere zur Abgrenzung von anderen Krankheiten basierend auf körpereigenen fehlgefalteten Proteinen.

Protein-Aggregate werden ferner in WO 2005/018424 A1, WO 2008/070229 A2, DE 10 2006 010 647 A1, Bannach et al. (Plosone, May 2012, Vol. 7, issue 5, e36620), WO 2010/003227 A1, DE 10 2011 057 021 A1 und WO 2013/092952 A2 (veröffentlich 27.06.2013) erwähnt.

Die AA-Amyloidose ist eine recht seltene Folgeerkrankung bei chronisch-entzündlicher Darmerkrankung, wie Colitis ulcerosa und Morbus Crohn. Bei den überwiegend männlichen Patienten sind Niere, Gastrointestinaltrakt, Milz, Leber, Pankreas und Herz betroffen (in abnehmender Häufigkeit). Die AA-Amyloidose manifestiert sich binnen 10 bis 20 Jahren nach Erscheinung der Grundkrankheit. Die klinischen Symptome umfassen nephrotische Symtome, Proteinurie oder Kardiomyopathie. Die systemische AA-Amyloidose betrifft auch Nervenzellen. Zur Therpaie der AA-Amyloidose werden eine Reihe von Medikamenten eingesetzt, darunter Alkylanzien, Methotrexat, TNF-α-Blocker, u.a. Auch kann bei terminaler Niereninsuffizienz eine Dialyse oder eine Nierentransplantation erfolgen. Therapeutisches Hauptziel ist es aber, die Synthese des Serum-Amyloid-A -Vorläuferproteins zu inhibieren.

Die AL-Amyloidose kann sich systemisch oder lokal begrenzt manifestieren. Ursächlich zeigt sich eine Erkrankung klonaler Plasmazellen. Dabei werden amyloidogene Leichtketten produziert, welche Fibrillen bilden und sich im Interstitium verschiedener Organe ablagern. Am häufigsten betroffen sind Niere, Herz, Darm, Leber und autonomes Nervensystem. Typisch ist auch das Auftreten einer Polyneuropathie.

AApoAI ist eine proteinöse Komponente von high-density-lipoprotein (HDL), dessen natürliche Funktion es ist, wasserunlösliche Cholesterol und Phospholipide im Blut zu transportieren. Eine Mutation im N-terminalem Bereich, verleiht AApoAI amyloidogene Eigenschaften. Die hereditäre AApoAI-Amyloidose manifestiert sich in einer Polyneuropathie der Füße und Hände. Auch Niere, Herz, Leber (AP und γ-GT erhöht), peripheres Nervensystem, Haut, Larynx und Hoden sind in abnehmender Häufigkeit betroffen.

AApoAII ist eine proteinöse Komponente von high-density-lipoprotein (HDL), dessen natürliche Funktion es ist, wasserunlösliches Cholesterol und Phospholipide im Blut zu transportieren. Eine Mutation des Stop-Codons des ApoAII-Gens, verleiht AApoAII amyloidogene Eigenschaften. Die hereditäre AApoAII-Amyloidose manifestiert sich vorwiegend in Niere und Herz.

Die ATTR-Amyloidose ist die häufigste hereditäre Amyloidose. Sie wird autosomal dominant vererbt. Verantwortlich zeigt sich eine Mutation des Gens für Transthyretin, einem Transportprotein, welches mit dem Ritinol-bindenem Protein und Thyroxin im Plasma assoziiert ist. Die häufigste Mutation Val30Met verleiht dem Protein amyloidogene und somit pathogene Eigenschaften. Im Verlaufe der ATTR-Amyloidose kommt es zu Amyloidablagerungen im peripheren und autonomen Nervensystem. Leitsymptome umfassen erektile Dysfunktion, gastroinestinale Beschwerden, Malabsorption und restriktive Kardiomypathie. Unbehandelt ist die Prognose der ATTR-Amyloidose schlecht. Neben der symptomatischen Behandlung verbleibt als therapeutische Option lediglich eine Lebertransplantation.

In der klinischen Psychiatrie werden bislang alle Erkrankungen einschließlich der Schizophrenie oder der Depression rein klinisch in Form eines Interviews mithilfe eines Fragenkatalogs und ggf. durch Bewegungs- oder Verhaltenstests diagnostiziert. Aufgrund der Unkenntnis über die biologischen Grundlagen von mentalen Erkrankungen stehen keine Blut- oder Liquor-basierten diagnostischen Tests zur Verfügung, wie sie etwa bei neurologischen Erkrankungen verwendet werden, um die klinische Diagnose zu untermauern.

Diese Situation wird besonders von der pharmazeutischen forschenden Industrie als sehr unbefriedigend empfunden, weil die Abwesenheit objektiver und eindeutig quantifizierbarer Tests letztlich quantitatives Monitoring potenziell wirksamer Therapien verhindert.

Durch die Entdeckung von ursächlich wirkenden Krankheitsgenen wie dem DISC1 ist es nun erstmals möglich eine biologische Diagnostik zu entwickeln. DISC1 entwickelt bei einer Subgruppe chronisch mentaler Erkrankungen, wie der Schizophrenie und der rekurrierenden Depression submikroskopische Proteinaggregate im Gehirn. Mithilfe eigens entwickelter Antikörper lassen sich diese im post mortem Gehirn nachweisen.

Die ALS ist eine chronische, schnell voranschreitende Erkrankung des zentralen Nervensystems. Betroffen ist vor allem die willentliche Steuerung der Skelettmuskulatur. Im Verlauf der Krankheit kommt es zu einer Degeneration der für die Muskelbewegung verantwortlichen Motorneuronen. Dies führt zu einer erheblichen Beeinträchtigung bis hin zum völligen Ausfall von Körperbewegungen und -reflexen. Die durchschnittliche Lebenserwartung nach Eintritt der initialen Symptome beträgt nur drei Jahre. Für die ALS sind verschiedene Ätiologien beschrieben worden. Die häufigste Form der ALS ist sporadischer Natur. Es existieren aber auch familiär vererbte ALS-Fälle. Am häufigsten dabei ist eine Mutation der Superoxid Dismutase-1 (SOD1). Neuere Daten legen nahe, dass eine Hexanukleotid-Expansion des Gens C9orf72 ursächlich sowohl für ALS, als auch für Frontotemporaldemenz ist.

Die Diagnose der ALS kann erst spät im Verlauf der Krankheit gestellt werden. Der diagnostische Verzug beläuft sich in der Regel auf ein Jahr nach Einsetzen der ersten Symptome. Die Diagnose der ALS umfasst derzeit eine klinische Begutachtung, eine elektrophysiologische Untersuchung (Elektromyopgraphie) und eine neuropathologische Analyse (Biopsie). Zusätzlich kann eine Liquorpunktion vorgenommen und ein Differentialblutbild erstellt werden. Schließlich muss ALS gegenüber anderen Krankheiten differentialdiagnostisch abgegrenzt werden.

Pathologisches Merkmal der ALS sind unter anderem cytoplasmatische Einschlüsse des Proteins FUS. Bei Zelldegeneration werden diese Aggregate freigesetzt und können so in die Peripherie gelangen, wo sie einer Diagnostik zugänglich sind. Auch andere neurodegenerative Krankheiten zeigen Ablagerungen von FUS-Aggregaten, darunter die Frontotemporale Lobäre Degeneration (FTLD), deren Leitsymptome Veränderungen der Persönlichkeit, des Sozialverhaltens und der sprachlichen Fähigkeiten sind.

Ein alternatives pathologisches Merkmal der ALS sind Ablagerungen des Proteins SOD1 in den Motorneuronen. Bei Zelldegeneration werden diese Aggregate freigesetzt und können so in die Peripherie gelangen, wo sie einer Diagnostik zugänglich sind.

Ein alternatives pathologisches Merkmal der ALS sind cytoplasmatische Einschlüsse des ubiquitinierten Proteins TDP-43. Bei Zelldegeneration werden diese Aggregate freigesetzt und können so in die Peripherie gelangen, wo sie einer Diagnostik zugänglich sind.

Auch andere neurodegenerative Krankheiten gehen einher mit Ablagerungen von TDP-43-Aggregaten, darunter die frontotemporale lobäre Degeneration (FTLD), deren Leitsymptome Veränderungen der Persönlichkeit, des Sozialverhaltens und der sprachlichen Fähigkeiten sind. Neuere Studien sehen auch einen Zusammenhang zwischen TDP-43-Ablagerungen und Chronischer Traumatischer Enzephalopathie (CTE). Von CTE betroffen sind vor allem Leistungssportler und Soldaten, die wiederholt Schädel-Hirn-Traumata erleiden.

Auch bei Diabetes Mellitus Typ II (DMT2) spielen aggregierte, körpereigene Proteine eine Rolle. So findet man z.B. im Pankreas von DMT2-Patienten Plaques, die aus vorwiegend aus dem Insel-Amyloid-Polypeptid (IAPP) bestehen. IAPP ist ein Peptidhormon, das in den beta-Zellen des Pankreas produziert wird und gemeinsam mit Insulin sekretiert wird. Es wird spekuliert, dass die Aggregation des IAPP im Zusammenhang mit dem fortschreitenden Verlust der Insel-beta-Zellen steht.

Bisher sind keine ex vivo Nachweissysteme für IAPP-Oligomere -oder Aggregate in Geweben oder Körperflüssigkeiten im Routineeinsatz.

Die Parkinson-Krankheit ist eine degenerative neurologische Erkrankung. Die Leitsymptome umfassen Muskelstarre, verlangsamte Bewegung, Muskelzittern und Haltungsinstabilität. Darüber hinaus sind fakultative Begleitsymptome, wie z.B. psychische Störungen, beschrieben. Im Verlauf der Krankheit kommt es zum Absterben Dopamin-produzierender Neuronen in der Substantia nigra. Histopathologisch zeigen sich im Gehirngewebe von Parkinson-Patienten zytoplasmatische Einschlüsse, sog. Lewy-Körper, die vorwiegend aus α-Synuclein, Ubiquitin und anderen Proteinablagerungen bestehen.

Tauopathien sind eine Gruppe von neurodegenerativen Krankheiten, die durch eine Anreicherung des Tau-Proteins im Gehirn charakterisiert sind. Durch Hyperphosphorylierung kann das Mikrotubuli-assoziierte Tau in unlösliche Aggregate, sog. neurofibrilläre Bündel, überführt werden. Die Tauopathien umfassen verschiedene Krankheitsbilder wie Morbus Alzheimer, Kortikobasale Degeneration, Silberkornkrankheit, Morbus Pick, FTLD-MAPT (früher: FTDP-17, Frontotemporale Demenz und Parkinsonismus des Chromosom 17), progressive supranukleäre Blickparese, neurofibrilläre Tangle-Demenz, Tauopathie mit glialen Einschlüssen sowie unklassifizierbare Tauopathien. Neuere Studien zählen auch die chronische traumatische Enzephalopathie zu den Tauopathien. Manche der o.a. Krankheiten wie z.B. FTLD-MAPT sind erblich bedingt. Die Ursachen der meisten Tauopathien sind aber noch unbekannt.

Die klinische Präsentation der Tauopathien ist äußerst vielfältig und umfasst Bewegungsstörungen (L-Dopa-sensitive oder atypische Parkinsonsyndrome, Akinesie mit "freezing", supranukleäre Blickparese bis hin zu kortikobasalen Syndromen), Verhaltens- und Sprachstörungen und psychiatrische Symptome wie Aggressivität, psychotisches Zustandsbild und Depression.

Die Huntington-Krankheit ist eine genetische neurodegenerative Krankheit, die die Muskelkoordination betrifft und zur Verminderung der kognitiven Fähigkeiten und psychiatrischen Problemen führt. Die Krankheit betrifft sowohl Männer als auch Frauen und wird durch eine autosomal-dominante Mutation des Huntingtin-Gens bedingt. Eine Expansion eines CAG-Triplet-Repeats führt zu einer Verlängerung des Polyglutamin-Trakts im maturierten Protein, wodurch dieses verstärkt zur Fehlfaltung und Aggregation neigt.

Der familiäre viszeralen Amyloidose liegt eine Mutation des Gens für Lysozym zugrunde, welche dem maturierten Protein amyloidogene Eigenschaften verleiht. Amyloidablagerungen finden sich systemisch in einer Vielzahl von Organen.

Krankheitsindikatoren, also Indikatoren zur Erkennung von Krankheiten, auch Biomarker genannt, wurden schon immer zur Diagnose von Krankheiten herangezogen. Bekannte Beispiele für Biomarker sind z.B. der Gehalt von Glukose im Urin als Hinweis auf Diabetes Mellitus oder der Gehalt an HCG im Urin in Schwangerschaftstests.

Um eine immer größer und älter werdende Bevölkerung medizinisch zu versorgen, ist die Identifikation von neuen, spezifischen Biomarkern notwendig, die eine sichere Diagnose gewährleisteten. Eine hohe Spezifität ist insbesondere bei Krankheiten mit ähnlichen Symptomen und Krankheitsbildern notwendig, um hier eine erfolgversprechende Behandlung durchführen zu können. Ferner sollen die Krankheitsindikatoren Risikopersonen schon in einem frühen Krankheitsstadium oder im Falle eines Rückfalls möglichst bald identifizieren. Die Biomarker sollen in leicht zugänglichem Biomaterial vorhanden und identifizierbar sein und einen schnellen Nachweis ermöglichen. Dadurch ist es nicht nur möglich, den Verlauf der Krankheit, sondern auch die Wirkung der Behandlung und der Vorbeugung zu verfolgen.

Aufgabe der vorliegenden Erfindung war es, solche Biomarker zur Verfügung zu stellen, insbesondere für Krankheiten, die Aggregate fehlgefalteter Proteine aufweisen.

Weitere Aufgabe war es, ein schnelles und sicheres Verfahren zu deren selektiven Quantifizierung und/oder Charakterisierung zur Verfügung zu stellen. Insbesondere sollen die Krankheitsindikatoren für den klinischen Routineeinsatz geeignet sein. Dies bedeutet, dass sie in einem, bevorzugt Schnelltest und ggf. mittels Standards charakterisiert werden können, so dass vergleichbare und unabhängige Werte bestimmt werden.

Ferner sollten die o.g. Anforderungen an Biomarker erfüllt werden und die entsprechenden, gegenüber dem Stand der Technik verbesserten, Möglichkeiten und Werkzeuge zur Verfügung gestellt werden.

Die Diagnose der AA-Amyloidose umfasst eine Begutachtung der klinischen Präsentation. Problematisch ist dabei aber die differentialdiagnostische Abgrenzung gegenüber sehr vielen anderen Krankheitsbildern. Präklinisch ist die Diagnose nur durch den histologischen Nachweis des AA-Amyloids mittels Kongorotfärbung möglich. Dazu ist aber eine Fettaspirations-Biopsie erforderlich. Eine Routinediagnostik, welche die amyloiden Proteinaggregate als Biomarker in Körperflüssigkeiten nachweist, ist gegenwärtig nicht etabliert.

Die AA-Amyloidose ist gekennzeichnet durch Fehlfaltung des Serum-Amyloid-A - Vorläuferproteins (AA). Die AA-Aggregate könnten somit ein direkter Biomarker für die AA-Amyloidose sein. Der diagnostische Nachweis stellt indes eine technische Herausforderung dar, da AA auch im gesunden Organismus ubiquitär produziert wird. Daher muss das Verfahren insensitiv gegenüber einem hohen Überschuss an normal gefaltetem, monomerem AA sein.

Die Diagnose der AL-Amyloidose umfasst eine Begutachtung der klinischen Präsentation, welche sich aber stark variabel zeigen kann. Problematisch ist darüber hinaus die differentialdiagnostische Abgrenzung gegenüber sehr vielen anderen Krankheitsbildern. Goldstandard ist der histologische Nachweis des AL-Amyloids mittels Kongorotfärbung. Zusätzlich sollte die Immunhistochemie durchgeführt werden. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AL-Amyloidose ist gekennzeichnet durch Fehlfaltung des IgG-light-chain-Vorläuferproteins (AL). Die AL-Aggregate könnten somit ein direkter Biomarker für die AL-Amyloidose sein. Der diagnostische Nachweis stellt indes eine technische Herausforderung dar, da AL auch im gesunden Organismus ubiquitär produziert wird. Daher muss das Verfahren insensitiv gegenüber einem hohen Überschuss an normal gefaltetem, monomerem AL sein.

Die Diagnose der AApoAI-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des AApoAI-Amyloids mittels Kongorotfärbung. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AApoAI-Amyloidose ist gekennzeichnet durch Fehlfaltung des Apolipoprotein AI-Vorläufers (AApoAI). Die AApoAI-Aggregate könnten somit ein direkter Biomarker für die AApoAI-Amyloidose sein. Der diagnostische Nachweis stellt indes eine technische Herausforderung dar, da AApoAI auch im gesunden Organismus ubiquitär produziert wird. Daher muss das Verfahren insensitiv gegenüber einem hohen Überschuss an normal gefaltetem, monomerem AApoAI sein.

Die Diagnose der AApoAII-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des AApoAII-Amyloids mittels Kongorotfärbung. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AApoAII-Amyloidose ist gekennzeichnet durch Fehlfaltung des Apolipoprotein AII-Vorläufers (AApoAII). Die AApoAII-Aggregate könnten somit ein direkter Biomarker für die AApoAII-Amyloidose sein. Der diagnostische Nachweis stellt indes eine technische Herausforderung dar, da AApoAII auch im gesunden Organismus ubiquitär produziert wird. Daher muss das Verfahren insensitiv gegenüber einem hohen Überschuss an normal gefaltetem, monomerem AApoAII sein.

Die Diagnose der ATTR-Amyloidose umfasst eine Begutachtung der klinischen Präsentation. Problematisch ist dabei aber die differentialdiagnostische Abgrenzung gegenüber sehr vielen anderen Krankheitsbildern. Zudem sind Zeitpunkt der Erstsymptomatik, Verlauf der Erkrankung und Phänotyp regional unterschiedlich. Goldstandard ist der histologische Nachweis des ATTR-Amyloids mittels Kongorotfärbung. Dazu ist aber eine aufwendige Biopsie erforderlich. Eine Routinediagnostik, welche die amyloiden Proteinaggregate als Biomarker in Körperflüssigkeiten nachweist, ist gegenwärtig nicht etabliert.

Die ATTR-Amyloidose ist gekennzeichnet durch Fehlfaltung des Transthyretin-Vorläuferproteins (TTR). Die ATTR-Aggregate könnten somit ein direkter Biomarker für die ATTR-Amyloidose sein. Der diagnostische Nachweis stellt indes eine technische Herausforderung dar, da ATTR auch im gesunden Organismus ubiquitär produziert wird. Daher muss das Verfahren insensitiv gegenüber einem hohen Überschuss an normal gefaltetem, monomerem ATTR sein.

Schizophrene Psychosen können gegenwärtig erst drei bis fünf Jahre nach dem Auftreten der ersten Symptome diagnostiziert werden. Eine Diagnostik, die auf dem Nachweis eines Biomarkers beruht, ist für chronisch mentale Erkrankungen wie die Schizophrenie bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Neuere Studien sehen einen Zusammenhang zwischen chronisch mentalen Erkrankungen, wie der Schizophrenie oder der rekurrierenden Depression, und aggregiertem DISC1-Protein (disrupted-in-schizophrenia-1). Diese DISC1-Aggregate könnten ein direkter Biomarker für Schizophrenie, rekurrierende Depression und andere DISC1opathien sein. Der diagnostische Nachweis stellt indes eine technische Herausforderung dar, da DISC1 auch im gesunden Organismus produziert wird. Daher muss das Verfahren insensitiv gegenüber einem hohen Überschuss an normal gefaltetem, monomerem DISC1 sein.

Eine Diagnose, die auf dem Nachweis eines Biomarkers beruht, ist sowohl für ALS als auch für FTLD gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Bei der Amyotrophen Lateralsklerose (ALS) und der Frontotemporalen Lobärdegeneration (FTLD) wurden cytoplasmatische Ablagerungen des RNA-Bindeproteins FUS (Fused in Sarcoma) beschrieben. Diese FUS-Aggregate könnten ein direkter Biomarker für die beschriebenen Krankheitsbilder sein. Der diagnostische Nachweis stellt indes eine technische Herausforderung dar, da FUS auch im gesunden Organismus ubiquitär produziert wird. Daher muss das Verfahren insensitiv gegenüber einem hohen Überschuss an normal gefaltetem, monomerem FUS sein.

Gegenwärtig gilt der Blutzuckerspiegel als allgemein anerkannter Biomarker für DMT2, womit eine sichere Diagnose für Patienten erreicht werden kann. Wird der Blutzuckerspiegel mit Hilfe des Insulins reguliert, ist ein Nachweis von DMT2 nicht mehr möglich. Dies hat eine besondere Bedeutung für die Analyse von Blutkonserven, die für Transfusionen eingesetzt werden, da diese möglicherweise ein Gefährdungspotenzial für die Empfänger bedeuten. Auch für eine ursächliche Therapieentwicklung von DMT2 ist die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Seit einigen Jahren wird über einen Zusammenhang zwischen Diabetes Mellitus Typ 2 (DMT2) und der Alzheimerschen Demenz (AD) berichtet. Studien zufolge sind rund 80 % der AD-Patienten an DMT2 erkrankt bzw. weisen einen höheren Glucose-Gehalt im Blut auf. Zudem zeigen die DMT2 und AD Mäuse ähnliche verhaltensbezogene, kognitive und vaskuläre Anomalien. Beide Krankheiten weisen ähnliche pathologische Merkmale auf: die Entstehung von amyloiden Plaques im Pankreas (DMT2) oder im Gehirn (AD) infolge von Proteinfaltung und darauf folgenden Fibrillenbildung des IAPP- bzw. Aß-Peptids. Insbesondere die kleinen Aggregate gelten als hauptsächlicher Verursacher für die Entstehung und den Fortschritt von DMT2 und AD. IAPP-Aggregate könnten daher ein direkter Biomarker für DMT2 sein. Der diagnostische Nachweis stellt indes eine technische Herausforderung dar, da IAPP auch im gesunden Organismus ubiquitär produziert wird. Daher muss das Verfahren insensitiv gegenüber einem hohen Überschuss an normal gefaltetem, monomerem IAPP sein.

Eine Diagnose, die auf dem Nachweis eines Biomarkers beruht, ist für ALS gegenwärtig nicht im Routineeinsatz. Ebenso gibt es zum jetzigen Zeitpunkt keine ursächliche Therapie.

Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Bei der Amyotrophen Lateralsklerose (ALS) wurden Ablagerungen der Superoxiddismutase 1 (SOD1) beschrieben. Diese SOD1-Aggregate könnten ein direkter Biomarker für ALS sein. Der diagnostische Nachweis stellt indes eine technische Herausforderung dar, da SOD1 auch im gesunden Organismus ubiquitär produziert wird. Daher muss das Verfahren insensitiv gegenüber einem hohen Überschuss an normal gefaltetem, monomerem SOD1 sein.

Die Diagnose der Parkinson-Krankheit wird durch den sog. L-Dopa-Test geführt. Dabei wird dem Patienten L-Dopa in Kombination mit einem Decarboxylasehemmer verabreicht. Führt diese Behandlung zu einer deutlichen Verbesserung der Symptome, kann mit sehr hoher Wahrscheinlichkeit die Parkinson-Krankheit diagnostiziert werden.

Eine Diagnostik, die auf einem minimalinvasiven Nachweis eines Biomarkers in Körperflüssigkeiten beruht, ist für die Parkinson-Krankheit aber bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Die Parkinson-Krankheit ist gekennzeichnet durch pathologische Akkumulation von aggregiertem α-Synuclein. Diese α-Synuclein-Aggregate könnten ein direkter Biomarker für die Parkinson-Krankheit und anderer α-Synucleinopathien sein. Der diagnostische Nachweis stellt indes eine technische Herausforderung dar, da α-Synuclein auch im gesunden Organismus produziert wird. Daher muss das Verfahren insensitiv gegenüber einem hohen Überschuss an normal gefaltetem, monomerem α-Synuclein sein.

Der neuropathologischen Phänotyp der verschiedenen Tauopathien wird post mortem identifiziert. Eine Schwierigkeit stellt die erhebliche Überschneidung verschiedener neuropathologischer Veränderungen dar. Darüber hinaus liegen diverse Pathologien oft in Kombination vor, was die Notwendigkeit der Biomarkerforschung unterstreicht. Eine Diagnose, die auf dem Nachweis eines Biomarkers in Körperflüssigkeiten beruht, ist für Tauopathien gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Tauopathien sind gekennzeichnet durch pathologische Ablagerungen des Tau-Proteins. Diese Tau-Aggregate könnten ein direkter Biomarker für verschiedene Krankheitsbilder sein. Der diagnostische Nachweis stellt indes eine technische Herausforderung dar, da Tau auch im gesunden Organismus ubiquitär produziert wird. Daher muss das Verfahren insensitiv gegenüber einem hohen Überschuss an normal gefaltetem, monomerem Tau sein.

Eine Diagnose, die auf dem Nachweis eines Biomarkers beruht, ist sowohl für ALS als auch für FTLD und CTE gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Bei der Amyotrophen Lateralsklerose (ALS) und der Frontotemporalen Lobärdegeneration (FTLD) wurden Ablagerungen des TAR DNA-Bindeproteins 43 (TDP-43) beschrieben. Diese TDP-43-Aggregate könnten ein direkter Biomarker für die beschriebenen Krankheitsbilder sein. Der diagnostische Nachweis stellt indes eine technische Herausforderung dar, da TDP-43 auch im gesunden Organismus ubiquitär produziert wird. Daher muss das Verfahren insensitiv gegenüber einem hohen Überschuss an normal gefaltetem, monomerem TDP-43 sein.

Bei Verdacht auf die Huntington-Krankheit kann ein Gentest durchgeführt werden, der die Krankheit mit Sicherheit feststellen kann. Eine Diagnostik, die auf einem minimalinvasiven Nachweis eines Biomarkers in Körperflüssigkeiten beruht, ist für die Huntington-Krankheit aber bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für die genetisch bedingte Huntington-Krankheit, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Die Huntington-Krankheit ist gekennzeichnet durch pathologische Akkumulation von aggregiertem Huntingtin. Diese Huntingtin-Aggregate könnten ein direkter Biomarker für die Huntington-Krankheit sein. Der diagnostische Nachweis stellt indes eine technische Herausforderung dar, da Huntingtin auch im gesunden Organismus produziert wird. Daher muss das Verfahren insensitiv gegenüber einem hohen Überschuss an normal gefaltetem, monomerem Huntingtin sein.

Die Diagnose der ALys-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des ALys-Amyloids mittels Kongorotfärbung und Immunohistochemie. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die familiäre viszerale Amyloidose ist gekennzeichnet durch Fehlfaltung des Lysozym-Proteinvorläufers (ALys). Die ALys-Aggregate könnten somit ein direkter Biomarker für die ALys-Amyloidose sein. Der diagnostische Nachweis stellt indes eine technische Herausforderung dar, da Lysozym auch im gesunden Organismus ubiquitär produziert wird. Daher muss das Verfahren insensitiv gegenüber einem hohen Überschuss an normal gefaltetem, monomerem ALys sein.

Aufgabe der vorliegenden Erfindung war es auch, ein Routineverfahren zur quantitativen Bestimmung von Krankheitsindikatoren zur Verfügung zu stellen, insbesondere von mehreren Krankheitsindikatoren nebeneinander in derselben Probe. Ferner sollte das Verfahren die Möglichkeit bieten, die Probe mit nur wenigen oder bevorzugt keinen Aufarbeitungsschritten zu analysieren.

Eine Vielzahl von neurodegenerativen Krankheiten und Amyloidosen ist gekennzeichnet durch das simultane Auftreten verschiedener aggregierter Proteinspezies. Als Beispiel sei die Alzheimer'sche Demenz angeführt, bei der sowohl Ablagerungen von Amyloid beta als aus Tau zu beobachten sind (Jucker and Walker, Annals of neurology 70, 532-540, 2011; Spillantini and Goedert, Lancet neurology 12, 609-622, 2013; die unter Bezugnahme eingeschlossen sind). Bei der frontotemporalen Lobärdegeneration (FTLD) koexistieren Tau und TDP-43 als pathologische Proteinaggregate (Mackenzie et al., Acta neuropathologica 119, 1-4, 2010). TDP-43-Pathologie manifestiert sich wiederum auch in der Amyotrophen Lateralsklerose und Chronischen Traumatischen Enzephalopathie (Blennow et al., Neuron 76, 886-899, 2012; Blokhuis et al., Acta neuropathologica 125, 777-794, 2013; die unter Bezugnahme eingeschlossen sind).

Das Verfahren muss eine ausreichende Spezifität sicherstellen, um unterschiedliche Aggregat-Typen nebeneinander und/oder gleichzeitig zu untersuchen. Somit war es auch Aufgabe der Erfindung, ein Verfahren mit gleichzeitig hoher Spezifität für die Untersuchung unterschiedlicher Aggregat-Typen zu bieten, und so Störungen der Untersuchung eines Aggregat-Typs durch einen zweiten Aggregat-Typ auszuschließen. Es sollen auch Aggregat-Mischformen, bestehend aus verschiedenen Protein-Monomeren untersucht werden können.

Die bisher beschriebenen Protein-Aggregate der o.g. Krankheiten stellen lediglich ein pathologisches Hauptmerkmal des jeweiligen Krankheitsbildes dar. Ihre Verwendung als Biomarker ist nicht sichergestellt.

Diese Aufgabe wird gelöst durch ein Verfahren zur qualitativen und/oder quantitativen Bestimmung von Krankheitsindikatoren dadurch gekennzeichnet, dass eine Probe auf mindestens einen Aggregat-Typ, bevorzugt mindestens zwei Aggregat-Typen körpereigener fehlgefalteter Proteine untersucht wird, umfassend folgende Schritte:
a) Auftragen der zu untersuchenden Probe auf ein Substrat,
b) Hinzufügen von für die Detektion gekennzeichneten Sonden, die durch spezifische Bindung an das jeweilige Aggregat dieses markieren und
c) Detektion der markierten Aggregate,
   wobei
   - Schritt b) vor Schritt a) durchgeführt werden kann und
   - die Krankheit ausgewählt ist aus der Gruppe bestehend aus:
      AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer'sche Demenz und
   - falls A-beta-Aggregate in der Probe bestimmt wurden, die Probe auf mindestens einen weiteren Aggregat-Typ eines körpereigenen fehlgefalteten Proteins untersucht wird.

In einer Ausführung ist das Verfahren dadurch gekennzeichnet, dass die Probe ohne weitere Aufarbeitung und/oder Behandlung nach der Untersuchung auf einen ersten Aggregat-Typ auf mindestens einen weiteren, zum Beispiel zweiten unterschiedlichen Aggregat-Typen körpereigener fehlgefalteter Proteine untersucht wird.

In einer weiteren Ausführung ist das Verfahren dadurch gekennzeichnet, dass die Probe auf mindestens zwei unterschiedliche Aggregat-Typen in einem Verfahrensschritt untersucht wird.

In einer weiteren Ausführung ist das Verfahren dadurch gekennzeichnet, dass die Probe auf mindestens zwei unterschiedliche Aggregat-Typen auf demselben Substrat untersucht wird.

In einer weiteren Ausführung ist das Verfahren, dadurch gekennzeichnet, dass der Aggregat-Typ körpereigener fehlgefalteter Proteine ausgewählt wird aus der Gruppe bestehend aus Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer weiteren Ausführung ist das Verfahren dadurch gekennzeichnet, dass die Detektion in Schritt c) mittels einem Verfahren mit ortsaufgelöstem Signal, bevorzugt sFIDA-Verfahren erfolgt.

In einer weiteren Ausführung ist das Verfahren dadurch gekennzeichnet, dass vor Schritt a) eine Immobilisierung von Fängermolekülen auf dem Substrat erfolgt.

In einer weiteren Ausführung ist das Verfahren dadurch gekennzeichnet, dass die Fängermoleküle und/oder die Sonde mit Fluoreszenzfarbstoffen gekennzeichnet sind.

In einer weiteren Ausführung ist das Verfahren dadurch gekennzeichnet, dass die Fängermoleküle und/oder die Sonden spezifische Anti-Körper gegen ein Epitop der Proteine aufweisen, die die Aggregate bilden.

In einer weiteren Ausführung ist das Verfahren dadurch gekennzeichnet, dass ein interner oder externer Standard eingesetzt wird.

In einer weiteren Ausführung ist das Verfahren dadurch gekennzeichnet, dass die Fängermoleküle, Sonden und/oder Standards ein Polymer umfassen, aufgebaut aus Monomer-Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

Gegenstand der Erfindung sind auch Standards zur selektiven Quantifizierung und/oder Charakterisierung der oben genannten Krankheitsindikatoren enthaltend ein Polymer eingesetzt wird, aufgebaut aus Monomer-Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

Die erfindungsgemäßen Standard-Moleküle werden zur Inaktivierung der jeweiligen Protein-Aggregate verwendet, zum Beispiel durch Binden oder Zerstören der Protein-Aggregate, oder prophylaktisch, um die Bildung der Protein-Aggregate zu verhindern. Somit sind Gegenstand der Erfindung Standards zur Verwendung in der Medizin, zur Verwendung als Arzneimittel und/oder zur Verwendung in therapeutischen, diagnostischen und/oder chirurgischen Verfahren.

Weiteren Gegenstand is ein Substrat umfassend genau definierte Bereiche mit Fängermolekülen für die oben genannten Krankheitsindikatoren und/oder Aggregate.

Ferner ist Gegenstand der Erfindung eine Differential-Diagnose zur Bestimmung von Krankheiten die Aggregate fehllgefalteter Proteine aufweisen, umfassend folgende Schritte:
i) quantitativen Bestimmung von Krankheitsindikatoren bestimmt in dem erfindungsgemäßen Verfahren;
ii) Vergleich dieser Daten mit den Normwerten;
iii) Feststellen einer signifikant unterschiedlichen Menge Krankheitsindikatoren bei diesem Vergleich;
iv) Zuordnung der Abweichung zu einer Krankheit ausgewählt aus der Gruppe bestehend aus von AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer-Demenz.

Unter dem Begriff "Aggregat-Typ" ist im Sinne der Erfindung ein Aggregat einer bestimmten, definierten Zusammensetzung körpereigener fehlgefalteter Proteine zu verstehen. In einer Alternative ist ein Aggregat-Typ aus gleichen Peptiden (Monomeren) zusammengesetzt (aufgebaut), d.h. aus Peptiden derselben Aminosäure-Sequenz oder aus unterschiedlichen Homologen ein und desselben Peptids. In anderen Alternative ist ein Aggregat-Typ aus unterschiedlichen Peptiden (Monomeren) zusammengesetzt, aufgebaut, d.h. aus Peptiden unterschiedlicher Aminosäure-Sequenz oder aus Homologen der unterschiedlichen Peptide. In einer Probe können auch beide Alternativen vorhanden sein.

In einer Ausführung der vorliegenden Erfindung bedeutet die "qualitative Bestimmung von Krankheitsindikatoren" die Identifikation von neuen, spezifischen Biomarkern. Mit anderen Worten, es werden Krankheitsindikatoren identifiziert und neu definiert. Erfindungsgemäß sind Krankheitsindikatoren durch die Anwesenheit, gegebenenfalls in einer bestimmten Konzentration, und/oder Abwesenheit jeweils eines oder mehrerer Aggregate körpereigener fehlgefalteter Proteine definiert.

In einer weiteren Ausführung bedeutet die "qualitative Bestimmung von Krankheitsindikatoren" die Überprüfung bzw. Bestimmung der An- und/oder Abwesenheit dieser Biomarker.

Im Sinne der vorliegenden Erfindung bedeutet die "quantitative Bestimmung von Krankheitsindikatoren" in einer ersten Alternative die Bestimmung der Konzentration der Biomarker. In einer zweiten Alternative wird Zusammensetzung, Größe und/oder Form der Biomarker bestimmt. Es können auch beide Alternativen, bevorzugt gleichzeitig, in einem Verfahrensschritt) durchgeführt werden.

Die quantitative Bestimmung von Krankheitsindikatoren ist der selektiven Quantifizierung und/oder Charakterisierung von Aggregat-Typen als Biomarker äquivalent. Sobald die Merkmale eines Krankheitsindikators definiert sind, erfolgt seine quantitative Bestimmung, d.h. seine selektiven Quantifizierung: also Konzentrationsbestimmung die die Information über An- bzw. Abwesenheit enthält; und/oder seine Charakterisierung: also Bestimmung der Zusammensetzung, Größe und/oder Form.

In einer Alternative der vorliegenden Erfindung wird eine Probe auf mindestens zwei Aggregat-Typen körpereigener fehlgefalteter Proteine untersucht. In einer Alternative der vorliegenden Erfindung wird eine Probe auf mindestens drei Aggregat-Typen körpereigener fehlgefalteter Proteine untersucht. In einer Alternative der vorliegenden Erfindung wird eine Probe auf mindestens vier Aggregat-Typen körpereigener fehlgefalteter Proteine untersucht. In einer Alternative der vorliegenden Erfindung wird eine Probe auf mindestens fünf Aggregat-Typen körpereigener fehlgefalteter Proteine untersucht. In einer Alternative der vorliegenden Erfindung wird eine Probe auf mindestens sechs Aggregat-Typen körpereigener fehlgefalteter Proteine untersucht. In einer Alternative der vorliegenden Erfindung wird eine Probe auf mindestens sieben Aggregat-Typen körpereigener fehlgefalteter Proteine untersucht. In einer Alternative der vorliegenden Erfindung wird eine Probe auf mindestens acht Aggregat-Typen körpereigener fehlgefalteter Proteine untersucht. In einer Alternative der vorliegenden Erfindung wird eine Probe auf mindestens neun Aggregat-Typen körpereigener fehlgefalteter Proteine untersucht. In einer Alternative der vorliegenden Erfindung wird eine Probe auf mindestens zehn Aggregat-Typen körpereigener fehlgefalteter Proteine untersucht. In einer Alternative der vorliegenden Erfindung wird eine Probe auf mindestens 11, 12 ,13 ,14 ,15 oder mehr Aggregat-Typen körpereigener fehlgefalteter Proteine untersucht.

In einer Ausführung der vorliegenden Erfindung sind die Aggregate aus Peptiden bzw. Monomeren der SEQ ID NO: 1-15 und/oder Homologen davon aufgebaut mit folgender Zuordnung:
Serum-Amyloid-A-Protein-Aggregate : SEQ ID NO: 1; IgG-light-chain-Aggregate : SEQ ID NO: 2; AapoAI-Aggregate : SEQ ID NO: 3; AapoAII-Aggregate : SEQ ID NO: 4; ATTR-Aggregate : SEQ ID NO: 5; DISC1-Aggregate : SEQ ID NO: 6; FUS-Aggregate : SEQ ID NO: 7; IAPP-Aggregate : SEQ ID NO: 8; SOD1-Aggregate : SEQ ID NO: 9; α-Synuclein-Aggregate : SEQ ID NO: 10; Tau-Aggregate : SEQ ID NO: 11; TDP-43-Aggregate : SEQ ID NO: 12; Huntingtin-Aggregate : SEQ ID NO: 13; Lysozym-Aggregate : SEQ ID NO: 14 und A-beta-Aggregate : SEQ ID NO: 15.

In einer Ausführung wird mindestens auf einen Aggregat-Typ aus der oben genannten Gruppe und einen weiteren Aggregat-Typ untersucht.

Es können jedoch auch gemischte Aggregate vorliegen, aufgebaut aus mindestens zwei unterschiedlichen Proteinen, Monomeren, sogenannte Aggregat-Mischformen.

Der Begriff " Aggregat-Typ körpereigener fehlgefalteter Proteine untersuchen" bzw. Synonyme wie zum Beispiel "Untersuchung" davon bedeuten im Sinne der Erfindung die qualitative und/oder quantitative Bestimmung (Analyse) des jeweiligen Aggregat-Typs. Bei der qualitativen Bestimmung wird die An- bzw. Abwesenheit bestimmt während bei der quantitativen Bestimmung Konzentration, Zusammensetzung, Größe und/oder Form der bestimmt wird.

Der Begriff "ohne weitere Aufarbeitung und/oder Behandlung" der Probe zur Untersuchung auf einen zweiten Aggregat-Typ bedeutet erfindungsgemäß in einer Alternative, dass zwischen zwei Untersuchungen auf unterschiedliche Aggregat-Typen keine Aufarbeitung und/oder Behandlung der Probe stattfindet. In einer anderen Alternative findet in der Probe nach Untersuchung eines ersten oder eines weiteren Aggregat-Typs keine chemischen Reaktionen statt. Bevorzugt erfolgt die erste und zweite und gegebenenfalls jede weiter Untersuchung in einem Probenaliquot. Die Probe erfährt gegebenenfalls lediglich physikalische oder mechanische Einwirkungen wie zum Beispiel Temperaturänderung oder Pipettieren. In einer weiteren oder zusätzlichen Alternative erfährt die Probe keine physikalische oder mechanische Einwirkungen. In einer weiteren oder zusätzlichen Alternative hat die Probe zur Untersuchung eines zweiten Aggregat-Typs dieselbe chemische Zusammensetzung wie die Probe zur Untersuchung des ersten Aggregat-Typs, gegebenenfalls sind dieselben Verbindungen anwesend jedoch in geänderten Konzentrationen. Außerdem enthält die Probe zur Untersuchung eines zweiten Aggregat-Typs gegebenenfalls die Fängermoleküle, Sonden und/oder Standards zur Untersuchung des ersten Aggregat-Typs sowie gegebenenfalls die entsprechenden Verbindungen aus Aggregat, Fängermolekül und/oder Sonde. In einer weiteren oder zusätzlichen Alternative werden die Sonden für die Untersuchung auf einen zweiten Aggregat-Typ erst nach der Untersuchung auf den ersten Aggregat-Typ dazu gegeben, so dass als einzige chemische Reaktion die Bindung der spezifischen Sonde für den zweiten Aggregat-Typ an dieses Aggregat ist, sofern in der Probe enthalten.

Analog gilt der oben beschriebene Sachverhalt für die Untersuchung eines dritten, vierten, fünften, sechsten, siebenten, achten, neunten, zehnten und/oder weiteren Aggregat-Typs.

In einer Ausführung betrifft die Erfindung ein Verfahren zur Bestimmung von Indikatoren zur Bestimmung von Krankheiten die Aggregate fehlgefalteter Proteine aufweisen, umfassend folgende Schritte:
a) Auftragen der zu untersuchenden Probe auf ein Substrat,
b) Hinzufügen von für die Detektion gekennzeichneten Sonden, die durch spezifische Bindung an das jeweilige Aggregat dieses markieren und
c) Detektion der markierten Aggregate,
   wobei Schritt b) vor Schritt a) durchgeführt werden kann und
   die Krankheit ausgewählt ist aus der Gruppe bestehend aus::
      AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit und/oder Familiäre Viszerale Amyloidose.

Bei den Biomarkern, auch Krankheitsindikatoren genannt, handelt es sich erfindungsgemäß in Anlehnung an die Definition des NIH um Parameter, mit denen sich eine Eigenschaft objektiv messen lässt, um sie als Indikator für normale biologische, pathogene Prozesse oder pharmakologische Antworten auf eine therapeutische Intervention heranzuziehen. Die erfindungsgemäß bestimmten und verwendeten Krankheitsinhibitoren sind mit physikalischen und/oder chemischen Verfahren qualitativ und quantitativ zu charakterisieren.

Aggregate im Sinne der vorliegenden Erfindung sind
- Partikel, die aus mehreren, bevorzugt gleichen Bausteinen bestehen, die nicht kovalent miteinander verbunden sind und/oder
- nicht-kovalente Zusammenlagerungen mehrerer Monomere sowie
- Hetero-Aggregate, "Co-Aggregate", also Aggregate aus unterschiedlichen Monomeren.

Für das erfindungsgemäße Verfahren werden dem menschlichen oder tierischen Körper Proben entnommen und diesem nicht wieder zugeführt.

In einer Alternative des Verfahrens werden zur Ermittlung, insbesondere zur qualitativen Bestimmung von Krankheitsindikatoren Proben mit den potentiellen Biomarkern verglichen mit Proben gesund und/oder krank diagnostizierten Individuen. Diese Diagnose beruht z.B. auf einer Begutachtung der klinischen Präsentation oder Auswertung anderer Diagnoseverfahren. Durch den Vergleich der Menge der detektieren Aggregate ist eine Aussage über deren Eignung als Biomarker möglich.

In einer Alternative handelt es sich um Krankheitsindikatoren geeignet für den klinischen Routineeinsatz. Biomarker sind erfindungsgemäß für den klinischen Routineeinsatz geeignet sofern wissenschaftliche nachweisbare und reproduzierbare Beweise vorliegen, insbesondere auf Basis von physikalisch und/oder chemischen Methoden.

Gegenstand der Erfindung ist auch ein Verfahren zu selektiven Quantifizierung und/oder Charakterisierung der Krankheitsindikatoren umfassend folgende Schritte:
a) Auftragen der zu untersuchenden Probe auf ein Substrat,
b) Hinzufügen von für die Detektion gekennzeichneten Sonden, die durch spezifische Bindung an das jeweilige Aggregat dieses markieren und
c) Detektion der markierten Aggregate,
   wobei Schritt b) vor Schritt a) durchgeführt werden kann und
   die Krankheit ausgewählt ist aus der Gruppe bestehend aus: I
      AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit und/oder Familiäre Viszerale Amyloidose.

Gegenstand der vorliegenden Erfindung ist somit auch ein Verfahren zur Bestimmung der Zusammensetzung, Größe und/oder Form von Aggregaten. Hierbei werden die oben genannten und beschriebenen Verfahrensschritte eingesetzt.

In einer Variante der vorliegenden Erfindung erfolgt eine Vorbehandlung der Probe, bevorzugt nach einem oder mehreren der folgenden Verfahren:
- Erhitzen (auf eine Temperatur bis zum Siedepunkt der Probe)
- Ein oder mehrere Gefrierauftauzyklen,
- Verdünnen mit Wasser oder Puffer,
- Behandlung mit Enzymen, zum Beispiel Proteasen, Nuklease, Lipasen,
- Zentrifugieren,
- Präzipitation,
- Kompetition mit Sonden, um eventuell vorhandene Antikörper zu verdrängen.

Als Substrat wird erfindungsgemäß ein Material gewählt, das eine möglichst geringe, unspezifische Bindungskapazität, insbesondere bezüglich der Aggregate fehlgefalteter Proteine besitzt.

In einer Ausführung der vorliegenden Erfindung wird ein Substrat aus Glas gewählt.

Das Substrat kann mit hydrophilen Stoffen, bevorzugt Poly-D-Lysin, Polyethylenglykol (PEG), bevorzugt heterobiofunktionales Polyethylenglykol (NHS-PEG-COOH) oder Dextran, insbesondere Dextran, bevorzugt Carboxymethyl-Dextran (CMD), beschichtet werden.

In einer Ausführung der vorliegenden Erfindung wird die Glasoberfläche hydroxyliert und anschließend mit Aminogruppen aktiviert.

Zur Vorbereitung des Substrats auf die Beschichtung werden ein oder mehrere der folgenden Schritte durchgeführt:
- Waschen eines Substrats aus Glas bzw. eines Glasträgers im Ultraschallbad oder Plasma-cleaner, alternativ dazu in 5 M NaOH mindestens 3 Stunden inkubieren,
- Spülen mit Wasser und anschließendem Trocknen unter Stickstoff,
- Eintauchen in eine Lösung aus konzentrierter Schwefelsäure und Wasserstoffperoxid im Verhältnis 3:1 für die Aktivierung der Hydroxylgruppen,
- Spülen mit Wasser bis zu einer neutralen pH, anschließend mit Ethanol und Trocknen unter Stickstoffatmosphäre,
- Eintauchen in eine Lösung mit 3-Aminopropyltrietoxysilan (APTES) (1 - 7 %) in trockenem Toluol oder einer Lösung von Ethanolamin,
- Spülen mit Aceton oder DMSO und Wasser und Trocknen unter Stickstoffatmosphäre.

Für die Beschichtung mit Dextran, bevorzugt Carboxymethyl-Dextran (CMD), wird das Substrat mit einer wässrigen Lösung von CMD (in einer Konzentration von 10 mg/ml oder 20 mg/ml) und gegebenenfalls N-Ethyl-N-(3-Dimethylaminpropyl) Carbodiimid (EDC), (200 mM) und N-Hydroxysukzinimid (NHS), (50 mM) inkubiert und anschließend gewaschen.

Das Carboxymethyl-Dextran ist in einer Variante kovalent an die Glasoberfläche gebunden, die zunächst hydroxyliert und anschließend mit Amingruppen aktiviert wurde, wie oben beschrieben.

Als Substrat können auch Mikrotiterplatten, bevorzugt mit Glasboden, eingesetzt werden. Da bei der Verwendung von Polystryrolrahmen der Einsatz von konzentrierter Schwefelsäure nicht möglich ist, erfolgt die Aktivierung der Glasoberfläche in einer Ausführungsvariante der Erfindung analog Janissen et al. (Colloids Surf B Biointerfaces, 2009, 71(2), 200-207).

In einer Ausführung der vorliegenden Erfindung wird die Glasoberfläche mit Natronlauge (5 M) für 15 Minuten bei Raumtemperatur inkubiert, dreimal mit Wasser gespült, mit Salzsäure versetzt und erneut 15 Minuten bei Raumtemperatur inkubiert. Nach dreimaligem Waschen mit Wasser und zweimaligem Waschen mit Ethanol wird das Substrat (Glasoberfläche) unter Stickstoff-Atmosphäre getrocknet.

Zur Erzeugung von Aminogruppen auf der Glasoberfläche wird diese mit Ethanolamin (5,6 M) über Nacht bei Raumtemperatur inkubiert. Anschließend wird das Substrat dreimal mit DMSO, zweimal mit Ethanol gespült und unter Stickstoff-Atmosphäre getrocknet.

Heterobiofunktionales Polyethylenglykol (NHS-PEG-COOH, MW 3.400 Da) wird zu 50 mM in DMSO bei 70 °C für 1 min gelöst, auf Raumtemperatur abgekühlt und auf 2% Triethylamin eingestellt. Mit dieser Lösung wird mindestens eine Stunde bei Raumtemperatur inkubiert. Die Lösung wird aus den entfernt, und die Glasoberfläche dreimal mit Wasser gewaschen.

Zur Aktivierung der PEG-Beschichtung werden NHS und EDC zu jeweils 100 mM in MES-Puffer (0,1 M, pH 5) gelöst und im Verhältnis 1:1 zu Endkonzentrationen von jeweils 50 mM gemischt. Mit dieser Lösung wird das Substrat 30-60 Minuten inkubiert. Nach Entfernen der Lösung wird dreimal mit MES-Puffer (0,1 M, pH 5) gewaschen.

Der Fänger-Antikörper wird auf 30 ng/µl in PBS verdünnt und damit das Substart für 1-3 Stunden bei Raumtemperatur inkubiert. Anschließend wird die Lösung entfernt und dreimal mit PBST, dann dreimal mit PBS gewaschen.

3% BSA wurde zuvor bei 100.000 g (1 Stunde bei 4°C) zentrifugiert. Mit dem Überstand wird wird das Substart für eine Stunde bei Raumtemperatur inkubiert. Die BSA-Lösung wird entfernt und dreimal mit PBST gewaschen.

Die Probe (z.B. Aggregate aus rekombinantem Protein und natürliche Patientenprobe) wird - sofern erforderlich - in PBS verdünnt und aufgetragen. Das Substart mit Probe wird bei 1.000g für eine Stunde bei 4 °C in einem Ausschwingrotor zentrifugiert. Der Überstand wird entfernt und die Glasoberfläche dreimal mit PBST und dreimal mit PBS gewaschen.

Als Detektionssonden werden fluoreszenzmarkierte Antikörper eingesetzt. Diese werden auf 1-2 ng/µl in PBS verdünnt und mit 1.5% BSA versetzt. Die Ansätze werden bei 100.000 g für eine Stunde bei 4 °C zentrifugiert. Der Überstands wird auf das Substart aufgetragen und für 1-2 Stunden bei Raumtemperatur inkubiert. Anschließend wird die Lösung entfernt und fünfmal mit PBST und fünfmal mit PBS gewaschen.

In einer Ausführung der vorliegenden Erfindung erfolgt das Auftragen der Probe unmittelbar auf dem Substrat (nichtbeschichteten Substrat), gegebenenfalls durch kovalente Bindung auf der gegebenenfalls aktivierten Oberfläche des Substrats.

In einer Alternative der vorliegenden Erfindung werden auf dem Substrat Fängermoleküle immobilisiert, um die Aggregate fehlgefalteter, körpereigener Proteine zu fangen und fixieren.

Bevorzugt werden als Fängermoleküle Antikörper der körpereigenen Proteine, die aggregieren, eingesetzt. Die Antikörper binden spezifisch ein Epitop der körpereigenen Proteine, die aggregieren.

In einer Alternative sind die Fängermoleküle kovalent an das Substrat gebunden.

In einer weiteren Alternative sind die Fängermoleküle kovalent mit der Beschichtung, bevorzugt Dextranschicht verbunden.

In einer Ausführung der vorliegenden Erfindung werden die Fängermoleküle (Antikörper), gegebenenfalls nach einer Aktivierung des mit CMD beschichteten Trägers durch eine Mischung aus EDC/NHS (200 bzw. 50 mM), auf dem Substrat immobilisiert.

Verbleibende Carboxylat-Endgruppen, an die keine Fängermoleküle gebunden wurden, können deaktiviert werden.

Zur Deaktivierung dieser Carboxylat-Endgruppen auf dem CMD-Spacer wird Ethanolamin in DMSO verwendet. Vor dem Auftrag der Proben werden die Substrate bzw. Träger mit PBS gespült.

Die zu vermessende Probe wird auf dem so präparierten Substrat inkubiert.

In einer Ausführung die umfassen die Fängermoleküle und/oder die Sonden ein Polymer, aufgebaut aus Monomer-Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

In diesem Sinne beschreibt hier der Begriff "Monomer-Sequenz" einen Teilbereich der einzelnen Proteine die Aggregate fehlgefalteter Proteine bilden.

Bevorzugt handelt es sich bei den Monomer-Sequenzen um Bereiche die ein Epitop bilden an das Anti-Körper binden, die spezifisch an die Proteine binden, die Aggregate bilden.

In einer Ausführung der Erfindung sind als körpereigene Proteine die Polypeptide der Sequenzen mit SEQ ID Nr. 1 - 15 sowie Homologe davon, die insbesondere durch genetische Mutationen gebildet wurden, zu verstehen. Bei den körpereigene Proteinen kann es sich auch um Oligomere oder Polymere dieser Peptide handeln. Die SEQ ID NO: 1-15 werden auch als Peptid oder Polypeptid bezeichnet.

Als "Homologe" oder "homologe Sequenzen" ist im Sinne der Erfindung eine Aminosäuresequenz zu verstehen, die eine Identität mit einer Aminosäuresequenz eines Peptids aus einem körpereigenem pathogenen Aggregat oder Oligomeren der fehlgefalteten Proteine oder der fehlgefalteten Proteine, von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch, wenn sie die selbe Aminosäuresequenz besitzen.

In einem weiteren Schritt werden die Aggregate von Sonden markiert, die für eine spätere Detektion gekennzeichnet sind.

In einer Ausführung sind die Fängermoleküle und/oder die Sonde mit Fluoreszenzfarbstoffen gekennzeichnet.

In einer Alternative enthalten Fängermoleküle und/oder die Sonden spezifische Anti-Körper gegen ein Epitop der Proteine, die die Aggregate bilden.

In einer Variante der vorliegenden Erfindung werden als Sonden Antikörper eingesetzt. Fängermoleküle und Sonden können identisch sein.

In einer Ausführung der vorliegenden Erfindung unterscheiden sich Fängermoleküle und Sonden. So können z.B. unterschiedliche Antikörper als Fängermoleküle und Sonden eingesetzt werden.

In einer weiteren Ausführung der vorliegenden Erfindung werden Fängermoleküle und Sonden eingesetzt, die mit Ausnahme der eventuellen Farbstoffmarkierung identisch zueinander sind. In einer Alternative der vorliegenden Erfindung werden verschiedene Sonden eingesetzt, die mit Ausnahme der eventuellen Farbstoffmarkierung identisch zueinander sind. In einer weiteren Alternative der vorliegenden Erfindung werden mindestens 2 oder mehrere unterschiedliche Fängermoleküle und/oder Sonden eingesetzt, die unterschiedliche Antikörper enthalten oder sind und gegebenenfalls auch unterschiedliche Farbstoffmarkierung haben.

Als Fängermoleküle können aber auch unterschiedliche Moleküle eingesetzt werden, wie z.B. unterschiedliche Antikörper oder Moleküle enthaltend Antikörper.

Ebenso können als Sonden mehrere, unterschiedliche Moleküle eingesetzt werden, wie z.B. unterschiedliche Antikörper enthalten oder sind.

Zur späteren Qualitätskontrolle der Oberfläche, zum Beispiel Gleichmäßigkeit der Beschichtung mit Fängermolekülen, können Fängermoleküle, gekennzeichnet mit Fluoreszensfarbstoffen, eingesetzt werden. Hierzu wird bevorzugt ein Farbstoff verwendet, der nicht mit der Detektion interferiert. Dadurch wird eine nachträgliche Kontrolle des Aufbaus möglich sowie eine Normierung der Messergebnisse.

Zur Detektion sind die Sonden so gekennzeichnet, dass sie ein optisch detektierbares Signal aussenden, ausgewählt aus der Gruppe bestehend aus Fluoreszenz-, Biolumineszenz- und Chemolumineszenz-Emission sowie Absorption.

In einer Alternative sind die Sonden mit Farbstoffen gekennzeichnet. Bevorzugt handelt es sich hierbei um Fluoreszenzfarbstoffe.

In einer Ausführung der vorliegenden Erfindung werden mindestens 1, 2, 3, 4, 5, 6 oder mehr unterschiedliche Sonden eingesetzt. Die Sonden können sich sowohl bezüglich ihrer spezifischen Bindung an die Aggregate als auch bezüglich ihrer unterschiedlichen Kennzeichnung, z.B. durch Fluoreszenzfarbstoffe, unterscheiden.

Es können auch Sonden miteinander kombiniert werden, die geeignet sind FRET (Fluorescence Resonance Energy Transfer) als Detektion zu verwenden.

Der Einsatz mehrerer, unterschiedlicher Sonden, die durch unterschiedliche Fluroreszenzfarbstoffe gekennzeichnet sind, erhöht die Spezifität des bei der Messung erhaltenen Korrelationssignals. Zusätzlich wird dadurch auch das Ausblenden von Monomeren, also nicht aggregierten Molekülen möglich. Die Detektion von Monomeren kann insbesondere ausgeschlossen werden, falls Sonde und Fängermolekül identisch sind, oder beide ein überlappendes Epitop erkennen.

Gegenstand der vorliegenden Erfindung ist somit auch die Nutzung oder Verwendung Aggregat-spezifischer, bzw. Oligomer-spezifischer Sonden. Diese binden spezifisch an ein bestimmtes Aggregat, bzw. Oligomer, bevorzugt für die oben genannten Spezies der fehlgefalteten Proteine. Durch die spezifische Bindung an ein bestimmtes Aggregat, bzw. Oligomer kann die Art und/oder Größe sowie der Aufbau des Aggregats, bzw. Oligomers bestimmt werden.

Weiterer Gegenstand der vorliegenden Erfindung sind somit auch Aggregat-spezifische, bzw. Oligomer-spezifische Sonden.

Im Sinne der vorliegenden Erfindung bezeichnet der Begriff Monomer ein Peptid-Molekül, ein einzelnes Protein, das Aggregate fehlgefalteter Proteine bildet. Je nach Herkunfts-Spezies (Mensch und/oder Tier) und Prozessierung kann die genaue Aminosäuresequenz eines Monomeres in Länge und Art variieren. Bevorzugte Monomere sind ausgewählt aus den SEQ ID NO: 1-15 sowie Homologe davon.

Im Sinne der vorliegenden Erfindung bezeichnet der Begriff Oligomere sowohl Aggregate als auch Oligomere und auch kleine, frei diffundierende Oligomere. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomeren oder Vielfachen davon. Dabei können, es müssen aber nicht, alle Monomere in einem Oligomer identisch zueinander sein.

Somit sind unter Aggregaten sowohl Oligomere als auch kleine, frei Oligomere zu verstehen.

In einer weiteren Alternative können als Sonden körpereigene Proteine, gekennzeichnet mit Fluoreszensfarbstoffen, verwendet werden.

Als zu untersuchende Probe können körpereigene Flüssigkeiten oder Gewebe eingesetzt werden. In einer Ausführung der vorliegenden Erfindung wird die Probe ausgewählt aus Liquor (CSF), Blut, Plasma, Urin, Speichel, Schleimhaut und/oder Biopsie-Material . Die Proben können unterschiedliche, dem Fachmann bekannte, Aufbereitungsschritte durchlaufen.

Vorteil der vorliegenden Erfindung ist die Möglichkeit der Bestimmung von Aggregaten in unbehandelten Proben, bevorzugt CSF.

In einer Variante der vorliegenden Erfindung werden interne oder externe Standards eingesetzt.

Solche Standards enthalten oder sind ein Polymer, aufgebaut aus Monomer-Sequenzen, bevorzugt einer der Sequenzen nach SEQ ID NO: 1-15 oder Teilen einer der SEQ ID NO: 1-15, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

Erfindungsgemäß wird eine Monomer-Detektion von körpereigenen Proteinen ausgeschlossen indem im Testsystem drei unterschiedliche bzw. drei unterschiedlich markierte Sonde eingesetzt werden, die an einem ähnlichen bzw. gleichen Epitop binden. Alternativ oder zusätzlich kann die Detektion von Monomeren ausgeschlossen werden indem Signale mit einer niedrigeren Intensität durch ein Intensität-cut-off nicht gewertet werden. Da größere Aggregate mehrere Bindungsstellen für die beiden mit unterschiedlichen markierten Farbstoffen Sonde besitzen, kann eine Monomer-Detektion alternativ oder zusätzlich durch Kreuzkorelation dieser Signale ausgeschlossen werden.

Die Detektion der markierten Aggregate erfolgt durch Scannen oder andere Arten der Oberflächenabbildung. Die Detektion erfolgt bevorzugt mit konfokaler Fluoreszenzmikroskopie, Fluoreszenzkorrelationsspektroskopie (FCS), insbesondere in Kombination mit Kreuzkorrelation und Single-Particie-Immunosolvent Laserscanning-Assay und/oder Laser-Scanning-Mikroskop (LSM). Alternativ erfolgt die Detektion mittels Total Internal Reflection Fluoreszenz Mikroskopie (TIRFM).

Die Detektion wird in einer Alternative der vorliegenden Erfindung mit einem konfokalen Laser-Scanning-Mikroskop durchgeführt.

In einer Ausführung der vorliegenden Erfindung wird ein Laserfokus, wie er z.B. in der Laser-Scanning-Mikroskopie verwendet wird, oder ein FCS (Fluorescence Correlation Spectroscopy System), dazu eingesetzt, sowie die entsprechenden superauflösenden Varianten wie zum Beispiel STED oder SIM. Alternativ dazu kann die Detektion durch ein TIRF-Mikroskop erfolgen, sowie die entsprechenden superauflösenden Varianten davon, wie zum Beispiel STORM, dSTORM.

Demgemäß sind in der Ausführung der Erfindung Verfahren, die auf einem nichtortsaufgelöstem Signal beruhen, wie ELISA oder Sandwich-ELISA, ausgeschlossen.

Bei der Detektion ist eine hohe Ortsauflösung vorteilhaft. In einer Ausführung des erfindungsgemäßen Verfahrens werden dabei so viele Datenpunkte gesammelt, dass die Detektion eines Aggregates vor einem Hintergrundsignal, welches z.B. durch gerätespezifisches Rauschen, andere unspezifische Signale oder unspezifisch gebundene Sonden verursacht wird, ermöglicht. Auf diese Weise werden so viele Werte ausgelesen (Readout-Werte), wie orts-aufgelöste Ereignisse, wie z.B. Pixel, vorhanden sind. Durch die Ortsauflösung wird jedes Ereignis vor dem jeweiligen Hintergrund bestimmt und stellt so einen Vorteil gegenüber ELISA-Verfahren mit nur einem Readout-Wert, wenigen Readout-Werten und/oder ohne ortsaufgelöstem Signal dar.

In einer Alternative werden in dem erfindungsgemäßen Verfahren mehrere unterschiedliche Sonden eingesetzt. Dadurch wird die Information, d.h. die ausgelesenen Werte vervielfacht, da für jeden Punkt, für jedes Aggregat oder für jedes Detektionsereignis eine separate Information erhalten wird in Abhängigkeit der jeweiligen Sonde, die das Signal liefert. So wird für jedes Ereignis die Spezifität des Signals erhöht. Dadurch kann für jedes detektierte Aggregat auch seine Zusammensetzung bestimmt werden, d.h. die Art des Aggregats, also die Zusammensetzung aus Monomeren oder Mischungen daraus.

Die Zahl der unterschiedlichen Sonden wird dabei nur durch die Interferenz der zu verwendenden Fluoreszenzfarbstoffen begrenzt. So können 1, 2, 3, 4 oder mehrere unterschiedliche Sonden-Farbstoff-Kombinationen verwendet werden.

Wesentlich für die Auswertung gemäß dem oben beschriebenen Verfahren, wenn mehr als eine Sonde verwendet wird, sind orts- und zeitaufgelöste Informationen. Dabei kann es sich z.B. um die Art und/oder Intensität der Fluoreszenz handeln. Bei Auswertung dieser Daten für alle eingesetzten und detektierten Sonden wird erfindungsgemäß die Anzahl der Aggregate, deren Form, Größe und/oder deren Zusammensetzung bestimmt. Dabei können Informationen über die Größe der Oligomere direkt oder indirekt erhalten werden, abhängig davon, ob die Partikel kleiner oder größer als die Zeit- oder Ortsauflösung der verwendeten Abbildungsverfahren sind, in einer Ausführung können Algorithmen zur Hintergrundminimierung eingesetzt werden und/oder Intensität-Schwellenwerte angewendet werden.

Als Fluoreszenzfarbstoff können die dem Fachmann bekannten Farbstoffe eingesetzt werden. Alternativ können GFP (Green Fluorescence Protein), Konjugate und/oder Fusionsproteine davon, sowie Quantendots verwendet werden.

Durch die Verwendung von internen oder externen Standards sind Testergebnisse objektiv miteinander vergleichbar und damit aussagekräftig.

In einer Ausführung der vorliegenden Erfindung werden ein interner oder externer Standard zur Quantifizierung von Aggregaten eingesetzt.

In Anlehnung an die Analyse der Verteilung der Fluoreszenzintensität (FIDA-Fluoreszenz-Intensity-Distribution-Analysis) handelt es sich bei dem erfindungsgemäßen Verfahren um ein sog. Oberflächen-FIDA (Surface-FIDA, sFIDA). Die Detektion erfolgt mit konfokaler Fluoreszenzmikroskopie, Fluoreszenzkorrelationsspektroskopie (FCS), Laser-Scanning-Mikroskop (LSM) und/oder Total Internal Reflection Fluoreszenz Mikroskopie (TIRFM), bevorzugt LSM und/oder Total Internal Reflection Fluoreszenz Mikroskopie (TIRFM).

In einer Ausführung der Erfindung werden pro Well bis zu 50 Einzelbilder (1000x1000 Pixel, 114 nm/pixel) pro Fluoreszenzkanal mit einer hochsensitiven CCD-Kamera abgebildet.

Hintergrundsignale in den Bilddaten werden über einen Intensitätsschwellenwert bereinigt. Die mittlere Anzahl der Pixel mit Graustufenwerten oberhalb des Schwellenwerts wird bestimmt (sFIDA-readout). Kommen mehrere Detektionssonden zum Einsatz, werden ausschließlich die in allen Fluoreszenzkanälen colokalisierten Ereignisse gewertet.

Das sFIDA-Verfahren wurde in Bannach et al. (2012. Detection of prion protein particles in blood plasma of scrapie infected sheep. PloS one 7, e36620) sowie Wang-Dietrichet al.(2013. The amyloid-beta oligomer count in cerebrospinal fluid is a biomarker for Alzheimer's disease. Journal of Alzheimer's disease 34, 985-994) beschrieben, die unter Bezugnahme eingeschlossen sind.

Durch Wahl der Fänger - und Sonden-Moleküle lässt sich bestimmen, welche Größe die Oligomere besitzen müssen, um zur Detektion (Signal) beitragen zu können. So können zum Beispiel Monomere, Dimere, Trimere etc. detektiert werden. Es können aber auch Fänger - und Sonden-Moleküle eingesetzt werden, dass als kleinste detektierbare Einheit zum Beispiel Dimere oder andere Polymere sind.

In einer Ausführung werden die Fängermoleküle und/oder die Sonden eingesetzt, die ein Polymer sind oder umfassen, aufgebaut aus Monomer-Sequenzen, bevorzugt einer der Sequenzen nach SEQ ID NO: 1-15 oder Teilen einer der SEQ ID NO: 1-15, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

Mit dem erfindungsgemäßen Verfahren ist zusätzlich auch die genaue Analyse von kleinen, frei diffundierbaren Aggregate möglich. Aufgrund ihrer Größe, die unterhalb ihrer Auflösung für Lichtmikroskope liegt, konnten kleine Oligomere schwer von der Hintergrundfluoreszenz (verursacht z.B. durch nicht gebundene Antikörper) unterschieden werden.

Neben der extrem hohen Sensitivität zeigt das erfindungsgemäße Verfahren auch eine Linearität in Bezug auf die Anzahl der Aggregate über einen großen Bereich.

Weiterer Gegenstand der vorläufigen Erfindung ist die Verwendung der kleinen, frei diffundierbaren A-Beta-Aggregate in Verbindung mit Tau-Aggregaten als Biomarker zum Nachweis und für die Erkennung von Proteinaggregationserkrankungen, insbesondere AD. Die Erfindung betrifft auch ein Verfahren zur Erkennung und/oder Nachweis von Proteinaggregationserkrankungen, insbesondere ausgewählt aus der Gruppe bestehend aus: AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer'sche Demenz , dadurch gekennzeichnet, dass eine Probe einer Körperflüssigkeit eines Patienten, bevorzugt CSF, oder Blut, insbesondere CSF mit dem erfindungsgemäßen, oben beschriebenen Verfahren analysiert wird.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht überzogen). Darauf werden (bevorzugt kovalent) Fängermoleküle für AA-enthaltende Partikel (z.B. anti-AA-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AA-Partikel (z.B. AA-Aggregate) werden mit einer für die weitere Detektion dienlichen Sonde (z.B. einem Fluoreszenzfarbstoff-gebundene anti-AA-Antikörper) markiert. Bevorzugte Fluoreszenzfarbstoffe könnten u.a. auch Quantumdots sein. Bevorzugt werden die AA-enthaltende Partikel mit mindestens einer weiteren Sonde (z.B. einem Fluoreszenzfarbstoff-gebunden anti-AA-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AA-Monomeren. Besonders, wenn eine der Detektionssonden identisch mit dem Fängermolekül (aus Schritt 1) ist oder beide ein überlappendes Epitop erkennen, kann die Detektion von AA-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde AA-Antikörper, bevorzugt Anti-Serum Amyloid A antibody clone 115, mc1 und/oder 291 eingesetzt.In einer Alternative werden als Fängermolekül und/oder Sonde (AF488-markiert): EPR 4134 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird oder zum Beispiel TIRFM) oder durch andere Arten der Oberflächen-Abbildung (z.B. durch ein TIRF-Mikroskop). Je höher die Zeit und- Ortsauflösung dabei ist, desto mehr Datenpunkte entstehen, die jeweils die Detektion eines Aggregates vor einem Hintergrundsignal (z.B. durch gerätespezifisches Rauschen, unspezifische

Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AL-enthaltende Partikel (z.B. anti-AL-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AL-Partikel (z.B. AL-Aggregate) werden mit einer für die weitere Detektion dienlichen Sonde (z.B. einem Fluoreszenzfarbstoff-gebundene anti-AL-Antikörper) markiert. Bevorzugte Fluoreszenzfarbstoffe könnten u.a. auch Quantumdots sein. Bevorzugt werden die AL-enthaltende Partikel mit mindestens einer weiteren Sonde (z.B. einem Fluoreszenzfarbstoff-gebunden anti-AL-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AL-Monomeren. Besonders, wenn eine der Detektionssonden identisch mit dem Fängermolekül (aus Schritt 1) ist oder beide ein überlappendes Epitop erkennen, kann die Detektion von AL-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde AAnti-Lambda Light chain antibody clone EPR5367, HP6054 und/oder 2G9 eingesetzt. In einer Alternative werden als Fängermolekül und/oder Sonde (AF488-markiert): EPR 5367 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische

Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AApoAI-enthaltende Partikel (z.B. anti-AApoAI-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AApoAI-Partikel (z.B. AApoAI-Aggregate) werden mit einer für die weitere Detektion dienlichen Sonde (z.B. einem Fluoreszenzfarbstoff-gebundene anti-AApoAI-Antikörper) markiert. Bevorzugte Fluoreszenzfarbstoffe könnten u.a. auch Quantumdots sein. Bevorzugt werden die AApoAI-enthaltende Partikel mit mindestens einer weiteren Sonde (z.B. einem Fluoreszenzfarbstoff-gebunden anti-AApoAI-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AApoAI-Monomeren. Besonders, wenn eine der Detektionssonden identisch mit dem Fängermolekül (aus Schritt 1) ist oder beide ein überlappendes Epitop erkennen, kann die Detektion von AApoAI-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Apolipoprotein AI antibody clone 12C8, 1409 und/oder G2 eingesetzt. In einer Alternative werden als Fängermolekül und/oder Sonde (AF488-markiert): EPR 1368Y eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AApoAII-enthaltende Partikel (z.B. anti-AApoAII-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe a enthaltene AApoAII-Partikel (z.B. AApoAII-Aggregate) werden mit einer für die weitere Detektion dienlichen Sonde (z.B. einem Fluoreszenzfarbstoff-gebundene anti-AApoAII-Antikörper) markiert. Bevorzugte Fluoreszenzfarbstoffe könnten u.a. auch Quantumdots sein. Bevorzugt werden die AApoAII-enthaltende Partikel mit mindestens einer weiteren Sonde (z.B. einem Fluoreszenzfarbstoff-gebunden anti-AApoAII-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AApoAII-Monomeren. Besonders, wenn eine der Detektionssonden identisch mit dem Fängermolekül (aus Schritt 1) ist oder beide ein überlappendes Epitop erkennen, kann die Detektion von AApoAII-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Apolipoprotein AII antibody clone 4F3, EPR2913 und/oder EP2912 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Ortsauflösung dabei ist, desto mehr Datenpunkte entstehen, die jeweils die Detektion eines Aggregates vor einem Hintergrundsignal (z.B. durch gerätespezifisches Rauschen, unspezifische

Signale, unspezifisch gebunden Sonden) erlauben. Auf diese Art entsteht nicht nur ein Readout-Wert (wie dies bei einem ELISA der Fall wäre), sondern so viele Readout-Werte, wie Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für ATTR-enthaltende Partikel (z.B. anti-ATTR-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltende ATTR-Partikel (z.B. ATTR-Aggregate) werden mit einer für die weitere Detektion dienlichen Sonde (z.B. einem Fluoreszenzfarbstoff-gebundene anti-ATTR-Antikörper) markiert. Bevorzugte Fluoreszenzfarbstoffe könnten u.a. auch Quantumdots sein. Bevorzugt werden die ATTR-enthaltende Partikel mit mindestens einer weiteren Sonde (z.B. einem Fluoreszenzfarbstoff-gebunden anti-ATTR-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von ATTR-Monomeren. Besonders, wenn eine der Detektionssonden identisch mit dem Fängermolekül (aus Schritt 1) ist oder beide ein überlappendes Epitop erkennen, kann die Detektion von ATTR-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Prealbumin antibody clone EP2929Y, EPR3119 und/oder 10E1 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für DISC1-enthaltende Partikel (z.B. anti-DISC1-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene DISC1-Partikel (z.B. DISC1-Aggregate) werden mit einer für die weitere Detektion dienlichen Sonde (z.B. einem Fluoreszenzfarbstoff-gebundene anti-DISC1-Antikörper) markiert. Bevorzugte Fluoreszenzfarbstoffe könnten u.a. auch Quantumdots sein. Bevorzugt werden die DISC1-enthaltende Partikel mit mindestens einer weiteren Sonde (z.B. einem Fluoreszenzfarbstoff-gebunden anti-DISC1-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von DISC1-Monomeren. Besonders, wenn eine der Detektionssonden identisch mit dem Fängermolekül (aus Schritt 1) ist oder beide ein überlappendes Epitop erkennen, kann die Detektion von DISC1-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-DISCl antibody clone 14F2, 2C7 und/oder FFD5 eingesetzt. In einer Alternative werden Anti-DISC1-AK 14F2 als Fänger-Antikörper und 14F2-AF633 als Detektionssonde eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische

Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für FUS-enthaltende Partikel (z.B. anti-FUS-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene FUS-Partikel (z.B. FUS-Aggregate) werden mit einer für die weitere Detektion dienlichen Sonde (z.B. einem Fluoreszenzfarbstoff-gebundene anti-FUS-Antikörper) markiert. Bevorzugte Fluoreszenzfarbstoffe könnten u.a. auch Quantumdots sein. Bevorzugt werden die FUS-enthaltende Partikel mit mindestens einer weiteren Sonde (z.B. einem Fluoreszenzfarbstoff-gebunden anti-FUS-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von FUS-Monomeren. Besonders, wenn eine der Detektionssonden identisch mit dem Fängermolekül (aus Schritt 1) ist oder beide ein überlappendes Epitop erkennen, kann die Detektion von FUS-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-TLS/FUS antibody clone EPR5812, EPR5813 und/oder CL0190 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für IAPP-enthaltende Partikel (z.B. anti-IAPP-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene IAPP-Partikel (z.B. IAPP-Aggregate) werden mit einer für die weitere Detektion dienlichen Sonde (z.B. einem Fluoreszenzfarbstoff-gebundene anti-IAPP-Antikörper) markiert. Bevorzugte Fluoreszenzfarbstoffe könnten u.a. auch Quantumdots sein. Bevorzugt werden die IAPP-enthaltende Partikel mit mindestens einer weiteren Sonde (z.B. einem Fluoreszenzfarbstoff-gebunden anti-IAPP-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von IAPP-Monomeren. Besonders, wenn eine der Detektionssonden identisch mit dem Fängermolekül (aus Schritt 1) ist oder beide ein überlappendes Epitop erkennen, kann die Detektion von IAPP-Monomeren ausgeschlossen werden. Zur Steigerung der Spezifität kann als zusätzliche Detektionssonde der amyloidspezifische Farbstoff Thioflavin T eingesetzt werden.

In einer Alternative wird als Fängermolekül und/oder Sonde Anti-Amylin antibody clone R10/99 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für SOD1-enthaltende Partikel (z.B. anti-SOD1-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene SOD1-Partikel (z.B. SOD1-Aggregate) werden mit einer für die weitere Detektion dienlichen Sonde (z.B. einem Fluoreszenzfarbstoff-gebundene anti-SOD1-Antikörper) markiert. Bevorzugte Fluoreszenzfarbstoffe könnten u.a. auch Quantumdots sein. Bevorzugt werden die SOD1-enthaltende Partikel mit mindestens einer weiteren Sonde (z.B. einem Fluoreszenzfarbstoff-gebunden anti-SOD1-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von SOD1-Monomeren. Besonders, wenn eine der Detektionssonden identisch mit dem Fängermolekül (aus Schritt 1) ist oder beide ein überlappendes Epitop erkennen, kann die Detektion von SOD1-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Superoxide Dismutase 1 antibody clone 2F5, 71G8 und/oder EPR1726 eingesetzt. In einer Alternative werden als Fängermolekül und/oder Sonde (AF488-markiert): EPR 1726 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assays, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für α-Synuclein-enthaltende Partikel (z.B. anti-α-Synudein-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene α-Synudein-Partike! (z.B. α-Synuclein-Aggregate) werden mit einer für die weitere Detektion dienlichen Sonde (z.B. einem Fluoreszenzfarbstoff-gebundene anti-α-Synuclein-Antikörper) markiert. Bevorzugte Fluoreszenzfarbstoffe könnten u.a. auch Quantumdots sein. Bevorzugt werden die α-Synuclein-enthaltende Partikel mit mindestens einer weiteren Sonde (z.B. einem Fluoreszenzfarbstoff-gebunden anti-α-Synuclein-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von α-Synuclein-Monomeren. Besonders, wenn eine der Detektionssonden identisch mit dem Fängermolekül (aus Schritt 1) ist oder beide ein überlappendes Epitop erkennen, kann die Detektion von α-Synuclein-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Alpha-Synuclein antibody clone 2B2A11, 3H2897 und/oder 211 eingesetzt. In einer Alternative werden als Fänger-Antikörper Anti-aSyn-AB 2B2A11 und als Detektionssonde die fluoreszenzmarkierten Anti-aSyn-ABs 3H2897-AF633 bzw. 211-AF488 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für Tau-enthaltende Partikel (z.B. anti-Tau-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene Tau-Partikel (z.B. Tau-Aggregate) werden mit einer für die weitere Detektion dienlichen Sonde (z.B. einem Fluoreszenzfarbstoff-gebundene anti-Tau-Antikörper) markiert. Bevorzugte Fluoreszenzfarbstoffe könnten u.a. auch Quantumdots sein. Bevorzugt werden die Tau-enthaltende Partikel mit mindestens einer weiteren Sonde (z.B. einem Fluoreszenzfarbstoff-gebunden anti-Tau-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von Tau-Monomeren. Besonders, wenn eine der Detektionssonden identisch mit dem Fängermolekül (aus Schritt 1) ist oder beide ein überlappendes Epitop erkennen, kann die Detektion von Tau-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Tau antibody clone E178, Tau46 und/oder Tau 5 eingesetzt. In einer Alternative werden als Fängermolekül und/oder Sonde Anti-Tau 6E10/Atto488 und/oder Nab228 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für TDP-43-enthaltende Partikel (z.B. anti-TDP-43-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene TDP-43-Partikel (z.B. TDP-43-Aggregate) werden mit einer für die weitere Detektion dienlichen Sonde (z.B. einem Fluoreszenzfarbstoff-gebundene anti-TDP-43-Antikörper) markiert. Bevorzugte Fluoreszenzfarbstoffe könnten u.a. auch Quantumdots sein. Bevorzugt werden die TDP-43-enthaltende Partikel mit mindestens einer weiteren Sonde (z.B. einem Fluoreszenzfarbstoff-gebunden anti-TDP-43-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von TDP-43-Monomeren. Besonders, wenn eine der Detektionssonden identisch mit dem Fängermolekül (aus Schritt 1) ist oder beide ein überlappendes Epitop erkennen, kann die Detektion von TDP-43-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-TARDBP antibody clone EPR5810, 3H8 und/oder K1B8 eingesetzt. In einer Alternative werden als Fängermolekül und/oder Sonde (AF488-markiert): EPR 5810 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assays, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für Huntingtin-enthaltende Partikel (z.B. anti-Huntingtin-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltende Huntingtin-Partikel (z.B. Huntingtin-Aggregate) werden mit einer für die weitere Detektion dienlichen Sonde (z.B. einem Fluoreszenzfarbstoff-gebundene anti-Huntingtin-Antikörper) markiert. Bevorzugte Fluoreszenzfarbstoffe könnten u.a. auch Quantumdots sein. Bevorzugt werden die Huntingtin-enthaltende Partikel mit mindestens einer weiteren Sonde (z.B. einem Fluoreszenzfarbstoff-gebunden anti-Huntingtin-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von Huntingtin-Monomeren. Besonders, wenn eine der Detektionssonden identisch mit dem Fängermolekül (aus Schritt 1) ist oder beide ein überlappendes Epitop erkennen, kann die Detektion von Huntingtin-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Huntingtin antibody clone EP867Y, D7F7 und/oder HIP-1 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assays, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für ALys-enthaltende Partikel (z.B. anti-ALys-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene ALys-Partikel (z.B. ALys-Aggregate) werden mit einer für die weitere Detektion dienlichen Sonde (z.B. einem Fluoreszenzfarbstoff-gebundene anti-ALys-Antikörper) markiert. Bevorzugte Fluoreszenzfarbstoffe könnten u.a. auch Quantumdots sein. Bevorzugt werden die ALys-enthaltende Partikel mit mindestens einer weiteren Sonde (z.B. einem Fluoreszenzfarbstoff-gebunden anti-ALys-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von ALys-Monomeren. Besonders, wenn eine der Detektionssonden identisch mit dem Fängermolekül (aus Schritt 1) ist oder beide ein überlappendes Epitop erkennen, kann die Detektion von ALys-Monomeren ausgeschlossen werden. Zur Steigerung der Spezifität kann als zusätzliche Detektionssonde der amyloidspezifische Farbstoff Thioflavin T eingesetzt werden.

In einer Alternative werden als Fängermolekül und/oder Sonde Anti-Lysozyme antibody clone BGN/06/961 und/oder BGN/0696/2B10 eingesetzt. In einer Alternative werden als Fängermolekül und/oder Sonde (AF488-markiert): EPR 2994 eingesetzt.

4. Die Schritte 2 und 3 können auch vertauscht werden. Durch geeignete Waschschritte werden überschüssige Sonden entfernt.

5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.

6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.

7. Um die Testergebnisse miteinander (über Entfernungen, Zeiten und Experimentatoren hinweg) vergleichbar zu machen, können Standards (interne und/oder externe) eingesetzt werden.

In einer Ausführung ist der Gegenstand der vorliegenden Erfindung ein Verfahren zur selektiven Quantifizierung und/oder Charakterisierung von Aggregaten des Typs Serum-Amyloid-A-Protein-Aggregaten in Körperflüssigkeiten als Biomarker für AA-Amyloidose. In einer Ausführung wird zusätzlich auf einen weitere Aggregat-Typ untersucht, ausgewählt aus der Gruppe bestehend aus: IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs Serum-Amyloid-A-Protein-Aggregate mit einem oder mehreren Proteinen ausgewählt aus der Gruppe bestehend aus: IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate in Körperflüssigkeiten als Biomarker für AA-Amyloidose.

In einer Ausführung ist der Gegenstand der vorliegenden Erfindung ein Verfahren zur selektiven Quantifizierung und/oder Charakterisierung von Aggregaten des Typs IgG-light-chain-Aggregaten in Körperflüssigkeiten als Biomarker für AL-Amyloidose. In einer Ausführung wird zusätzlich auf einen weitere Aggregat-Typ untersucht, ausgewählt aus der Gruppe bestehend aus: Serum-Amyloid-A-Protein-Aggregate, AapoAI-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs IgG-light-chain-Aggregate mit einem oder mehreren Proteinen ausgewählt aus der Gruppe bestehend aus: Serum-Amyloid-A-Protein-Aggregate, AapoAI-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate in Körperflüssigkeiten als Biomarker für AL-Amyloidose.

In einer Ausführung ist der Gegenstand der vorliegenden Erfindung ein Verfahren zur selektiven Quantifizierung und/oder Charakterisierung von Aggregaten des Typs AApoAI-Aggregaten in Körperflüssigkeiten als Biomarker für AapoAI-Amyloidose. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs AapoAI-Aggregate mit einem oder mehreren Proteinen ausgewählt aus der Gruppe bestehend aus: Serum-Amyloid-A-Protein-Aggregate, IgG-light-chain-Aggregate, AapoAII-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate in Körperflüssigkeiten als Biomarker für AApoAI-Amyloidose.

In einer Ausführung ist der Gegenstand der vorliegenden Erfindung ein Verfahren zur selektiven Quantifizierung und/oder Charakterisierung von Aggregaten des Typs AApoAII-Aggregaten in Körperflüssigkeiten als Biomarker für AapoAII-Amyloidose. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs AapoAII-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, ATTR-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate in Körperflüssigkeiten als Biomarker für AApoAII-Amyloidose.

In einer Ausführung ist der Gegenstand der vorliegenden Erfindung ein Verfahren zur selektiven Quantifizierung und/oder Charakterisierung von Aggregaten des Typs ATTR-Aggregaten in Körperflüssigkeiten als Biomarker für ATTR-Amyloidose. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, DISC1-Aggregate, FUS-Aggregate, IAPP-Aggregate, SOD1-Aggregate, α-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs ATTR-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, DISC1-Aggregate, FUS-Aggregate, IAPP-Aggregate, SOD1-Aggregate, a-Synuclein-Aggregate, Tau-Aggregate, TDP-43-Aggregate, Huntingtin-Aggregate, Lysozym-Aggregate und A-beta-Aggregate in Körperflüssigkeiten als Biomarker für ATTR-Amyloidose.

In einer Ausführung ist der Gegenstand der vorliegenden Erfindung ein Verfahren zur selektiven Quantifizierung und/oder Charakterisierung von Aggregaten des Typs DISC1-Aggregaten in Körperflüssigkeiten als Biomarker für Schizophrenie und andere DISC1opathien. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, FUS-Aggregate, IAPP-Aggregate, SOD1-Aggregate, a-Synuclein-Aggregate, Tau-Aggregate, TDP-43-Aggregate, Huntingtin-Aggregate, Lysozym-Aggregate und A-beta-Aggregate, bevorzugt FUS-Aggregate, SOD1-Aggregate und/oder TDP-43-Aggregate.

In einer Ausführung ist der Gegenstand der vorliegenden Erfindung ein Verfahren zur selektiven Quantifizierung und/oder Charakterisierung von gemischten Aggregaten des Typs DISC1-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, FUS-Aggregate, IAPP-Aggregate, SOD1-Aggregate, a-Synuclein-Aggregate, Tau-Aggregate, TDP-43-Aggregate, Huntingtin-Aggregate, Lysozym-Aggregate und A-beta-Aggregate in Körperflüssigkeiten als Biomarker für Schizophrenie und andere DISC1opathien.

In einer Ausführung ist der Gegenstand der vorliegenden Erfindung ein Verfahren zur selektiven Quantifizierung und/oder Charakterisierung von Aggregaten des Typs FUS-Aggregaten in Körperflüssigkeiten als Biomarker für Amyotrophe Lateralsklerose, Frontotemporale Lobärdegeneration und andere FUS-Proteinopathien. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, IAPP-Aggregate, SOD1-Aggregate, a-Synuclein-Aggregate, Tau-Aggregate, TDP-43-Aggregate, Huntingtin-Aggregate, Lysozym-Aggregate und A-beta-Aggregat, bevorzugt DISC1-Aggregate, SOD1-Aggregate und/oder TDP-43-Aggregate.

In einer Ausführung ist der Gegenstand der vorliegenden Erfindung ein Verfahren zur selektiven Quantifizierung und/oder Charakterisierung von gemischten Aggregaten des Typs FUS-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, IAPP-Aggregate, SOD1-Aggregate, a-Synuclein-Aggregate, Tau-Aggregate, TDP-43-Aggregate, Huntingtin-Aggregate, Lysozym-Aggregate und A-beta-Aggregate in Körperflüssigkeiten als Biomarker für Amyotrophe Lateralsklerose, Frontotemporale Lobärdegeneration und andere FUS-Proteinopathien.

In einer Ausführung ist der Gegenstand der vorliegenden Erfindung ein Verfahren zur selektiven Quantifizierung und/oder Charakterisierung von Aggregaten des Typs IAPP-Aggregaten in Körperflüssigkeiten als Biomarker für Diabetes Mellitus Typ 2. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, SOD1-Aggregate, a-Synuclein-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs IAPP-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, SOD1-Aggregate, a-Synuclein-Aggregate, Tau-Aggregate, TDP-43-Aggregate, Huntingtin-Aggregate, Lysozym-Aggregate und A-beta-Aggregate in Körperflüssigkeiten als Biomarker für Diabetes Mellitus Typ 2.

In einer Ausführung ist der Gegenstand der vorliegenden Erfindung ein Verfahren zur selektiven Quantifizierung und/oder Charakterisierung von Aggregaten des Typs SOD1-Aggregaten in Körperflüssigkeiten als Biomarker für Amyotrophe Lateralsklerose. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, a-Synuclein-Aggregate, Tau-Aggregate, TDP-43-Aggregate, Huntingtin-Aggregate, Lysozym-Aggregate und A-beta-Aggregate, bevorzugt DISC1-Aggregate, FUS-Aggregate und/oder TDP-43-Aggregate.

In einer Ausführung ist der Gegenstand der vorliegenden Erfindung ein Verfahren zur selektiven Quantifizierung und/oder Charakterisierung von gemischten Aggregaten des Typs SOD1-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, a-Synuclein-Aggregate, Tau-Aggregate, TDP-43-Aggregate, Huntingtin-Aggregate, Lysozym-Aggregate und A-beta-Aggregate in Körperflüssigkeiten als Biomarker für Amyotrophe Lateralsklerose.

In einer Ausführung ist der Gegenstand der vorliegenden Erfindung ein Verfahren zur selektiven Quantifizierung von Aggregaten des Typs α-Synuclein-Aggregaten in Körperflüssigkeiten als Biomarker für die Parkinson-Krankheit und anderer Synucleinopathien. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Huntingtin-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs a-Synuclein-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Huntingtin-Aggregate, Lysozym-Aggregate und A-beta-Aggregate in Körperflüssigkeiten als Biomarker für Parkinson-Krankheit und anderer Synucleinopathien.

In einer Ausführung ist der Gegenstand der vorliegenden Erfindung ein Verfahren zur selektiven Quantifizierung und/oder Charakterisierung von Aggregaten des Typs Tau-Aggregaten in Körperflüssigkeiten als Biomarker für Tauopathien. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregaten, a-Synuclein-Aggregate, TDP-43-Aggregate, Huntingtin-Aggregate, Lysozym-Aggregate und A-beta-Aggregate, bevorzugt A-beta-Aggregate.

In einer Ausführung ist der Gegenstand der vorliegenden Erfindung ein Verfahren zur selektiven Quantifizierung und/oder Charakterisierung von gemischten Aggregaten des Typs Tau-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, a-Synuclein-Aggregate, TDP-43-Aggregate, Huntingtin-Aggregate, Lysozym-Aggregate und A-beta-Aggregate in Körperflüssigkeiten als Biomarker für Tauopathien.

In einer Ausführung ist der Gegenstand der vorliegenden Erfindung ein Verfahren zur selektiven Quantifizierung und/oder Charakterisierung von Aggregaten des Typs TDP-43-Aggregaten in Körperflüssigkeiten als Biomarker für Amyotrophe Lateralsklerose, Frontotemporale Lobärdegeneration, Chronische Traumatische Enzephalopathie und andere TDP-43-Proteinopathien. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, a-Synuclein-Aggregate, Tau-Aggregate, Huntingtin-Aggregate, Lysozym-Aggregate und A-beta-Aggregate, bevorzugt DISC1-Aggregate, FUS-Aggregate und/oder SOD1-Aggregate.

In einer Ausführung ist der Gegenstand der vorliegenden Erfindung ein Verfahren zur selektiven Quantifizierung und/oder Charakterisierung von gemischten Aggregaten des Typs TDP-43-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, a-Synuclein-Aggregate, Tau-Aggregate, Huntingtin-Aggregate, Lysozym-Aggregate und A-beta-Aggregate in Körperflüssigkeiten als Biomarker für TDP-43-Proteinopathien.

In einer Ausführung ist der Gegenstand der vorliegenden Erfindung ein Verfahren zur selektiven Quantifizierung und/oder Charakterisierung von Aggregaten des Typs Huntingtin-Aggregaten in Körperflüssigkeiten als Biomarker für die Huntington-Krankheit. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Lysozym-Aggregate und A-beta-Aggregate.

In einer Ausführung ist der Gegenstand der vorliegenden Erfindung ein Verfahren zur selektiven Quantifizierung und/oder Charakterisierung von gemischten Aggregaten des Typs Huntingtin-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Lysozym-Aggregate und A-beta-Aggregate in Körperflüssigkeiten als Biomarker für Huntington-Krankheit.

In einer Ausführung ist der Gegenstand der vorliegenden Erfindung ein Verfahren zur selektiven Quantifizierung und/oder Charakterisierung von Aggregaten des Typs Lysozym-Aggregaten in Körperflüssigkeiten als Biomarker für Familiäre Viszerale Amyloidose.In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Huntingtin-Aggregaten und A-beta-Aggregate.

In einer Ausführung ist der Gegenstand der vorliegenden Erfindung ein Verfahren zur selektiven Quantifizierung und/oder Charakterisierung von gemischten Aggregaten des Typs Lysozym-Aggregate mit einem oder mehreren Proteinen ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Huntingtin-Aggregate und A-beta-Aggregate in Körperflüssigkeiten als Biomarker für Familiäre Viszerale Amyloidose.

In einer Ausführung ist der Gegenstand der vorliegenden Erfindung ein Verfahren zur selektiven Quantifizierung und/oder Charakterisierung von Aggregaten des Typs A-beta-Aggregaten und mindestens eines weiteren Aggregat-Typs, bevorzugt des Typs Tau-Aggregaten, in Körperflüssigkeiten als Biomarker für Alzheimer-Demenz. In einer Ausführung wird zusätzlich auf einen weitere Aggregat-Typ untersucht, ausgewählt aus der Gruppe bestehend aus: Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, TDP-43-Aggregate, Huntingtin-Aggregate und Lysozym-Aggregate.

Gegenstand der vorliegenden Erfindung ist ferner ein Standard zur selektiven Quantifizierung und/oder Charakterisierung der Krankheitsindikatoren enthaltend ein Polymer eingesetzt wird, aufgebaut aus Monomer Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.

Als Standard im Sinne der vorliegenden Erfindung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von (pathogenen) Aggregaten aus körpereigenen (fehlgefalteten) Proteinen. Der Standard im Sinne der vorliegenden Erfindung kann zum Kalibrieren von Geräten und/oder Messungen verwendet werden.

Wesentlich für die erfindungsgemäßen Standards ist, dass die Standards nicht aggregieren, bevorzugt durch die Verwendung von Monomeren-Sequenzen, die nicht aggregieren, da der entsprechende Teilbereich körpereigener Proteine für die Aggregation nicht verantwortlich ist, oder die durch Blockierung der für die Aggregation verantwortlichen Gruppen nicht aggregieren.

In diesem Sinne beschreibt hier der Begriff "Monomer-Sequenz" einen Teilbereich, ein Fragment der einzelnen Proteine (Monomeren) die Aggregate fehlgefalteter Proteine bilden.

In einer Ausführung ist der Teilbereich ein Epitop oder ein Homolog mit mindestens 50% Identität dazu und der biologischen Aktivität eines Epitops, bevorzugt eines Epitops enthaltend in einer der SEQ ID NO: 1-15.

Eine so ausgewählte Monomer-Sequenz wird in der gewünschten Anzahl bei dem Aufbau der erfindungsgemäßen Standards eingebaut und/oder miteinander erfindungsgemäß verknüpft.

Die erfindungsgemäßen Standards sind Polymere, die aus den oben beschriebenen Monomer-Sequenzen, bevorzugt Epitope aufgebaut sind, gegebenenfalls enthaltend weitere Elemente.

In einer weiteren Ausführung der vorliegenden Erfindung stellen oben beschriebenen Monomer-Sequenzen, bevorzugt Epitope, und/oder deren Homologe mit der biologischen Aktivität des entsprechenden Epitops, die gleiche oder größte Anzahl an Monomeren bezogen auf die Anzahl jeweils einer der restlichen Monomer-Arten des Standards und/oder bezogen auf die Anzahl aller anderen Monomere.

Das erfindungsgemäße Standardmolekül ist ein Polymer aus den oben definierten Monomer-Sequenzen. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomer-Sequenz, oder Vielfachen davon, bevorzugt 2 - 16, 4-16, 8-16, besonders bevorzugt 8 oder 16, oder Vielfachen davon.

In einer Alternative der vorliegenden Erfindung sind die Standards wasserlöslich.

In einer Alternative der vorliegenden Erfindung sind die erfindungsgemäßen Standard aus gleichen Monomer-Sequenz aufgebaut.

In einer Alternative der vorliegenden Erfindung sind die erfindungsgemäßen Standard aus unterschiedlichen Monomer-Sequenz aufgebaut.

In einer Alternative der vorliegenden Erfindung werden solche oben definierte Monomer-Sequenz in einer linearen-Konformation aneinandergereiht.

In einer Alternative der vorliegenden Erfindung werden solche oben definierte Monomer-Sequenzen zu einem verzweigten, erfindungsgemäßen Oligomer aneinandergereiht.

In einer Alternative der vorliegenden Erfindung werden solche oben definierte Monomer-Sequenzen zu einem cross-linked, erfindungsgemäßen Oligomer aneinandergereiht.

Verzweigte oder cross-linked, erfindungsgemäße Oligomere können durch Verknüpfung einzelner Bausteine mittels Lysin oder mittels Click-Chemie hergestellt werden.

Wie oben beschrieben, können die erfindungsgemäßen Standards, also die erfindungsgemäßen Oligomere beziehungsweise Polymere, zusätzlich zu den in genau definierter Anzahl vorliegenden Monomer-Sequenzen, bevorzugt Epitopen, noch zusätzliche Aminosäuren, Spacer und/oder funktionelle Gruppen enthalten, über welche die Monomer-Sequenzen, bevorzugt Epitope, kovalent miteinander verknüpft sind.

In einer Alternative ist die unmittelbare Verknüpfung der Monomer-Sequenzen, bevorzugt Epitope mit Cystein, insbesondere mittels Disulfid-Verbrückung durch Cysteine ausgeschlossen (um zu vermeiden, dass reduzierende Agenzien die Verbrückung lösen). Ebenso ist in einer weiteren Variante eine unmittelbare Verknüpfung der Spacer mit der Monomer-Sequenz einerseits und mit Cystein andererseits ausgeschlossen.

Die Vervielfältigung der Epitope durch funktionelle Gruppen kann vor oder nach der Synthese der einzelnen Bausteine durchgeführt werden. Charakteristisch für die erfindungsgemäßen Standards ist die kovalente Verknüpfung der Monomer-Sequenzen.

Die erfindungsgemäß einzusetzenden Monomer-Sequenzen können identisch mit der Sequenz eines Proteins sein oder zeigen eine Homologie von 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % mit der Sequenz eines der Volllängen-Proteine die Aggregate bilden.

Alternativ werden auch zum Aufbau der erfindungsgemäßen Standard-Moleküle Monomer-Sequenzen eingesetzt, die identisch mit einem Teilbereich eines Volllängen-Proteins sind, bzw. eine Homologie von 50, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit einem Teilbereich eines Volllängen-Proteins zeigen.

Wesentlich für die erfindungsgemäß eingesetzten Sequenzen ist ihre Eigenschaft nicht (oder nur gemäß den Bedingungen kontrolliert) zu aggregieren und/oder ihre die Aktivität als Epitop.

Standards haben in einer Alternative vorteilhaft eine höhere Löslichkeit im Wässrigen als pathogene Aggregate oder Oligomere aus körpereigenen Proteinen.

In einer Ausführung der vorliegenden Erfindung besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Bindungspartner.

In diesem Sinne mit Bezug auf die Standards werden Bindungspartner ausgewählt aus der Gruppe bestehend aus: Antikörper, Nanobody und Affibody. Bindungspartner sind darüber hinaus alle Moleküle, die eine hinreichende Bindespezifität für das zu detektierende Aggregat besitzen, z.B. Farbstoffe (ThioflavinT, Kongorot, etc.).

Ein erfindungsgemäßes Standardmolekül kann Epitope für mindestens 2, 3, 4, 5, 6, 7, 8, 9, 10 oder mehr verschiedene Bindungspartner enthalten.

Epitope charakteristisch für verschiedene Bindungspartner können in die erfindungsgemäßen Standards dadurch eingebaut werden, dass Monomer-Sequenzen verwendet werden, die identisch mit unterschiedlichen Bereichen eines oder unterschiedlicher Proteine (die Aggregate fehlgefalteter Proteine bilden) sind, bzw. mindestens eine 50 %ige Homologie dazu aufweisen, aber die Aktivität des entsprechenden Epitops besitzen.

In einer Ausführung der vorliegenden Erfindung enthalten die Standardmoleküle sog. Spacer.

Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der erfindungsgemäßen Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die erfindungsgemäßen Standards enthalten in einer Alternative der vorliegenden Erfindung (weitere) funktionelle Gruppen.

Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die erfindungsgemäßen Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren Alternative der vorliegenden Erfindung enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

In einer weiteren Ausführung der vorliegenden Erfindung sind die Standards als Dendrimere aufgebaut. Die erfindungsgemäßen Dendrimere sind aus den oben beschriebenen erfindungsgemäß zu verwendenden Monomer-Sequenzen aufgebaut und können ein zentrales Gerüstmolekül enthalten. Bevorzugt ist das Gerüstmolekül ein Streptavidin-Monomer, besonders bevorzugt ein Polymer, insbesondere Tetramer.

Die erfindungsgemäßen Dendrimere enthalten in einer Variante Monomer-Sequenzen, die eine Sequenz besitzen, die identisch mit einem Teilbereich Proteins ist, oder eine mindestens 50 %ige Homologie zu dem entsprechenden Teilbereich zeigt.

Erfindungsgemäß ist unter dem Begriff mindestens 50 %ige Homologie auch eine höhere Homologie zu verstehen, ausgewählt aus der Gruppe bestehend aus 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % zu verstehen.

Weiterer Gegenstand der vorliegenden Erfindung sind Dendrimere enthaltend Polypeptide, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen.

Die erfindungsgemäßen Dendrimere können jede der oben beschriebenen Merkmale der Standards oder jede beliebige Kombination davon enthalten.

In einer Alternative der vorliegenden Erfindung handelt es sich um:
- Dendrimere enthaltend eine genau definierte Anzahl von Epitopen für die kovalente Bindung von Bindungspartnern,
- Dendrimer enthaltend Epitope eines oder mehrerer der Proteine die Aggregate fehlgefalteter Proteine bilden, bevorzugt gemäß SEQ ID NO: 1-15,
- Dendrimer dadurch gekennzeichnet, dass er eine höhere Löslichkeit im Wässrigen besitzt, als die pathogenen Aggregate aus körpereigenen Proteinen,
- Dendrimer enthaltend funktionelle Gruppen,
- Dendrimer enthaltend mindestens ein Spacer-Molekül und/oder
- Dendrimer enthaltend Farbstoffe zur spektralphotometrischen Bestimmung und/oder aromatische Aminosäuren.

Erfindungsgemäß haben die Dendrimere eine radiale Symmetrie.

In einer Variante erfolgt die Verzweigung der ersten Generation des Dendrimers über Lysin, inbesondere drei Lysin-Aminosäuren.

In einer weiteren Alternative der vorliegenden Erfindung sind in den Standards, insbesondere Dendrimeren, die Polypeptid-Sequenzen, bevorzugt Epitope, nicht über eine Bindung zu einem Schwefelatom, nicht über eine Thioether-Bindung und/oder nicht über Cystein (gegebenenfalls mittels Disulfid-Verbrückung durch Cystein) miteinander oder mit anderen Elementen der Standards wie Aminosäuren, Spacer und/oder funktionelle Gruppen und/oder andere oben beschriebenen Elementen verknüpft, insbesondere kovalent gebunden. Ebenso sind in einer weiteren Variante die Polypeptid-Sequenzen, bevorzugt Epitope, und ein daran gebundener Spacer am Spacer nicht über eine Bindung zu einem Schwefelatom, nicht über eine Thioether-Bindung und/oder nicht über Cystein miteinander oder mit anderen Elementen der Standards wie Aminosäuren, weitere Spacer und/oder funktionelle Gruppen und/oder andere oben beschriebenen Elementen verknüpft, insbesondere kovalent gebunden.

Die vorliegende Erfindung betrifft ferner ein Verfahren zur Herstellung eines Standards, wie oben beschrieben.

In einer Ausführung wird der erfindungsgemäße Standard mittels Peptidsynthese oder rekombinater Verfahren hergestellt, die dem Fachmann bekannt sind

Ein weiterer Gegenstand der vorliegenden Erfindung ist die Verwendung eines oben beschriebenen Standards oder eines oben beschriebenen Dendrimers zur Quantifizierung von pathogenen Aggregaten oder Oligomeren aus körpereigenen Proteinen.

Erfindungsgemäß werden die erfindungsgemäßen Standard in einem Verfahren zur Quantifizierung von pathogenen Aggregaten oder Oligomeren aus körpereigenen Proteinen eingesetzt.

Die erfindungsgemäßen Standards werden in einer Ausführung der vorliegenden Erfindung für die Kalibrierung bei der Surface-FIDA-Methode (sFIDA), Elisa (Sandwich-Elisa) oder FACS eingesetzt.

In einer anderen Ausführungsform betrifft die vorliegende Erfindung ein Kit, welches erfindungsgemäßen Standard umfasst. Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotitterplatte, absorbiert sein.

Ein solches Kit kann ein oder mehrere der folgenden Komponenten enthalten:
- Substrat aus Glas, das mit einem hydrophoben Stoff beschichtet ist, bevorzugt Dextran, bevorzugt Carboxymethyl-Dextran oder Polyethylenglykol, bevorzugt Heterobiofunktionales Polyethylenglykol (NHS-PEG-COOH);
- Standard;
- Fängermolekül;
- Sonde;
- Substrat mit Fängermolekül.

Ferner kann das Kit Anweisungen für den Gebrauch des Kits für eine Beliebige der Ausführungsformen enthalten.

In einer Alternative der vorliegenden Erfindung werden die Standards zur Quantifizierung von pathogenen Aggregaten oder Oligomeren aus körpereigenen Proteinen verwendet indem:
in einem ersten Schritt die Standards oder die Dendrimere mit Bindungspartnern markiert werden und die Anzahl der an die Standards oder Dendrimere gebundenen Bindungspartner bestimmt wird,
in einem zweiten Schritt pathogene Aggregaten oder Oligomere aus körpereigenen Proteinen mit Sonden markiert werden, die Zahl der an jeweils ein pathogenes Aggregat oder Oligomer bindende Sonden bestimmt wird,
in einem dritten Schritt die Zahl der an jeweils ein Standard oder Dendrimer bindende Bindungspartner aus Schritt 1 mit der Zahl der an ein Aggregat bindenden Sonden aus Schritt 2 verglichen wird, und
in einem vierten Schritt dadurch die Zahl und die Größe der Oligomere aus der Körperflüssigkeit bestimmt wird.

In einer Variante der vorliegenden Erfindung werden die erfindungsgemäßen Standards, bevorzugt Dendrimere, für die Kalibrierung der Surface-FIDA-Methode eingesetzt. In einem ersten Schritt werden körpereigene pathogene Aggregate aus Körperflüssigkeiten auf eine Glasfläche immobilisiert. Nach der Immobilisierung werden die Aggregate durch zwei unterschiedliche Sonden markiert. Die Detektionssonden sind mit, bevorzugt unterschiedlichen Fluoreszenzfarbstoffen markiert. Dadurch werden sie im Mikroskop, z.B. Laserscanningmikroskop sichtbar.

Die erfindungsgemäßen Standards könne auch als Fängermoleküle und/oder Sonden eingesetzt werden, oder Teile davon sein. Somit sind Gegenstand der Erfindung auch Fängermoleküle und/oder Sonden enthaltend die erfindungsgemäßen Standards.

Gegenstand der vorliegenden Erfindung ist ein Substrat umfassend genau definierte Bereiche mit Fängermolekülen für die Krankheitsindikatoren und/oder Aggregate.

Diese Substrat wird verwendet in einem Verfahren zur Diagnose einer Krankheit ausgewählt aus der Gruppe bestehend aus:
Alzheimer-Demenz, AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer-Demenz.

Alle Verfahrensschritte nach der Entnahme der Probe werden ex vivo (in vitro), also außerhalb des menschlichen oder tierischen Körpers durchgeführt.

Die Probe kann einem Menschen oder einem Tier entnommen werden. Aggregat-Typen körpereigener fehlgefalteter Proteine werden bei folgenden Krankheit gefunden und können als Biomarker oder Teil eines Biomarkers eingesetzt werden: Tab. A, Zeilen 1-15

**Tab A:**

| | **Biomarker oder Teil davon** | **Krankheit** |
|---|---|---|
| 1 | Serum-Amyloid-A-Protein-Aggregate | AA-Amyloidose |
| 2 | IgG-light-chain-Aggregate | AL-Amyloidose |
| 3 | AApoAI -Aggregate | AApoAI -Amyloidose |
| 4 | AApoAII-Aggregate | AApoAII-Amyloidose |
| 5 | ATTR-Aggregate | ATTR -Amyloidose |
| 6 | DISC1-Aggregate | Schizophrenie und andere DISC1opathien |
| 7 | FUS-Aggregate | Amyotrophe lateralsklerose, Frontotemporale lobärdegeneration und andere FUS-Proteinopathien |
| 8 | IAPP-Aggregate | Diabetes Mellitus Typ 2 |
| 9 | SOD1-Aggregate | Amyotrophe Lateralsklerose |
| 10 | a-Synuclein-Aggregate | Parkinson-Krankheit und andere Synucleinopathien |
| 11 | Tau-Aggregate | Tauopathien |
| 12 | TDP-43-Aggregate | Amyotrophe Lateralsklerose, Frontotemporale Lobärdegeneration, Chronische Traumatische Enephalopathie und andere TDP-43 Proteinopathien |
| 13 | H unti ngti n-Aggregate | Huntington-Krankheit |
| 14 | Lysozym-Aggregate | Familiäre viszerale Amyloidose |
| 15 | A-beta-Aggregate | Alzheimer Demenz |

In einer Ausführung der vorliegenden Erfindung ist ein Biomarker der Tabelle A auch als Krankheitsindikator für eine andere Krankheit der Tabelle A zu verwenden, als in derselben Zeile des Biomarkers in der Tabelle angegeben.

Beispielsweise können Tauaggregate auch als Biomarker für Alzheimer Demenz dienen oder wie in der Tabelle gezeigt DISC1-Aggregate, FUS-Aggregate, Tau-Aggregate oder TDP-43-Aggregate für mehrere Krankheiten.

Beispielsweise können auch Mischaggregate, zum Beispiel aus Tau und a-Synuclein, als Biomarker für z.B. Parkinson dienen. Auch alle anderen Mischaggregate können als Biomarker verwendet werden.

Somit ist nicht nur die Diagnose von Kombinationen von Krankheiten möglich, sondern aus diesen Ergebnissen lassen sich andere Krankheiten auch ausschließen. Dadurch wird eine genauere Diagnose ebenfalls ermöglicht.

Gegenstand der vorliegenden Erfindung ist eine Differenzialdiagnose zur Bestimmung von Krankheiten die Aggregate fehlgefalteter Proteine aufweisen, umfassend folgende Schritte:
i) Bestimmung der Menge fehlgefalteter Protein-Aggregate in einer Probe in einem Verfahren enthaltend:
   a) Entnehmen der Probe einem menschlichen oder tierischen Körper, gegebenenfalls vorbehandeln und Auftragen der zu untersuchenden Probe auf ein Substrat,
   b) Hinzufügen von für die Detektion gekennzeichneten Sonden, die durch spezifische Bindung an das jeweilige Aggregat dieses markieren und
   c) Detektion der markierten Aggregate,
      wobei Schritt b) vor Schritt a) durchgeführt werden kann und
      die Krankheit ausgewählt ist aus der Gruppe bestehend aus:
         AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer-Demenz;
ii) Vergleich dieser Daten mit den Normwerten;
iii) Feststellen einer signifikant höheren Menge an Protein-Aggregaten bei diesem Vergleich;
iv) Zuordnung der Abweichung zu einem Krankheitsbild gemäß den Krankheitsindikatoren.

Auf dem erfindungsgemäßen Substrat werden in mindestens zwei genau definierten Bereichen spezifische Fängermoleküle immobilisiert, die an unterschiedliche Aggregate körpereigene Proteine binden. In einer Alternative werden 3, 4, 5, 6, 7, 8, 9, 10, 11, 12, 13, 14, 15, 16 oder mehrere unterschiedliche spezifische Fängermoleküle auf genau definierten Bereichen des Substrats immobilisiert. Entsprechend der Bindung und anschließender Detektion der Aggregate ist dann eine Differenzialdiagnose möglich.

In einer Alternative können pro Well 16 oder mehrere unterschiedliche Fängermoluküle auf definierte Subflächen aufgebracht (gespottet) werden. Somit können verschiedene Aggregattypen und sogar heterogene Aggregate aus verschiedenen Monomeren in einer einzigen Probe identifiziert werden. Dadurch reichen kleine Mengen Körperflüssigkeiten aus. Außerdem kann so schon die Rasterposition zur Identifizierung der Aggregate dienen.

In einer Alternative kann ein Gemisch an verschiedenen Fängerantikörpern in einem einzigen Substrat (Well) immobilisiert werden, oder durch ein "Spotting"-Verfahren auf verschiedenen Positionen innerhalb einer einzigen Oberfläche, z.B. am Boden eines Mikrotiterplatten-Wells unterschiedliche Fängermoleküle (z.B. Antikörper) aufgebracht werden, die zum Beispiel spezifisch eine oder mehrere der Sonden binden oder die spezifisch eine oder mehrere der aggregierten Proteine binden. Durch den Einsatz verschiedener Sonden, die jeweils einen anderen Fluorophor tragen, können so verschiedene Aggregatspezies nahezu simultan in einem einzigen Probenaliquot analysiert werden.

Auch können so Aggregat-Mischformen, bestehend aus verschiedenen Protein-Monomeren identifiziert werden.

Somit ist nicht nur die Diagnose von Kombinationen von Krankheiten möglich, sondern aus diesen Ergebnissen lassen sich andere Krankheiten auch ausschließen. Dadurch wird eine genauere Diagnose ebenfalls ermöglicht.

Ein Vorteil des erfindungsgemäßen Verfahrens ist die Möglichkeit, mittels einer einzigen Patientenprobe, die eine Vielzahl von Entitäten umfasst, die mit Proteinaggregaten einhergehen, auf alle möglichen Aggregat-Typen zu untersuchen, die auf körpereigenen fehlgefalteten Proteinen beruhen. Weiterer Vorteil ist die Möglichkeit, die Proteinaggregate unmittelbar, ex vivo in Proben, wie Blut und Cerebrospinalflüssigkeitsproben oder Urin, Speichel, Schleimhaut, Biopsie-Material, insbesondere Blut und Cerebrospinalflüssigkeitsprobenunmittelbar auf Aggregat-Typen zu untersuchen.

Durch das erfindungsgemäße Verfahren können neue Krankheitsindikatoren qualitativ und/oder quantitativ bestimmt werden. Gegenstand der Erfindung sind somit auch Krankheitsindikatoren, Biomarker, gekennzeichnet durch die Anwesenheit eines Aggregat-Typs körpereigener fehlgefalteter Proteine, die erfindungsgemäß untersucht werden und ggf. die An- und/oder Abwesenheit mindestens eines weiteren -Aggregat-Typs.

Durch das erfindungsgemäße Verfahren und die neuen Biomarker kann eine der Krankheiten basierend auf fehlgefalteten Protein-Aggregaten (wie oben genannt) identifiziert und von den anderen Krankheiten der Gruppe, ggf. mit ähnlichen oder übereinstimmenden Symptomen, eindeutig abgegrenzt werden.

Gegenstand der Erfindung ist somit ferner ein Biomarker bestimmt mittels des erfindungsgemäßen Verfahrens der durch die Anwesenheit mindestens eines Aggregat-Typs körpereigener fehlgefalteter Proteine ausgewählt wird aus der Gruppe bestehend aus Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, Tau-Aggregate, TDP-43-Aggregate, Huntingtin-Aggregate, Lysozym-Aggregate und A-beta-Aggregate oder Aggregat-Mischformen davon und gegebenenfalls durch die An- und/oder Abwesenheit mindestens eines weiteren der oben genannten Aggregat-Typen oder Aggregat-Mischform.

Das erfindungsgemäße Verfahren kann ferner zur Festlegung von Grenzwerten der Konzentration der Protein-Aggregate verwendet werden, die für ein Krankheitsbild oder die Diagnose einer Krankheit relevant sind.

Gegenstand der vorliegenden Erfindung ist ein Kit zur Differenzialdiagnose enthalten einen oder mehrere der folgenden Komponenten:
- Substrat wie oben beschrieben,
- Standard wie oben beschrieben,
- Sonde,
- Lösungen,
- Puffer,
wobei die Krankheit ausgewählt wird aus der Gruppe bestehend aus von AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Tauopathien, Chronische Traumatische Enzephalopathie und andere TDP-43-Proteinopathien, Huntington-Krankheit, Familiäre Viszerale Amyloidose und/oder Alzheimer-Demenz.

Weiterer Gegenstand der vorliegenden Erfindung ist die Verwendung des erfindungsgemäßen Verfahrens zur Diagnose, Frühdiagnose und/oder Prognose der oben genannten Krankheiten.

Weiterer Gegenstand der vorliegenden Erfindung ist die Verwendung des erfindungsgemäßen Verfahrens zur Überwachung von Therapien der oben genannten Krankheiten sowie zur Überwachung und/oder Überprüfung der Wirksamkeit von Wirkstoffen und/oder Heilverfahren. Dies kann bei klinischen Tests, Studien als auch beim Therapie-Monitoring eingesetzt werden. Hierzu werden Proben gemäß dem erfindungsgemäßen Verfahren vermessen und die Ergebnisse verglichen.

Weiterer Gegenstand der vorliegenden Erfindung ist die Verwendung erfindungsgemäßen Verfahrens und der Biomarker zur Entscheidung, ob eine Person in klinische Studie aufgenommen wird. Hierzu werden Proben gemäß dem erfindungsgemäßen Verfahren vermessen und in Bezug auf einen Grenzwert die Entscheidung getroffen.

Weiterer Gegenstand der vorliegenden Erfindung ist ein Verfahren zur Bestimmung der Wirksamkeit von Wirkstoffen und/oder Heilverfahren mittels des erfindungsgemäßen Verfahrens, bei dem die Ergebnisse von Proben miteinander verglichen werden. Bei den Proben handelt es sich um Körperflüssigkeiten, entnommen vor beziehungsweise nach, oder zu unterschiedlichen Zeitpunkten nach Gabe der Wirkstoffe beziehungsweise Durchführung des Heilverfahrens. Anhand der Ergebnisse werden Wirkstoffe und/oder Heilverfahren ausgewählt, durch die eine Verringerung der Aggregate erfolgte. Erfindungsgemäß werden die Ergebnisse mit einer Kontrolle verglichen, die nicht dem Wirkstoff und/oder Heilverfahren unterworfen wurde.

Die Erfindung ist im folgendem durch weitere Aspekte beschrieben:
1. Aspekt Verfahren zur qualitativen und/oder quantitativen Bestimmung von Krankheitsindikatoren dadurch gekennzeichnet, dass eine Probe auf mindestens einen Aggregat-Typ, bevorzugt mindestens zwei Aggregat-Typen körpereigener fehlgefalteter Proteine untersucht wird, umfassend folgende Schritte:
   a) Auftragen der zu untersuchenden Probe auf ein Substrat,
   b) Hinzufügen von für die Detektion gekennzeichneten Sonden, die durch spezifische Bindung an das jeweilige Aggregat dieses markieren und
   c) Detektion der markierten Aggregate,
      wobei
      - Schritt b) vor Schritt a) durchgeführt werden kann und
      - die Krankheit ausgewählt ist aus der Gruppe bestehend aus:
      Tauopathien, AA-Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Chronische Traumatische Enzephalopathie und andere TDP-43-Proteinopathien, Huntington-Krankheit, Familiäre Viszerale Amyloidose und/oder Alzheimer'sche Demenz und - falls A-beta-Aggregate in der Probe bestimmt wurden, die Probe auf mindestens einen weiteren Aggregat-Typ eines körpereigenen fehlgefalteten Proteins untersucht wird.
2. Aspekt Verfahren nach Aspekt 1, dadurch gekennzeichnet, dass die Probe ohne weitere Aufarbeitung und/oder Behandlung auf mindestens zwei unterschiedliche Aggregat-Typen körpereigener fehlgefalteter Proteine untersucht wird.
3. Aspekt Verfahren nach Aspekt 2, dadurch gekennzeichnet, dass die Probe auf mindestens zwei unterschiedliche Aggregat-Typen in einem Verfahrensschritt untersucht wird.
4. Aspekt Verfahren nach Aspekt 2, dadurch gekennzeichnet, dass die Probe auf mindestens zwei unterschiedliche Aggregat-Typen auf demselben Substrat untersucht wird.
5. Aspekt Verfahren nach Aspekt 1, dadurch gekennzeichnet, dass der Aggregat-Typ körpereigener fehlgefalteter Proteine ausgewählt wird aus der Gruppe bestehend aus Tau-Aggregate, Serum-Amyloid-A-Protein-Aggregate, IgG-light-chain-Aggregate, AapoAI-Aggregate, AapoAII-Aggregate, ATTR-Aggregate, DISC1-Aggregate, FUS-Aggregate, IAPP-Aggregate, SOD1-Aggregate, TDP-43-Aggregate, Huntingtin-Aggregate, Lysozym-Aggregate, A-beta-Aggregate und Mischaggregate.
6. Aspekt Verfahren nach Aspekt 1, dadurch gekennzeichnet, dass die Detektion in Schritt c) mittels einem Verfahren mit ortsaufgelöstem Signal, bevorzugt sFIDA-Verfahren erfolgt.
7. Aspekt Verfahren nach Aspekt 1, dadurch gekennzeichnet, dass vor Schritt a) eine Immobilisierung von Fängermolekülen auf dem Substrat erfolgt.
8. Aspekt Verfahren nach einem der vorangehenden Aspekte, dadurch gekennzeichnet, dass die Fängermoleküle und/oder die Sonde mit Fluoreszenzfarbstoffen gekennzeichnet sind.
9. Aspekt Verfahren nach einem der vorangehenden Aspekte, dadurch gekennzeichnet, dass die Fängermoleküle und/oder die Sonden spezifische Anti-Körper gegen ein Epitop der Proteine aufweisen, die die Aggregate bilden.
10. Aspekt Verfahren nach Aspekt 1, dadurch gekennzeichnet, dass ein interner oder externer Standard eingesetzt wird.
11. Aspekt Verfahren nach Aspekt 8 oder 10, dadurch gekennzeichnet, dass die Fängermoleküle, Sonden und/oder Standards ein Polymer umfassen, aufgebaut aus Monomer-Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.
12. Aspekt Standard zur selektiven Quantifizierung und/oder Charakterisierung der Krankheitsindikatoren gemäß Aspekt 1 enthaltend ein Polymer eingesetzt wird, aufgebaut aus Monomer-Sequenzen, die bezüglich ihrer Sequenz identisch mit einem Teilbereich der körpereigenen Proteine ist oder eine Homologie von mindestens 50 % über den entsprechenden Teilbereich aufweisen, wobei diese Polymere nicht aggregieren und es sich bei den körpereigenen Proteinen um jene handelt, die aggregieren.
13. Aspekt Substrat umfassend genau definierte Bereiche mit Fängermolekülen für die Krankheitsindikatoren gemäß Aspekt 1 und/oder Aggregate.
14. Aspekt Differential-Diagnose zur Bestimmung von Krankheiten die Aggregate fehllgefalteter Proteine aufweisen, umfassend folgende Schritte:
   i) quantitativen Bestimmung von Krankheitsindikatoren nach Aspekt 1;
   ii) Vergleich dieser Daten mit den Normwerten;
   iii) Feststellen einer signifikant unterschiedlichen Menge Krankheitsindikatoren bei diesem Vergleich;
   iv) Zuordnung der Abweichung zu einer Krankheit nach Aspekt 1.
15. Aspekt KIT zur Differenzialdiagnose enthalten einen oder mehrere der folgenden Komponenten:
   - Substrat nach Aspekt 13,
   - Standard nach Aspekt 12,
   - Sonde,
   - Lösungen,
   - Puffer,
   wobei die Krankheit ausgewählt wird aus der Gruppe bestehend aus von AA-Tauopathien, Amyloidose, AL-Amyloidose, AApoAI-Amyloidose, AApoAII-Amyloidose, ATTR-Amyloidose, Schizophrenie und andere DISC1opathien, Amyotrophe Lateralsklerose, Frontotemporale Lobärdegeneration und andere FUS-Proteinopathien, Diabetes Mellitus Typ 2, Parkinson-Krankheit und andere Synucleinopathien, Chronische Traumatische Enzephalopathie und andere TDP-43-Proteinopathien, Huntington-Krankheit, Familiäre Viszerale Amyloidose und/oder Alzheimer-Demenz.

### Beispiele

### 1. Material und Methode

Als Probenträger wurden im Assay Multiwellplatten mit einem 170 µm starken Glasboden eingesetzt (SensoPlate Plus 384 Greiner Bio One, Kremsmünster, Österreich). Alle zum Einsatz kommenden Reagenzien und Lösungen wurden im höchsten Reinheitsgrad bezogen und vor Verwendung partikelfrei sterilisiert.

Im ersten Schritt wurde jede Probenkammer (Well) mit 100 µl Natronlauge (5 M) gefüllt, 15 Minuten bei Raumtemperatur inkubiert, dreimal mit Wasser gespült, mit 100 µl Salzsäure versetzt und erneut 15 Minuten bei Raumtemperatur inkubiert. Nach dreimaligem Waschen mit Wasser und zweimaligem Waschen mit Ethanol wurden die Wells unter Stickstoff-Atmosphäre getrocknet.

Zur Erzeugung von Aminogruppen auf der Glasoberfläche wurden jeweils 20 µl Ethanolamin (5,6 M) in die Wells gegeben und über Nacht bei Raumtemperatur inkubiert. Die Wells wurden dreimal mit DMSO, zweimal mit Ethanol gespült und unter Stickstoff-Atmosphäre getrocknet.

Heterobiofunktionales Polyethylenglykol (NHS-PEG-COOH, MW 3.400 Da) wurde zu 50 mM in DMSO bei 70 °C für 1 min gelöst, auf Raumtemperatur abgekühlt und auf 2% Triethylamin eingestellt. Von dieser Lösung wurde jeweils 15 µl in die Wells gegeben und für mindestens eine Stunde bei Raumtemperatur inkubiert. Die Lösung wurde aus den Wells entfernt, und die Wells werden dreimal mit Wasser gewaschen.

Zur Aktivierung der PEG-Beschichtung wurden NHS und EDC (Carbodiimid) zu jeweils 100 mM in MES-Puffer (0,1 M, pH 5, MES: 2-N-Morpholinoethansulfonsäure) gelöst und im Verhältnis 1:1 zu Endkonzentrationen von jeweils 50 mM gemischt. Je 30 µl dieser Lösung wurde in die Wells gefüllt und 30-60 Minuten inkubiert. Nach Entfernen der Lösung wurden die Wells dreimal mit MES-Puffer (0,1 M, pH 5) gewaschen.

Der Fänger-Antikörper wurde auf 30 ng/µl in PBS verdünnt. Davon wurden 15 µl in die Wells gegeben und für 1-3 Stunden bei Raumtemperatur inkubiert. Anschließend wurden die Lösung entfernt und dreimal mit PBST (PBS mit Tween 20), dann dreimal mit PBS gewaschen.

3% BSA wurde zuvor bei 100.000 g (1 Stunde bei 4°C) zentrifugiert. Je 50 µl des Überstands wurde in die Wells gefüllt und für eine Stunde bei Raumtemperatur inkubiert. Die BSA-Lösung wurde entfernt und dreimal mit PBST gewaschen.

Die Probe (z.B. Aggregate aus rekombinantem Protein und natürliche Patientenprobe) wurde - sofern erforderlich - in PBS verdünnt und davon jeweils 15 µl in die Wells gegeben. Die Multiwellplatte wurde bei 1.000g für eine Stunde bei 4 °C in einem Ausschwingrotor zentrifugiert. Der Überstand wurde entfernt und die Wells dreimal mit PBST und dreimal mit PBS gewaschen.

Als Detektionssonden wurden fluoreszenzmarkierte Antikörper eingesetzt. Diese werden auf 1-2 ng/µl in PBS verdünnt und mit 1.5% BSA versetzt. Die Ansätze wurden bei 100.000 g für eine Stunde bei 4 °C zentrifugiert. Je 15 µl des Überstands wurde in die Wells gefüllt und für 1-2 Stunden bei Raumtemperatur inkubiert. Anschließend wurde die Lösung entfernt und fünfmal mit PBST und fünfmal mit PBS gewaschen.

Die Oberflächenfluoreszenz wurde mittels Total Internal Reflection Fluoreszenz Mikroskopie (TIRFM) visualisiert. Alternativ können die Proteinpartikel mit konfokalem Laser Scanning auf Ebene der Glasoberfläche sichtbar gemacht werden. Pro Well wurden bis zu 50 Einzelbilder (1000x1000 Pixel, 114 nm/pixel) pro Fluoreszenzkanal mit einer hochsensitiven CCD-Kamera abgebildet.

Hintergrundsignale in den Bilddaten wurden über einen Intensitätsschwellenwert bereinigt. Die mittlere Anzahl der Pixel mit Graustufenwerten oberhalb des Schwellenwerts wurde bestimmt (sFIDA-readout). Kamen mehrere Detektionssonden zum Einsatz, wurden ausschließlich die in allen Fluoreszenzkanälen colokalisierten Ereignisse gewertet.

### 2. sFIDA Protokoll

### 2.1. Vorbehandlung

- 15 min NaOH (5M) 100 µl/Well
- 3x H₂O
- 15 min HCl (1M) 100 µl/Well
- 3x H₂O
- 2x EtOH spülen
- Mit N₂ trocknen

### 2.2. Glasaktivierung (Aminogruppen)

| |
|---|
| • 20 µl/Well Ethanolamin (5,6 M)◊*Inkubation* ÜN (über Nacht) Raumtemperatur oder länger 4°C |
| • 3× DMSO |
| • 2× EtOH |
| • Mit N₂ trocknen |

### 2.3. Spacer NHS-PEG-COOH

- 17 mg PEG in 100 µl DMSO bei 70°C (kurz) lösen, abkühlen lassen, + 2 µl Triethylamin (TEA)
- 15 µl/Well ◊ *Inkubation*: mind. 1 h
- 3× H₂O

### 2.4. PEG Aktivierung mit NHS/EDC (50mM)

- 5,8 mg NHS und 9,6 mg EDC in 500 µl MES (0,1M) verdünnen, direkt vor dem auftragen 1:1 mischen
- 30 µl/Well ◊ *Inkubation*: 30 min - max. 1 h
- zügig 3x MES (0,1M)

### 2.5. Capture

- Capture-Antikörper in PBS verdünnen (30ng/µl)
- 15 µl/Well -> Inkubation 1-3 h RT oder länger 4°C
- 3x PBST, 3x PBS

### 2.6. Blocken

- 3% BSA 1 h bei 100.000 × g, 4°C (Rotor TLA-45)
- 50 µl/Well◊*Inkubation* 1 h
- 3x PBST
- 3x PBS Tag 2

### 2.7. Target

- 15 µl Target, verdünnt in PBS
- *Inkubation* 1h zf, 1000g , 25°C,
- HH: 3x 0,2% SDS/PBS; 5x PBST; 5x PBS
- Plasma und rekombinante Aggregate: 3x PBST; 3x PBS

### 2.8. Detektionsantikörper

- 1-2 ng/µl in 1,5% BSA mit PBS nach 100.000 × g, 4°C (Rotor TLA-45)
- je 15 µl Überstand/Well◊ *Inkubation* >=1-2h, RT
- 5x PBST
- 5x PBS
(für Waschschritte wurden jeweils 100µl der entsprechenden Lösung verwendet)

### 3. Anwendung mit Aggregaten aus rekombinantem Protein

Zunächst wurde die prinzipielle Machbarkeit des sFIDA-Assays zum Nachweis verschiedener Aggregatspezies demonstriert. Exemplarisch wurden Aggregate von verschiedenen Proteinen (AapoAI, AL, SOD1, ALys, AA, TDP-43) präpariert, und entsprechende Verdünnungsreihen im sFIDA-Assay untersucht. Dabei wurde als Fänger- und Detektionsantikörper jeweils derselbe, gegen das jeweilige Protein gerichtete Antikörper verwendet. Die Detektionssonde wurde jeweils mit Alexafluor 488 markiert. Alle Aggregate können konzentrationsabhängig bis in den picomolaren Bereich mittels sFIDA nachgewiesen werden.

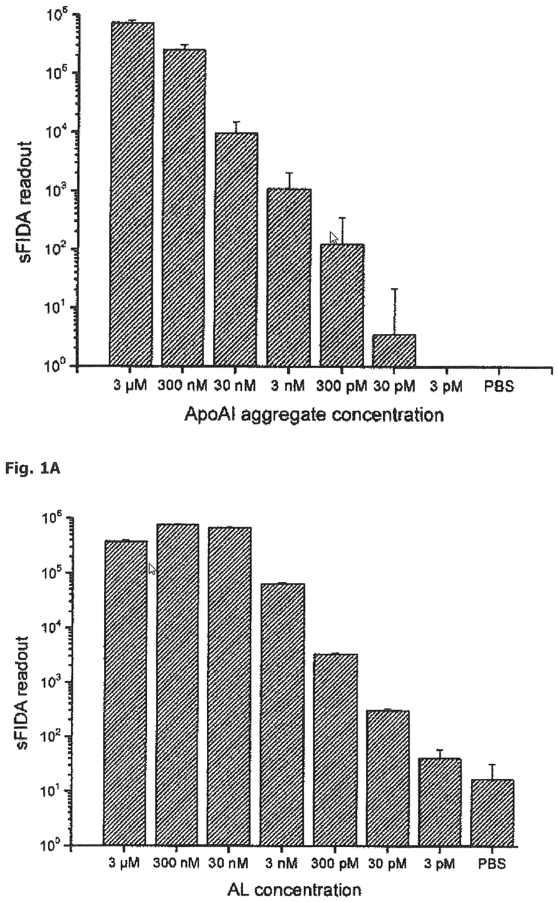

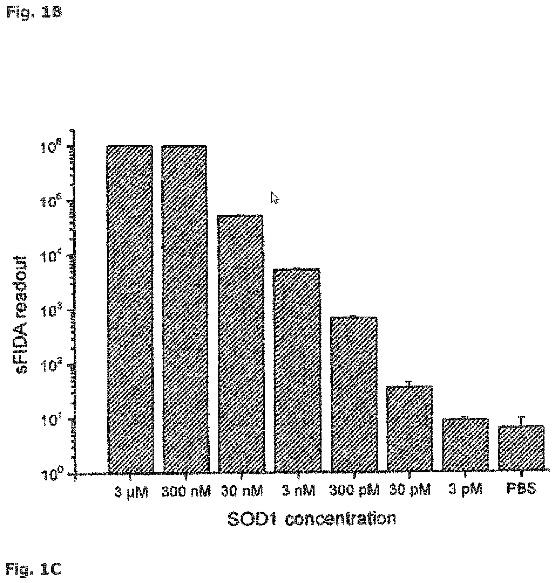

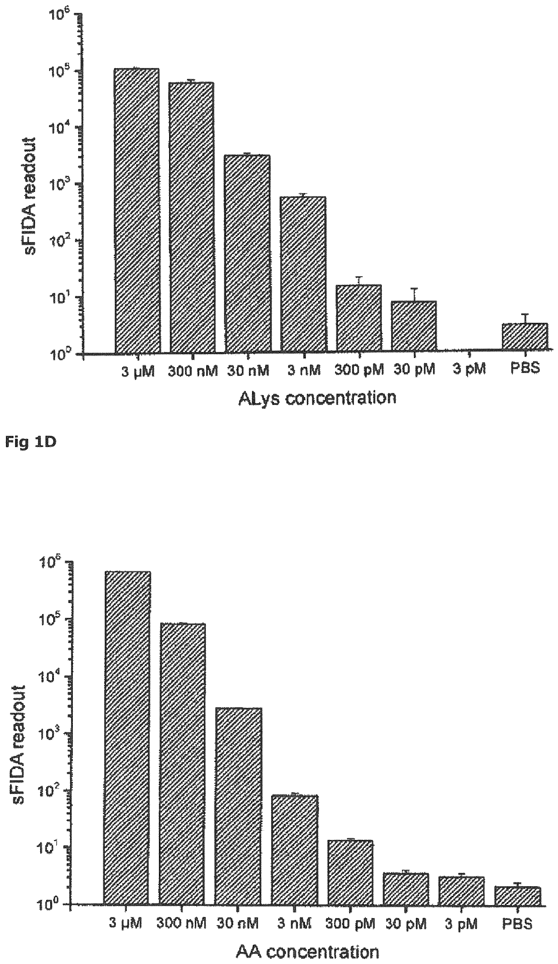

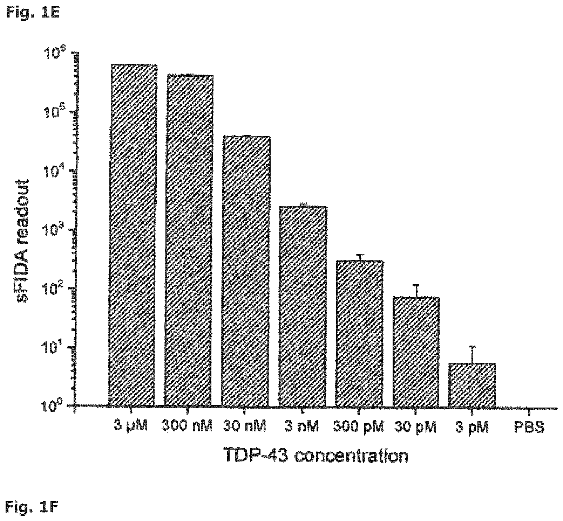

Fig. 1A-F: sFIDA von verschiedenen Proteinaggregaten. Die Proteinaggregate wurden in den angegebenen Konzentrationen in PBS-Puffer verdünnt und im sFIDA-Assay analysiert. Als Neagtivkontrolle wurde PBS-Puffer verwendet. **A)** ApoAI, Fänger- und Detektionssonde (AF488-markiert): EPR 1368Y **B)** AL, Fänger- und Detektionssonde (AF488-markiert): EPR 5367 **C)** SOD1, Fänger- und Detektionssonde (AF488-markiert): EPR 1726 **D)** ALys, Fänger- und Detektionssonde (AF488-markiert): EPR 2994 (2) **E)** AA, Fänger- und Detektionssonde (AF488-markiert): EPR 4134 **F)** TDP-43, Fänger- und Detektionssonde (AF488-markiert): EPR 5810

### 4. Anwendung an nativen Proben

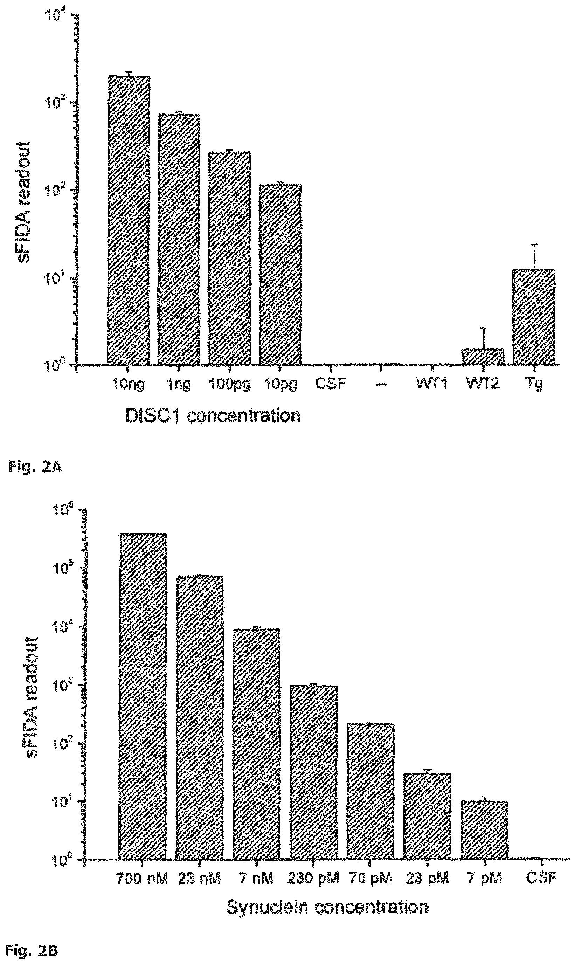

Um exemplarisch zu demonstrieren, dass Proteinaggregate in Blut- und Cerebrospinalflüssigkeitsproben (CSF-Proben) oder Urin, Speichel, Schleimhaut, Biopsie-Material detektiert werden können, wurden Proteinaggregate (α-Synuclein und DISC1) in CSF verdünnt und mittels sFIDA analysiert. Für den Nachweis von α-Synuclein wurde als Fänger-Antikörper Anti-aSyn-AB 2B2A11 und als Detektionssonde die fluoreszenzmarkierten Anti-aSyn-ABs 3H2897-AF633 bzw. 211-AF488 eingesetzt. Zur Detektion von DISC1-Aggregaten wurde Anti-DISC1-AK 14F2 als Fänger-Antikörper und 14F2-AF633 als Detektionssonde eingesetzt. Sowohl Synuclein als auch DISC1-Aggregate lassen sich konzentrationsabhängig bis in den picomolaren Bereich mittels sFIDA nachweisen (Siehe Fig. 2A und B).

Zur Untersuchung der molekularen Grundlagen der Schizophrenie wurde im Labor von Carsten Korth ein Rattenmodell entwickelt, welches das Protein DISC1 verstärkt exprimiert. Um zu überprüfen, ob dieses Protein nativ in Aggregaten vorliegt, wurden CSF-Proben eines transgenen Tiers mittels sFIDA analysiert. Im Vergleich zu zwei Kontrolltieren zeigte sich in der transgenen Probe ein deutlich erhöhter Titer an DISC1-Aggregaten (Fig. 2A, Proben WT1, WT2, Tg).

Fig. 2: sFIDA-Nachweis von Proteinaggregaten in CSF. Aggregate aus rekombinantem Protein wurden in humanem CSF verdünnt und in einem sFIDA-Assay untersucht. A) DISC1-Nachweis. Als Fängerantikörper und Detetktionssonde (AF633-markiert) kam mAB 14F2 zum Einsatz. Zusätzlich zu rekombinanten DISC1-Aggregaten (10pg-10ng) wurden CSF-Proben von Ratten (WT1, WT2, Tg) einer sFIDA-Analyse unterzogen. B) Synuclein-Nachweis mittels 2D sFIDA. Als Fänger-Antikörper wurde Anti-aSyn-AB 2B2A11 und als Detektionssonde die fluoreszenzmarkierten Anti-αSyn-ABs 3H2897-AF633 bzw. 211-AF488 eingesetzt. Nur die in beiden Farbkanälen kolokalisierten Signale oberhalb eines Schwellenwertes wurden berücksichtigt.

### 5. Differenzialdiagnostische Anwendung von sFIDA

Eine Probe wurde auf verschiedene Reaktionskammern (Wells) verteilt, welche mit den verschiedenen Fängerantikörpern beschichtet sind. Alternativ können ein Gemisch an verschiedenen Fängerantikörpern in einem einzigen Well immobilisiert werden, oder durch ein "Spotting"-Verfahren auf verschiedenen Positionen innerhalb einer einzigen Oberfläche, Z.B. am Boden eines Mikrotiterplatten-Wells unterschiedliche Fängerantikörper aufgebracht werden.

Durch den Einsatz verschiedener Sonden, die jeweils einen anderen Fluorophor tragen, wurden so verschiedene Aggregatspezies nahezu simultan in einem einzigen Probenaliquot analysiert.

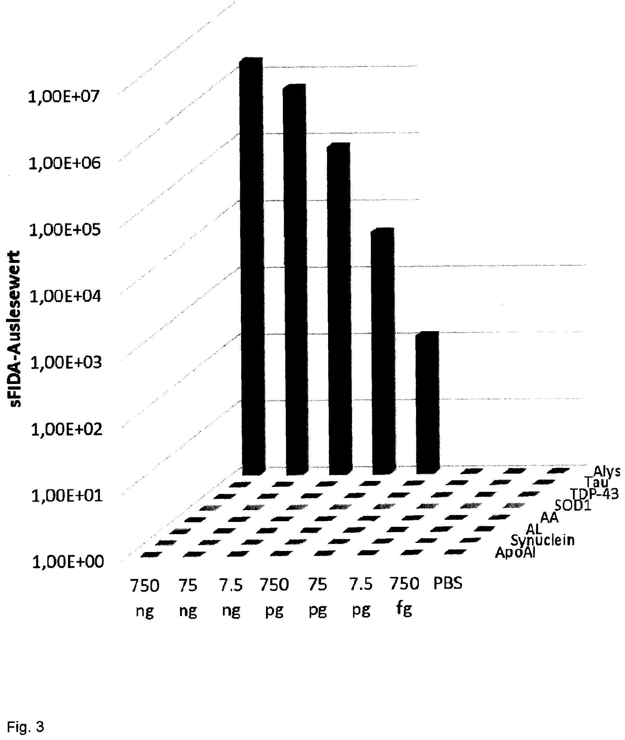

In diesem Ausführungsbeispiel wurde gezeigt, dass ein ALys-spezifischer sFIDA-Assay (Anti-Lysozym Antikörper als Sonde und Fänger) in einem breiten Konzentrationsbereich nicht mit anderen Proteinaggregaten kreuzreagiert (Abb. 3). Die Anti-Lysozym Antikörper als Sonde und Fänger interagieren nur mit Aggregaten des Typs ALys.

In einem weiteren Beispiel wurde gezeigt, dass die Anwesenheit andere Proteine keinen negativen Einfluss auf die Nachweisbarkeit von Amyloid beta-Aggregaten haben. Dazu wurde ein Gemisch aus Amyloid-beta-Aggregaten und Tau-Aggregaten einer sFIDA-Analyse unterzogen.

Fig. 3. Spezifitätsanalyse. In einem ALys-spezifischen sFIDA-Assay mit Fängerantikörper Rabbit monoclonal EPR2994(2) und Detektionssonde EPR2994(2)-AF488 wurden neben einer Verdünnung von rekombinanten ALys-Aggregaten andere Proteinaggregate (Tau, TDP-43, SOD1, AA, AL, Synuclein, ApoAI) auf Kreuzreaktivität geprüft.

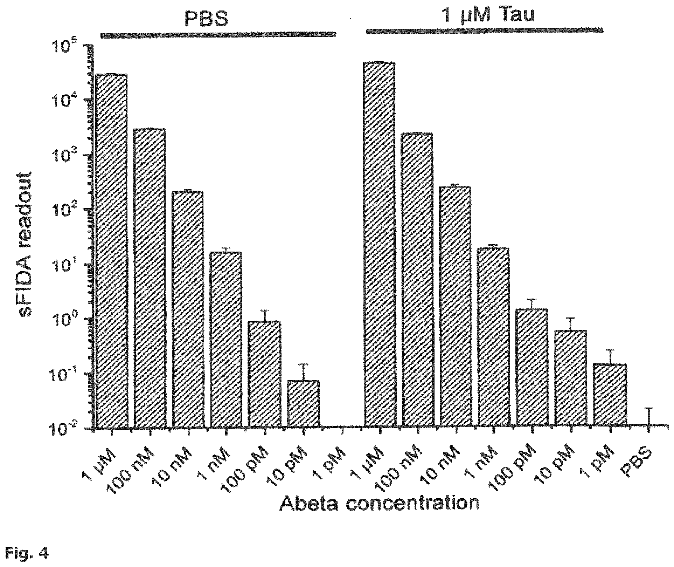

Fig. 4: Abeta-spezifischer sFIDA in Gegenwart von Tau-Protein. Proteinaggregate aus Abeta wurden in den angegebenen Konzentrationen verdünnt und in An- und Abwesenheit von 1 µM Tau-Protein-Aggregat in einem Abeta-spezifischen sFIDA-Assay (Detektionssonde 6E10/Atto488, Capture Nab228) analysiert.

## Patentansprüche

1. "Verfahren zur qualitativen und/oder quantitativen Bestimmung von Krankheitsindikatoren, **dadurch gekennzeichnet, dass** eine Probe auf mindestens zwei Aggregat-Typen körpereigener fehlgefalteter Proteine untersucht wird, wobei die mindestens zwei Aggregat-Typen körpereigener fehlgefalteter Proteine ausgewählt werden aus der Gruppe bestehend aus Tau-Aggregaten, Serum-Amyloid-A-Protein-Aggregaten, IgG-light-chain-Aggregaten, AapoAl-Aggregaten, AapoAll-Aggregaten, ATTR-Aggregaten, DISC1-Aggregaten, FUS-Aggregaten, IAPP-Aggregaten, SOD1-Aggregaten, α-Synuclein-Aggregaten, TDP-43-Aggregaten, Huntingtin-Aggregaten, Lysozym-Aggregaten, A-beta-Aggregaten und Mischaggregaten, umfassend folgende Schritte:
Vor Schritt a) Immobilisierung von Fängermolekülen auf einem Substrat, wobei die Fängermoleküle spezifische Anti-Körper gegen ein Epitop der Proteine aufweisen, die die Aggregate bilden,
a) Auftragen der zu untersuchenden Probe auf das Substrat, wobei als zu untersuchende Probe körpereigene Flüssigkeiten oder Gewebe ex vivo eingesetzt werden und wobei die Probe ohne weitere Aufarbeitung und/oder Behandlung auf mindestens zwei unterschiedliche Aggregat-Typen körpereigener fehlgefalteter Proteine untersucht wird,
b) Hinzufügen von für die Detektion gekennzeichneten Sonden, die durch spezifische Bindung an das jeweilige Aggregat dieses markieren, wobei als Sonden Antikörper eingesetzt werden, wobei mindestens 2 unterschiedliche Sonden eingesetzt werden, die sich sowohl bezüglich ihrer spezifischen Bindung an die Aggregate als such bezüglich ihrer unterschiedlichen Kennzeichnung unterscheiden und
c) Detektion der markierten Aggregate, wobei die Detektion in Schritt c) mittels einem Verfahren mit ortsaufgelostem Signal erfolgt, wobei
- Schritt b) vor Schritt a) durchgeführt werden kann,
und
- die Krankheit ausgewählt ist aus der Gruppe bestehend aus:
Tauopathien, Parkinson-Krankheit und andere Synucleinopathien und/oder Alzheimer'sche Demenz,
wobei eine Detektion von Monomeren ausgeschlossen wird, indem eine der Detektionssonden identisch mit dem Fängermolekül ist oder beide ein überlappendes Epitop erkennen,
und/oder indem Signale mit einer niedrigen Intensität durch ein Intensität-cut-off nicht gewertet werden,
und/oder durch Kreuzkorrelation.

2. Verfahren nach Anspruch 1, **dadurch gekennzeichnet, dass** die Probe auf mindestens zwei unterschiedliche Aggregat-Typen in einem Verfahrensschritt untersucht wird.

3. Verfahren nach Anspruch 2, wobei die zwei unterschiedlichen Aggregat-Typen Tau/TDP43 und TDP43/SOD1 sind.

4. Verfahren nach Anspruch 1, **dadurch gekennzeichnet, dass** die Probe auf mindestens zwei unterschiedliche Aggregat-Typen auf demselben Substrat untersucht wird.

5. Verfahren nach Anspruch 1, **dadurch gekennzeichnet, dass** die Detektion in Schritt c) mittels sFIDA-Verfahren erfolgt.

6. Verfahren nach einem der vorangehenden Ansprüche, **dadurch gekennzeichnet, dass** die Fängermoleküle und/oder die Sonde mit Fluoreszenzfarbstoffen gekennzeichnet sind.

7. Verfahren nach einem der vorangehenden Ansprüchen, **dadurch gekennzeichnet, dass** die Fängermoleküle und/oder die Sonden spezifische Anti-Körper gegen ein Epitop der Proteine aufweisen, die die Aggregate bilden.

8. Verfahren nach Anspruch 1, **dadurch gekennzeichnet, dass** ein interner oder externer Standard eingesetzt wird.

9. Verwendung eines Standards zur selektiven Quantifizierung und/oder Charakterisierung der Krankheitsindikatoren in einem Verfahren gemäß Anspruch 1, **dadurch gekennzeichnet, dass** es sich bei dem Standard um ein Dendrimer handelt, welches ein zentrales Gerüstmolekül und Epitope der Proteine a-Synuclein-Aggregate oder Tau-Aggregate gemäß SEQ ID NO: 10 oder SEQ ID NO: 11 enthält, wobei sowohl Standards, die aus den gleichen Monomer-Sequenzen aufgebaut sind, als auch solche, die aus unterschiedlichen Monomer-Sequenzen aufgebaut sind verwendet werden.

10. Verfahren zur Bestimmung von Krankheiten die Aggregate fehlgefalteter Proteine aufweisen, umfassend folgende Schritte:
i) quantitativen Bestimmung ex vivo nach Anspruch 1 von Krankheitsindikatoren;
ii) Vergleich dieser Daten mit den Normwerten;
iii) Feststellen einer signifikant unterschiedlichen Menge Krankheitsindikatoren bei diesem Vergleich;
iv) Zuordnung der Abweichung zu einer Krankheit ausgewählt aus der Gruppe bestehend aus Tauopathien, Parkinson-Krankheit und andere Synucleinopathien und/oder Alzheimer'sche Demenz.

11. KIT zur Verwendung in einem Verfahren nach Anspruch 10 enthaltend:
- Standard nach Anspruch 9,
- Sonde,
- Fängermoleküle, Substrat und Sonden nach Anspruch 1,
- Lösungen,
- Puffer,
wobei die Krankheit ausgewählt wird aus der Gruppe bestehend aus von Tauopathien, Parkinson-Krankheit und andere Synucleinopathien, und/oder Alzheimer-Demenz.
